# EUROPEAN PATENT APPLICATION

(11) **EP 4 053 127 A1**
(43) Date of publication of application: **07.09.2022**
(21) Application number: 20883371.5
(22) Date of filing: 29.10.2020
(51) Int. Cl.: C07D 487/04, C07D 487/14, C07D 471/14, A61K 31/4985, A61P 35/00

(54) **NOVEL COMPOUND AND PHARMACEUTICAL COMPOSITION FOR PREVENTION OR TREATMENT OF CANCER COMPRISING SAME**

(30) Priority: 31.10.2019 US 201962928398 P
(71) Applicant: Holosmedic, Anyang-si, Gyeonggi-do 14058 (KR)
(72) Inventor: YUN, Yeo Jin, Anyang-si, Gyeonggi-do 14058 (KR); LEE, In Hye, Anyang-si Gyeonggi-do 14058 (KR)
(74) Representative: BCKIP
(86) International application number: PCT/KR2020/014884
(87) International publication number: WO 2021/086038

(57) **Abstract**

The present invention relates to a novel compound, a method for preparing same, and a pharmaceutical composition for the prevention or treatment of refractory cancer, comprising the novel compound or a pharmaceutically acceptable salt thereof. When the composition of the present invention and anticancer drug are administered in combination, excellent anticancer effects can be exhibited.

## Description

### [Technical Field]

This application claims priority to and the benefit of U.S. Provisional Application 62/928,398 filed with the United States Patent and Trademark Office (USPTO) on October 31, 2019.

The present invention relates to a novel compound and a pharmaceutical composition for preventing or treating cancer including the compound or a pharmaceutically acceptable salt thereof.

### [Background Art]

Cancer is one of the most common causes of death around the world, and accounts for approximately 12% of deaths. Chemotherapy, which is a typical anticancer therapy, is currently being used as the most efficient therapeutic method of treating cancer alone or in combination with different therapeutic methods such as radiation therapy. However, in chemotherapy, the efficacy of a cancer therapeutic drug depends on its ability to kill cancer cells, but there is a problem of not only acting on cancer cells but also acting on normal cells when the drug is used.

It was found that, when an anticancer drug is administered, in general cancer cells, excessive stress is induced, calcium ions are excessively secreted from the endoplasmic reticulum (ER), and the secreted calcium ions are accumulated in the mitochondria, leading to the death of cancer cells, whereas cancer stem cells survive while the concentration of calcium ions is regulated by increasing the expression of SERCA which may reduce the secretion of excessive calcium ions and simultaneously return the excessively secreted calcium ions to the ER when an anticancer agent is administered. That is, the SERCA protein may play a role in survival signaling in an ER stress signaling process.

When a substance that can act as an inhibitor targeting the SERCA protein, which is a cause of anticancer drug resistance of cancer stem cells, is developed to selectively inhibit the growth of cancer stem cells, by increasing the efficacy of chemotherapy using the anticancer drug, an excellent anticancer effect may be exhibited even when a lower dose of the drug is used.

### [Disclosure]

### [Technical Problem]

The present invention is directed to providing a novel compound.

The present invention is also directed to providing a pharmaceutical composition for preventing or treating cancer, which includes the novel compound.

The present invention is also directed to providing a pharmaceutical composition for preventing or treating resistant cancer, which includes the novel compound.

The present invention is also directed to providing a use of the novel compound for preventing or treating resistant cancer.

However, technical problems to be solved in the present invention are not limited to the above-described problems, and other problems which are not described herein will be fully understood by those of ordinary skill in the art from the following descriptions.

### [Technical Solution]

According to one embodiment of the present invention, there is provided a compound represented by the following Formula 1 or a pharmaceutically acceptable salt thereof: 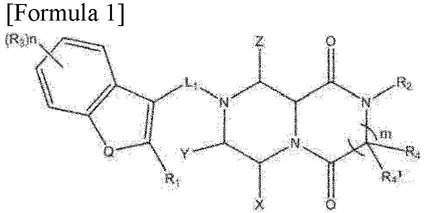 wherein:
R₁ is hydrogen, a linear or branched alkyl, an alkoxy, a haloalkyl, a haloalkoxy, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₃~C₆ cycloalkyl, a C₁~C₆ haloalkyl, a C₁~C₆ haloalkoxy, oxo, cyano, an unsubstituted or substituted aryl, or an unsubstituted or substituted heteroaryl;
R₃ is hydrogen, a linear or branched alkyl, a cycloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted aryl, or an unsubstituted or substituted heteroaryl;
L₁ is a C₁~C₁₀ alkylene, wherein the alkylene may be substituted with at least one selected from a C₁~C₆ alkyl, a C₃~C₆ cycloalkyl, a C₁~C₆ alkoxy, hydroxyl, oxo, or a halogen, and the C₁~C₆ alkyl, the C₃~C₆ cycloalkyl, and the C₁~C₆ alkoxy may be substituted with at least one selected from an unsubstituted or substituted aryl;
Q is S, Se, NR, P, P(O), P(O)₂, or P(O)OR, wherein R is hydrogen, a linear or branched alkyl, a cycloalkyl, a bi- or tri-cycloalkyl, an alkoxy, a haloalkyl, a haloalkoxy, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted aryl, or an unsubstituted or substituted heteroaryl;
R₂ is hydrogen, a linear alkyl, a cycloalkyl, an alkoxy, a haloalkoxy, a haloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl;
R₄ and R₄' are each independently hydrogen, a linear or branched alkyl, a cycloalkyl, an alkenyl, an alkynyl, an alkylthio, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl;
R₂ and R₄ may be connected to form a 5- to 10-membered monocyclic or bicyclic ring, wherein R₄' is hydrogen;
m is an integer of 1 or 2, and n is an integer ranging from 1 to 4; and
X, Y, and Z are each independently hydrogen, a linear or branched alkyl, a cycloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl.

According to another embodiment of the present invention, there is provided a pharmaceutical composition for preventing or treating cancer, which includes one or more selected from the compound of Formula 1 or a pharmaceutically acceptable salt thereof.

According to still another embodiment of the present invention, there is provided a pharmaceutical composition for preventing or treating resistant cancer, which includes one or more selected from the compound of Formula 1 or a pharmaceutically acceptable salt thereof.

### [Advantageous Effects]

The pharmaceutical composition including a compound of one embodiment of the present invention or a pharmaceutically acceptable salt thereof can exhibit an excellent anticancer effect.

The composition including the compound of one embodiment of the present invention or a pharmaceutically acceptable salt thereof can enhance the anticancer activity of an anticancer drug or radiation, and can effectively treat cancer by inhibiting the proliferation of cancer cells and inducing cell death.

The composition including the compound of one embodiment of the present invention or a pharmaceutically acceptable salt thereof can overcome the resistance of cancer with resistance to an anticancer drug or radiation, and thus effectively treat resistant cancer and can reduce side effects of anticancer treatment.

The composition including the compound of one embodiment of the present invention or a pharmaceutically acceptable salt thereof can effectively treat stem cell-like cancer.

The effects according to the present invention are not intended to limit the contents exemplified above, and various effects are included in the specification of the present specification.

### [Description of Drawings]

FIG. 1A shows an IC50 value of the anticancer drug paclitaxel with respect to SKOV3, and FIG. 1B shows an IC50 value of the anticancer drug paclitaxel with respect to SKOV3-TR.
FIG. 2A shows an IC50 value of the anticancer drug doxetaxel with respect to SKOV3, and FIG. 2B shows an IC50 value of the anticancer drug doxetaxel with respect to SKOV3-TR.
FIGS. 3 to 10 show the WST-1 analysis results of SKOV3-TR and SKOV3 against paclitaxel.
FIGS. 11 and 12 show the crystal violet staining results of SKOV3-TR and SKOV3 by treatment with paclitaxel.
FIGS. 13 to 20 show the optical density results of SKOV3-TR and SKOV3 by treatment with paclitaxel.
FIGS. 21 to 24 show the WST-1 analysis results of SKOV3-TR and SKOV3 by treatment with doxetaxel.
FIGS. 25 and 26 show the crystal violet staining results of SKOV3-TR and SKOV3 by treatment with doxetaxel
FIGS. 27 to 30 show the optical density results of SKOV3-TR and SKOV3 by treatment with doxetaxel.
FIG. 31 shows changes in cell counts after selected-MDA-MB231 cells and selected-MCF7 cells, which are stem cell-like cancer cells, are treated with 100 nM, 50 nM, and 10 nM of an S101 compound when no ER stress is applied to the cancer cells in the presence of glucose (G(+)) and ER stress is applied to the cancer cells in the absence of glucose (G(-)).
FIG. 32 shows the results of capturing images of the selected-MDA-MB231 and the selected-MCF7 cells stained with crystal violet.
FIG. 33 shows the results of measuring a cancer cell death effect of S101 after glucose deprivation using a TUNEL assay.
FIG. 34 shows changes in tumor volume when the cancer cells are treated with S101 and 2DG alone or in combination thereof.
FIG. 35 shows the weight and size of a dissected tumor when the cancer cells are treated with S101 and 2DG alone or in combination thereof.
FIG. 36 shows changes in body weight between respective groups by measuring anticancer effects when the cancer cells are treated with S101 and 2DG alone or in combination thereof in an s-231 xenograft model.
FIG. 37 shows whether liver tissue is degraded in order to decipher the degree of cytotoxicity when the cancer cells are treated with S101 and 2DG alone or in combination thereof.

### [Best Mode]

Advantages and features of the present invention and methods for achieving them will be apparent with reference to the embodiments described below in detail with the accompanying drawings. However, the present invention is not limited to embodiments disclosed below and may be implemented in various different forms. Therefore, it should be understood that the embodiments disclosed herein are merely provided to make the disclosure of the present invention complete and fully inform persons having ordinary skill in the art to which the present invention belongs of the scope of the present invention, which is defined only by the scope of the claims.

In this specification, the term "independently" indicates that the independently applied variables vary independently from application to application. Thus, in compounds such as R^{a}XYR^{a}, when R^{a} is "independently carbon or nitrogen," both R^{a} may be carbon, both R^{a} may be nitrogen, or one R^{a} may be carbon, and the other R^{a} may be nitrogen.

Unless otherwise specified in this specification, the term "alkyl" typically refers to a saturated linear or branched hydrocarbon chain of a C₁~C₁₀ alkyl, more specifically a saturated linear or branched hydrocarbon chain of a C₁~C₆ alkyl. The "C₁~C₁₀ alkyl" refers to a linear or branched alkyl having 1 to 10 carbon atoms. Specifically, the alkyl includes methyl, ethyl, propyl, isopropyl, butyl, isobutyl, t-butyl, pentyl, isopentyl, neopentyl, hexyl, isohexyl, cyclohexyl, cyclohexylmethyl, 3-methylpentyl, 2,2-dimethylbutyl, and 2,3-dimethylbutyl, and the like. The term includes both substituted and unsubstituted alkyl groups. An alkyl group may be optionally substituted with one or more moieties selected from the group consisting of hydroxyl, amino, alkylamino, arylamino, alkoxy, aryloxy, nitro, cyano, sulfonic acid, sulfate, phosphoric acid, phosphate, or phosphonate. One or more hydrogen atoms attached to the carbon atoms of the alkyl may be, for example, substituted with one or more halogen atoms such as fluorine, chlorine, or both, thereby obtaining trifluoromethyl, difluoromethyl, fluorochloromethyl, and the like. The hydrocarbon chain may also include a heteroatom such as N, O, or S in the middle thereof.

The term "alkaryl" or "alkylaryl" refers to an alkyl group having an aryl substituent. The term "aralkyl" or "arylalkyl" refers to an aryl group having an alkyl substituent, for example, benzyl.

As used in this specification, the term "halo" includes chloro, bromo, iodo, and fluoro.

Unless otherwise specified in this specification the term "alkyl" used herein refers to a "C₁~C₆ alkyl," and the "C₁~C₆ alkyl" refers to a linear or branched alkyl having 1 to 6 carbon atoms, and includes methyl, ethyl, propyl, isopropyl, butyl, isobutyl, sec-butyl, tert-butyl, amyl, hexyl, and the like, but the present invention is not limited thereto. Here, ethyl, propyl, isopropyl, butyl, isobutyl, sec-butyl, and tert-butyl are preferred.

Unless otherwise specified in this specification the term "alkoxy" used herein refers to a "C₁~C₆ alkoxy," and "C₁~C₆ alkoxy" refers to a linear or branched alkoxy having 1 to 6 carbon atoms, and includes methoxy, ethoxy, propoxy, isopropoxy, butoxy groups, and the like, but the present invention is not limited thereto.

Unless otherwise specified in this specification the term "alkenyl" used herein refers to a "C₂~C₆ alkenyl," and the "C₂~C₆ alkenyl" refers to a linear or branched alkenyl having 2 to 6 carbon atoms and containing one double bond, and includes vinyl, propenyl, butenyl, isobutenyl, pentenyl, hexenyl, and the like, but the present invention is not limited thereto.

Unless otherwise specified in this specification the term "alkynyl" used herein refers to a "C₂~C₆ alkynyl," and the "C₂∼C₆ alkynyl" refers to a linear or branched alkynyl having 2 to 6 carbon atoms and containing one triple bond, and includes ethynyl, propynyl, butynyl, isobutynyl, pentynyl, hexynyl, and the like, but the present invention is not limited thereto.

Unless otherwise specified in this specification the term "cycloalkyl" used herein refers to a "C₃~C₁₀ cycloalkyl," and the "C₃~C₁₀ cycloalkyl" refers to a cyclic alkyl having 3 to 10 carbon atoms in the ring thereof, and includes cyclopropyl, cyclobutyl, cyclopentyl, cyclohexyl, cycloheptyl, cyclooctyl,cyclodecyl, and the like. The terms "C₃~C₈ cycloalkyl," "C₃∼C₇ cycloalkyl," and "C₃~C₆ cycloalkyl" have similar connotations. The cycloalkyl group may have a ring substituted with an alkyl group, thereby obtaining cyclopropylmethyl, and the like.

Unless otherwise specified in this specification the term "aryl" used herein refers to a "C₆ to C₁₂ aryl group," and the "C₆~C₁₂ aryl group" refers to phenyl, biphenyl, or naphthyl having 6 to 12 carbon atoms and containing no heteroatom in the ring thereof. Preferably, the aryl is phenyl. The term includes both substituted and unsubstituted moieties. The "substituted aryl" may be substituted with one or more substituents including unprotected or protected hydroxyl, a halogen, amino, an alkylamino, an arylamino, a C₁~C₆ alkyl, a C₁~C₆ alkoxy, a C₃~C₆ cycloalkyl, a C₁~C₆ haloalkyl, a C₁~C₆ haloalkoxy, oxo, aryloxy, nitro, cyano, sulfonic acid, sulfate, phosphoric acid, phosphate, or phosphonate, when necessary, but the present invention is not limited thereto. The term "C₆~C₁₀ aryl group" has a similar connotation.

The term "heteroaryl" or "heterocyclic" used in this specification refers to a "3- to 12-membered heterocyclic group," and the term "3- to 12-membered heterocyclic" refers to a saturated or unsaturated 3- to 12-membered ring group containing a heteroatom selected from oxygen, sulfur, and nitrogen in the ring thereof. Thus, the heterocyclic group is, for example, dioxol. The term "3- to 10-membered heterocyclic" has a similar connotation. In this specification, the "substituted heteroaryl" may be substituted with one or more substituents including unprotected or protected hydroxyl, a halogen, amino, an alkylamino, an arylamino, a C₁~C₆ alkyl, a C₁~C₆ alkoxy, a C₃~C₆ cycloalkyl, a C₁~C₆ haloalkyl, a C₁~C₆ haloalkoxy, oxo, an aryloxy, nitro, cyano, sulfonic acid, sulfate, phosphoric acid, phosphate, or phosphonate, when necessary, but the present invention is not limited thereto.

The term "alkylene" used in this specification refers to a divalent hydrocarbyl group. In this case, the alkylene may be linked to two other groups because the alkylene is divalent. In general, it is -(CH₂)n-, wherein n is in a range of 1 to 8, desirably n is in a range of 1 to 4. In certain cases, the alkylene may also be substituted with other groups and may have different lengths, and open valences does not necessarily need to be on opposite sides of the chain.

In general, any alkyl, alkenyl, alkynyl, aryl, alkylaryl, or arylalkyl included in the substituent may itself be optionally substituted with other additional substituents. Unless otherwise described herein, the nature of these substituents is similar to that recited for the primary substituents themselves.

The term "heteroatom" used in this specification refers to an atom other than carbon or hydrogen, for example, nitrogen, oxygen or sulfur. When the heteroatom is a portion of the backbone or skeleton of a chain and a ring, the heteroatom should be at least divalent, and may be generally selected from N, O, P, and S.

The term "may be substituted" or "substituted" used in this specification indicates that a certain group(s) said to be optionally substituted may not have non-hydrogen substituents, or that the certain group(s) may have one or more non-hydrogen substituents whose chemical and pharmacological activities correspond to those of the resulting molecules. Unless otherwise stated, the total number of these substituents that may be present in the certain group(s) is identical to the total number of hydrogen atoms present in an unsubstituted form of the group(s) being described; and may be present in a smaller number than the maximum number of these substituents. When an optional substituent is bound through a double bond, for example, carbonyl oxygen (C = O), this group takes two available valences on carbon atoms to which the optional substituent is attached. Therefore, the total number of the substituents that may be included in the group decreases with the number of available valences. In this specification, the term "substituted" indicates that one or more hydrogen atoms are each independently replaced by substituent(s) which are the same or different from each other when the term is used to modify specified groups, moieties, or radicals, whether it is used as a portion of the term "may be substituted" or used otherwise.

Because the compounds described in this specification may have one or more chiral centers and/or double bonds, the compounds may exist as stereoisomers, for example double-bond isomers (i.e., geometrical isomers, for example, E and Z), enantiomers, or diastereomers. Also, the present application encompasses each isolated stereoisomeric form (for example, enantiomerically pure isomers, E and Z isomers, and other alternatives to the stereoisomers) and mixtures of stereoisomers having different chiral purities and E and Z percentages (unless otherwise specified as certain stereoisomers). Therefore, the chemical structures described in the present application encompass all possible enantiomers and stereoisomers of the described compounds, including stereoisomerically pure forms (for example, geometrically pure, enantiomerically pure, or diastereomerically pure forms), enantiomeric mixtures and stereoisomeric mixtures. The enantiomeric mixtures and the stereoisomeric mixtures may be divided into their corresponding enantiomeric or stereoisomeric components using separation technology or chiral synthesis technology well-known in the art. The present application also encompasses the individually isolated stereoisomeric forms, and a mixture of stereoisomers having different chiral purities, including a racemic mixture. The present application also encompasses various diastereomers. Other structures may appear to depict a certain isomer, but this is only for the sake of convenience. Therefore, it should be understood that the present application is not intended to be limited to such isomers specifically described as such. When a chemical name does not specify the isomeric form of a compound, it refers to all possible isomeric forms of the compound, or a mixture thereof.

Also, the words "comprise," "comprising," "include," "including," "encompass," and "encompassing" used in this specification and in the following claims are intended to specify the presence of stated features, integers, components, or steps, but do not preclude the presence or addition of one or more other features, integers, components, steps, or groups.

Hereinafter, exemplary embodiments of the present application will be described.

According to one embodiment of the present invention, there is provided a compound represented by the following Formula 1, or a pharmaceutically acceptable salt thereof.

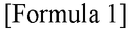

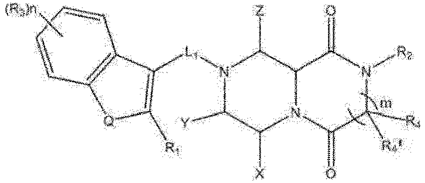

The compound of Formula 1 may be represented by the following Formula 2.

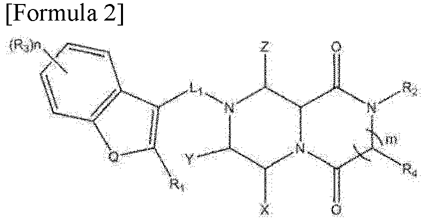

The compound of Formula 1 may be represented by the following Formula 3, Formula 4, Formula 5, or Formula 6.

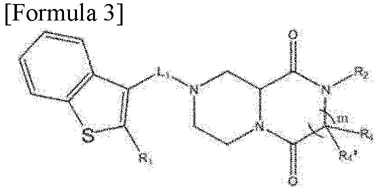

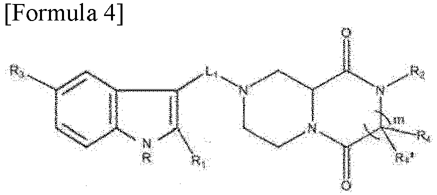

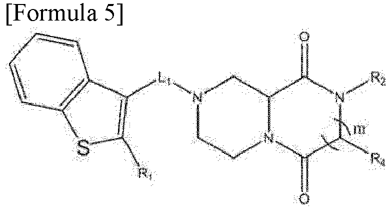

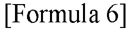

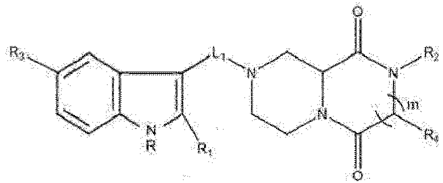

In Formulas 1 to 6, R₁ is hydrogen, a linear or branched alkyl, an alkoxy, a haloalkyl, a haloalkoxy, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₃~C₆ cycloalkyl, a C₁~C₆ haloalkyl, a C₁~C₆ haloalkoxy, oxo, cyano, an unsubstituted or substituted aryl, or an unsubstituted or substituted heteroaryl.

Specifically, R₁ is hydrogen, a C₁~C₆ linear alkyl, a C₃~C₆ branched alkyl, a C₁~C₆ alkoxy, a C₁~C₆ haloalkyl, a C₁~C₆haloalkoxy, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₃~C₆ cycloalkyl, a C₁~C₆ haloalkyl, a C₁~C₆ haloalkoxy, oxo, cyano, an unsubstituted or substituted C₆~C₁₀ aryl, or an unsubstituted or substituted C₅∼C₁₀ heteroaryl.

More specifically, R₁ is hydrogen, an unsubstituted or substituted C₁~C₆ linear alkyl, unsubstituted or substituted benzyl, or unsubstituted or substituted 2-phenylethyl, wherein the substituted C₁~C₆ linear alkyl may be substituted with a C₃~C₆ cycloalkyl, and the substituted benzyl or the substituted 2-phenylethyl may be substituted with halo at least one of the ortho, meta, and para positions, or may be substituted with phenyl.

Even more specifically, R₁ is hydrogen, a C₁~C₆ linear alkyl, a C₃~C₆ branched alkyl, a C₃~C₆ cycloalkyl, 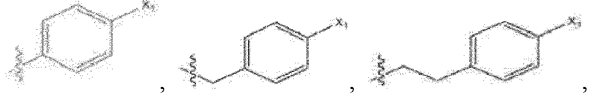 or 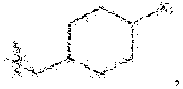 wherein X₁ may be hydrogen, halo, a C₁~C₃ linear alkyl, CF₃, CN, NO₂, or an aryl.

In Formulas 1 to 6, R₃ is hydrogen, a C₁~C₆ linear alkyl, a C₃~C₆ branched alkyl, a C₃~C₆ cycloalkyl, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted C₆~C₁₀ aryl, or an unsubstituted or substituted C₅∼C₁₀ heteroaryl.

Specifically, R₃ is hydrogen, an unsubstituted or substituted C₁~C₆ linear alkyl, an unsubstituted or substituted C₁~C₆ alkoxy, a halogen, or unsubstituted or substituted 2-phenylethyl, wherein the substituted 2-phenylethyl may be substituted with halo at least one of the ortho, meta, and para positions, and the substituted C₁~C₆ alkoxy may be substituted with phenyl or benzyl.

More specifically, R₃ is hydrogen, an unsubstituted or substituted C₁~C₆ linear alkyl, an unsubstituted or substituted C₁~C₆ alkoxy, a halogen, or unsubstituted or substituted 2-phenylethyl, wherein the substituted 2-phenylethyl may be substituted with halo at least one of the ortho, meta, and para positions, and the substituted C₁~C₆ alkoxy may be substituted with phenyl or benzyl.

Even more specifically, R₃ is hydrogen, a C₁~C₃ linear alkyl, an unsubstituted or substituted C₁~C₃ alkoxy, a halogen, 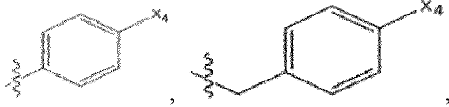 , or 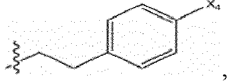 wherein X₄ is hydrogen or halo, and the substituted C₁~C₃ alkoxy may be substituted with phenyl or benzyl.

In Formulas 1 to 6, R₂ is hydrogen, a linear alkyl, a cycloalkyl, an alkoxy, a haloalkoxy, a haloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl.

R₂ is specifically hydrogen, a C₁~C₆ linear alkyl, a C₃~C₆ cycloalkyl, a C₁~C₆ alkoxy, a C₁~C₆ haloalkoxy, a C₁~C₆ haloalkyl, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted C₆~C₁₀ aryl, an unsubstituted or substituted C₅∼C₁₀ heteroaryl, oxo, nitro, cyano, or trifluoromethyl.

R₂ is more specifically hydrogen, an unsubstituted or substituted C₁~C₆ linear alkyl, unsubstituted or substituted benzyl, or unsubstituted or substituted 2-phenylethyl, wherein the substituted linear alkyl is substituted with halo, the substituted benzyl is substituted with halo, a C₁~C₃ linear alkyl, trifluoromethyl, cyano, nitro, a C₁~C₃ alkoxy, or an aryl at least one of the ortho, meta, and para positions, and the 2-phenylethyl is substituted with halo at least one of the ortho, meta, and para positions.

Even more specifically, R₂ is hydrogen, an unsubstituted or substituted C₁~C₆ linear alkyl, an unsubstituted or substituted C₁~C₃ alkoxy, 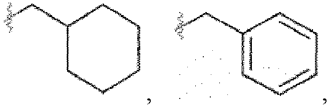 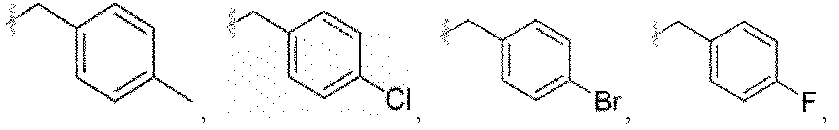 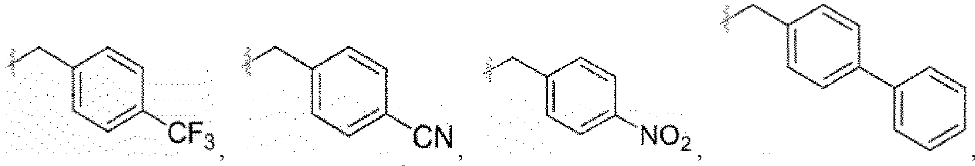 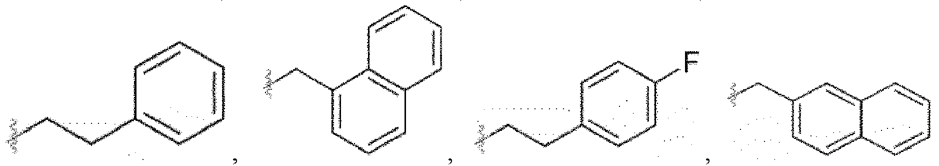 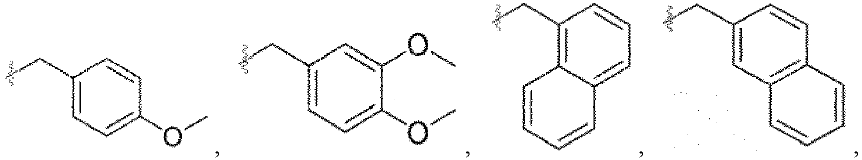 or 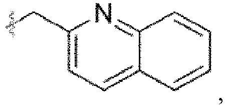 wherein the substituted C₁~C₆ linear alkyl is substituted with halo.

In Formulas 1 to 6, R₄ and R₄' are each independently hydrogen, a linear or branched alkyl, a cycloalkyl, an alkenyl, an alkynyl, an alkylthio, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl.

Specifically, R₄ and R₄' are each independently hydrogen, a C₁~C₆ linear alkyl, a C₃~C₆ branched alkyl, a C₃~C₆ cycloalkyl, a C₂∼C₆ alkenyl, a C₂∼C₆ alkynyl, a C₁~C₆ alkylthio, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted C₆~C₁₀ aryl, an unsubstituted or substituted C₅∼C₁₀ heteroaryl, oxo, nitro, cyano, or trifluoromethyl.

More specifically, R₄ and R₄' are each independently hydrogen, an unsubstituted or substituted C₁~C₆ linear alkyl, a C₃~C₆ branched alkyl, a C₃~C₆ cycloalkyl, a C₂∼C₆ alkynyl, a C₁~C₆ alkylthio, unsubstituted or substituted benzyl, 2-naphthylmethyl, or 1-naphthylmethyl, wherein the substituted linear alkyl may be substituted with methylthio, alkynyl, or benzyloxycarbonylamino, and the substituted benzyl may be substituted with halo, aryl, nitro, cyano, a C₁~C₃ alkoxy, trifluoromethyl, or benzyloxy at least one of the ortho, meta, and para positions.

Even more specifically, R₄ and R₄' are each independently hydrogen, an unsubstituted or substituted C₁~C₄ linear alkyl, a C₃~C₄ branched alkyl, a C₃~C₆ cycloalkyl, 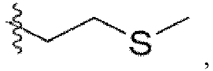 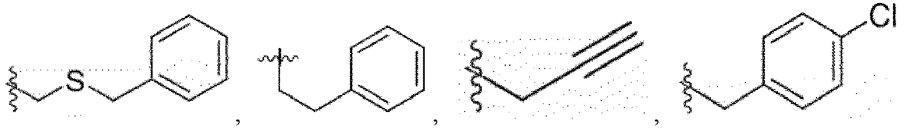 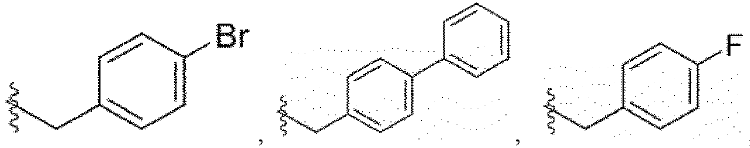 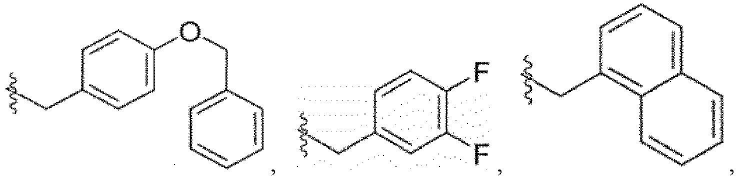 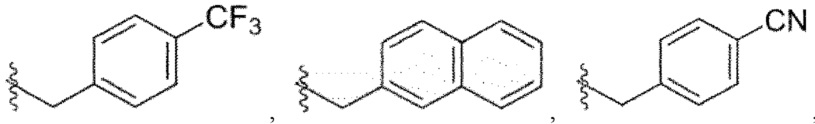 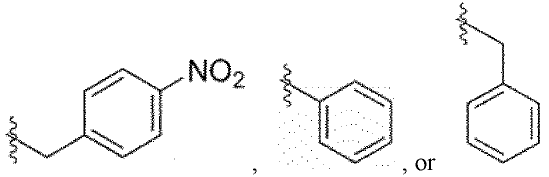 wherein the substituted C₁~C₄ linear alkyl is substituted with a C₃~C₆ cycloalkyl.

R₂ and R₄ may be connected to form a 5- to 10-membered monocyclic or bicyclic ring, wherein R₄' may be hydrogen.

In Formulas 1 to 6, L₁ is a C₁~C₁₀ alkylene, wherein the alkylene may be substituted with at least one selected from a C₁~C₆ alkyl, a C₃~C₆ cycloalkyl, a C₁~C₆ alkoxy, hydroxyl, oxo, or a halogen, and the C₁~C₆ alkyl, the C₃~C₆ cycloalkyl, and C₁~C₆ alkoxy may be substituted with an unsubstituted or substituted aryl. Specifically, L₁ is a C₁~C₄ alkylene, wherein the alkylene may be substituted with hydrogen, oxo, hydroxyl, a C₁~C₄ alkoxy, or a C₁~C₄ alkyl, and the C₁~C₄ alkoxy may be substituted with benzyl. More specifically, L₁ is a C₁~C₄ alkylene, wherein alkylene may be substituted with hydrogen, oxo, hydroxyl, a C₁~C₄ alkyl, or

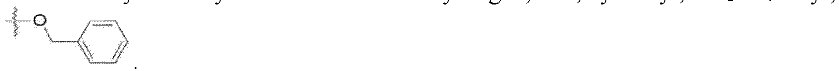

In Formula 1, Q may be S, Se, NR, P, P(O), P(O)₂, or P(O)OR.

In Formulas 1 to 6, R is hydrogen, a linear or branched alkyl, a cycloalkyl, a bi- or tri-cycloalkyl, an alkoxy, a haloalkyl, a haloalkoxy, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted aryl, or an unsubstituted or substituted heteroaryl. Specifically, R may be hydrogen, an unsubstituted or substituted C₁~C₄ linear alkyl, 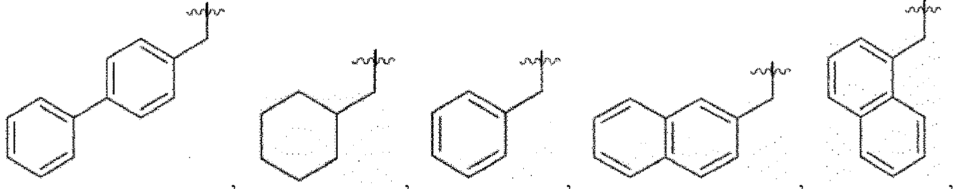 or

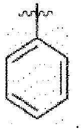

In Formulas 1 to 6, m may be an integer of 1 or 2, and n may be an integer ranging from 1 to 4. Specifically, m and n may be 1.

In Formula 1, X, Y, and Z are each independently hydrogen, a linear or branched alkyl, a cycloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl.

The compound according to the present application may include one or more compounds selected from the group consisting of Formulas of Table 1 below.

**[Table 1]**

| ID No. | Formula | Structure |
|---|---|---|
| S101 | 7 | 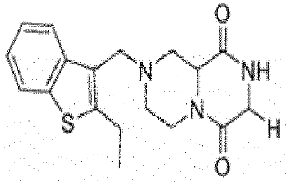 |
| S102 | 8 | 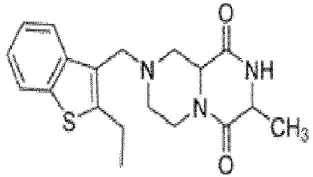 |
| S103 | 9 | 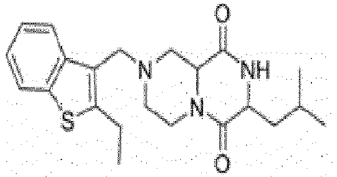 |
| S105 | 10 | 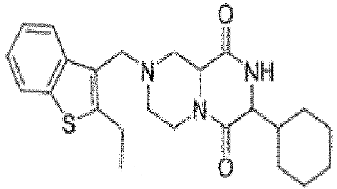 |
| S106 | 11 | 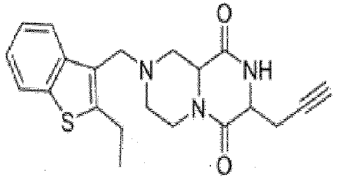 |
| S107 | 12 | 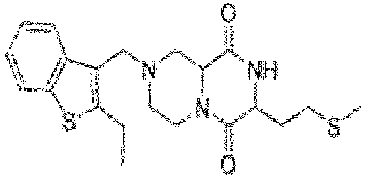 |
| S108 | 13 | 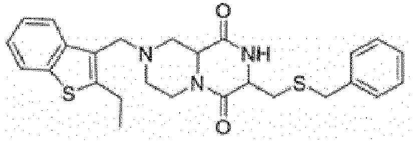 |
| S109 | 14 | 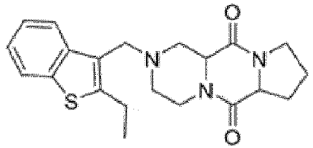 |
| S111 | 15 | 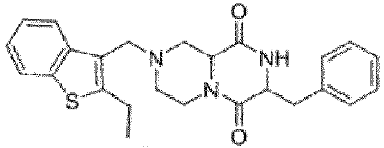 |
| S112 | 16 | 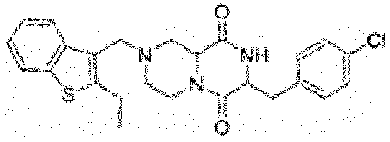 |
| S113 | 17 | 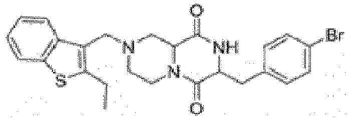 |
| S114 | 18 | 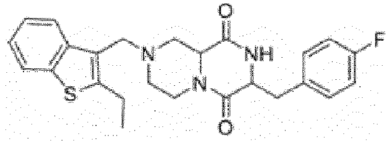 |
| S115 | 19 | 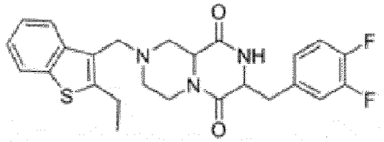 |
| S116 | 20 | 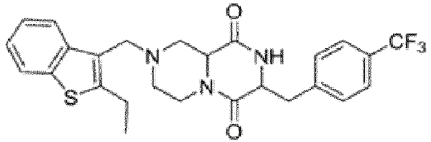 |
| S117 | 21 | 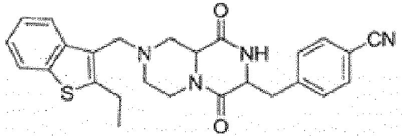 |
| S118 | 22 | 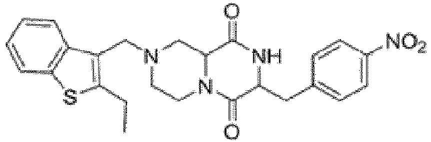 |
| S119 | 23 | 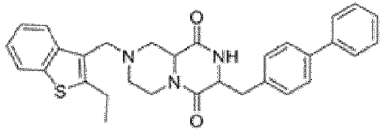 |
| S120 | 24 | 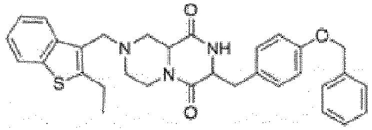 |
| S121 | 25 | 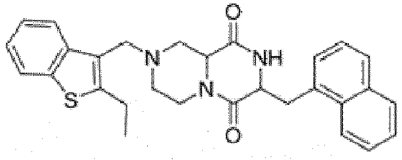 |
| S122 | 26 | 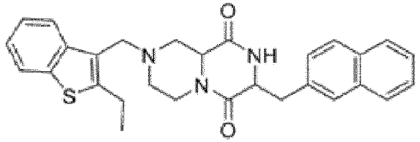 |
| S126 | 27 | 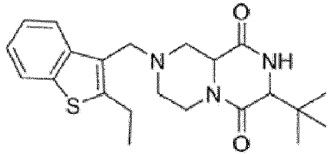 |
| S127 | 28 | 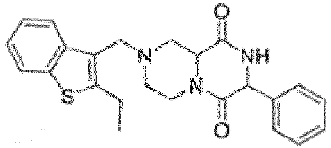 |
| S128 | 29 | 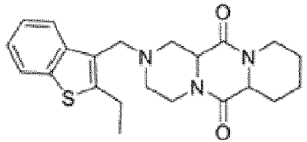 |
| S135 | 30 | 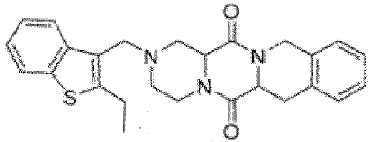 |
| S136 | 31 | 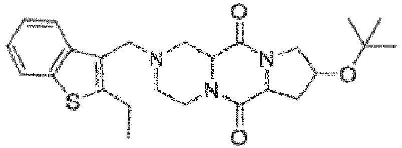 |
| S141 | 32 | 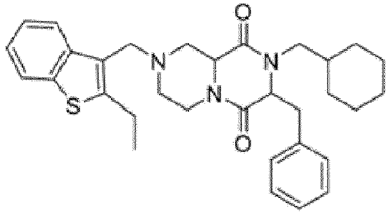 |
| S142 | 33 | 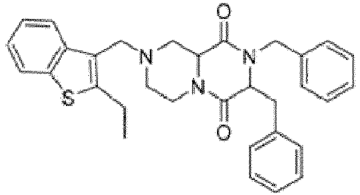 |
| S151 | 34 | 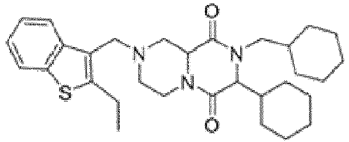 |
| S152 | 35 | 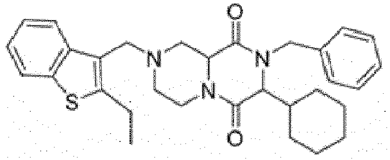 |
| S201 | 36 | 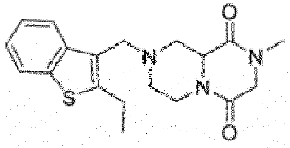 |
| S202 | 37 | 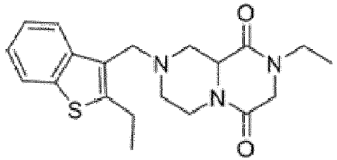 |
| S203 | 38 | 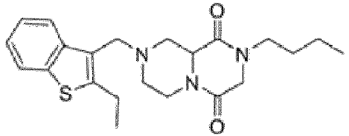 |
| S204 | 39 | 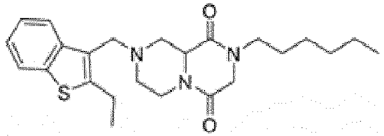 |
| S206 | 40 | 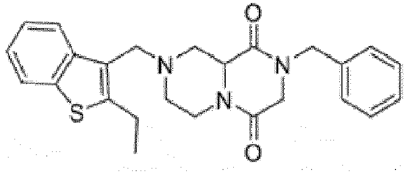 |
| S207 | 41 | 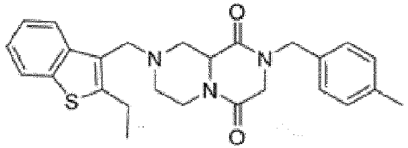 |
| S208 | 42 | 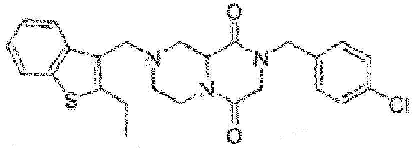 |
| S209 | 43 | 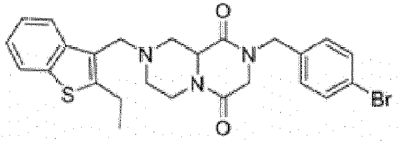 |
| S210 | 44 | 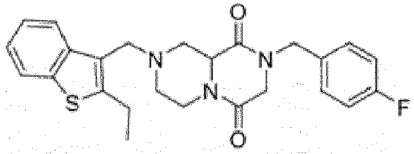 |
| S211 | 45 | 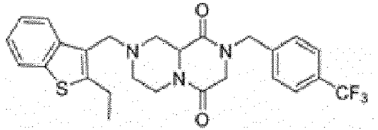 |
| S212 | 46 | 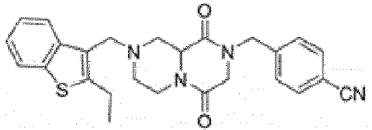 |
| S213 | 47 | 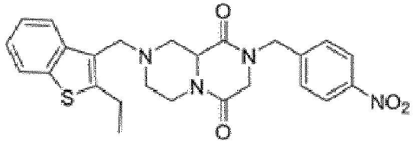 |
| S214 | 48 | 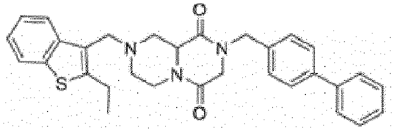 |
| S215 | 49 | 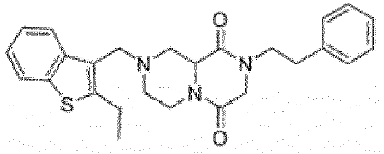 |
| S216 | 50 | 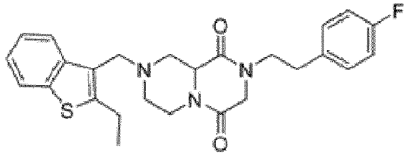 |
| S218 | 51 | 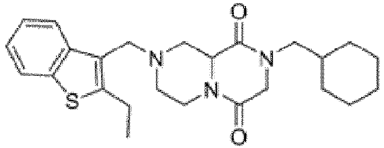 |
| S221 | 52 | 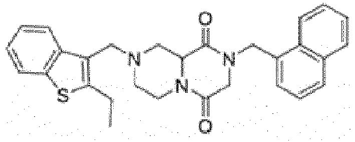 |
| S222 | 53 | 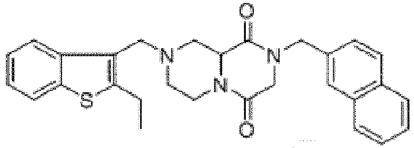 |
| S223 | 54 | 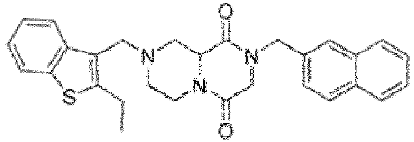 |
| S224 | 55 | 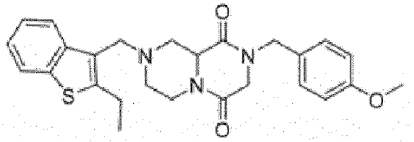 |
| S225 | 56 | 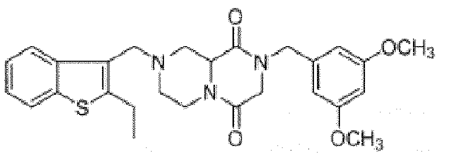 |
| S226 | 57 | 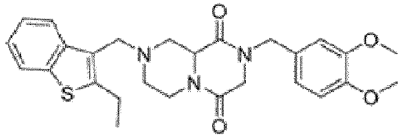 |
| S301 | 58 | 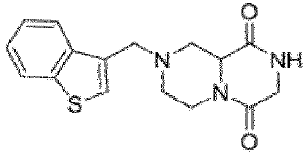 |
| S302 | 59 | 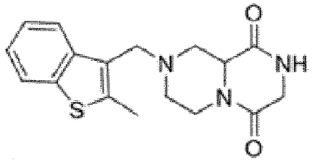 |
| S303 | 60 | 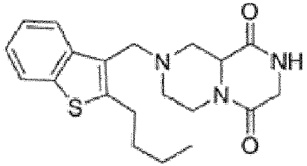 |
| S304 | 61 | 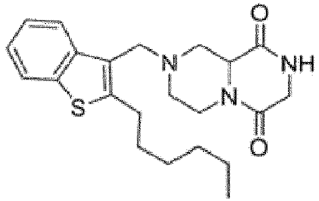 |
| S306 | 62 | 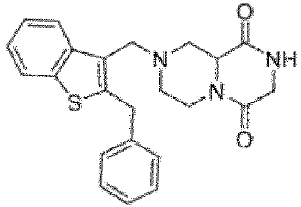 |
| S307 | 63 | 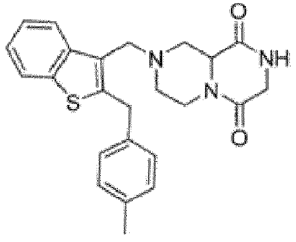 |
| S308 | 64 | 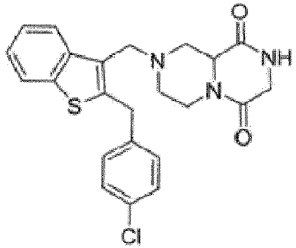 |
| S309 | 65 | 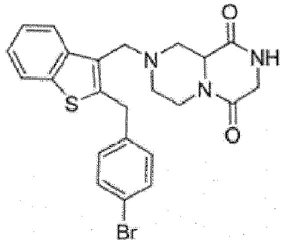 |
| S310 | 66 | 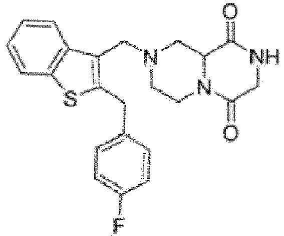 |
| S314 | 67 | 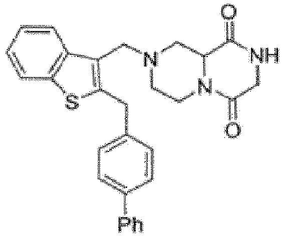 |
| S318 | 68 | 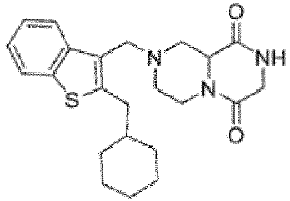 |
| N101 | 69 | 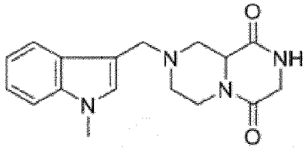 |
| N102 | 70 | 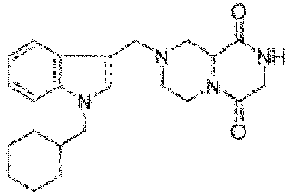 |
| N103 | 71 | 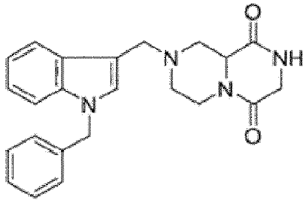 |
| N104 | 72 | 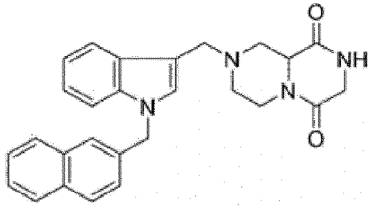 |
| N105 | 73 | 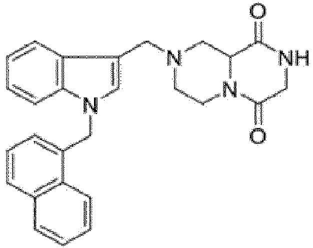 |
| N106 | 74 | 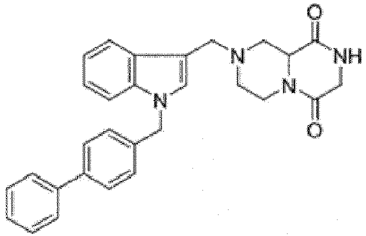 |
| N107 | 75 | 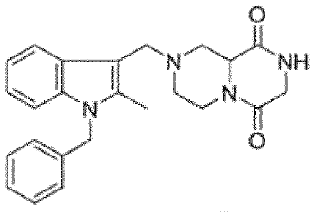 |
| N108 | 76 | 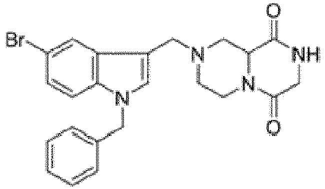 |
| N109 | 77 | 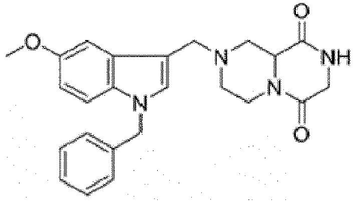 |
| N111 | 78 | 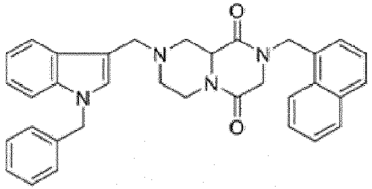 |
| N112 | 79 | 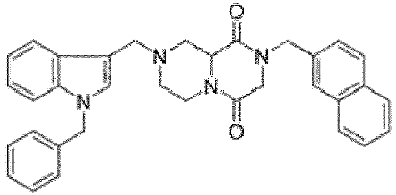 |
| N113 | 80 | 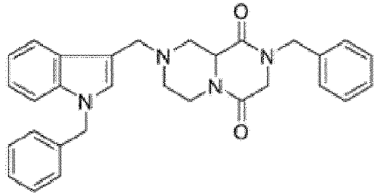 |
| N114 | 81 | 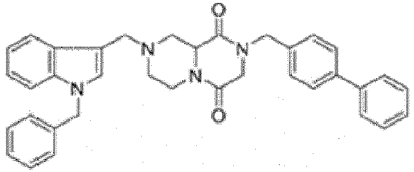 |
| N122 | 82 | 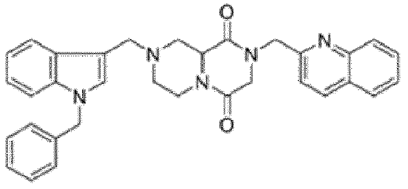 |
| N162 | 83 | 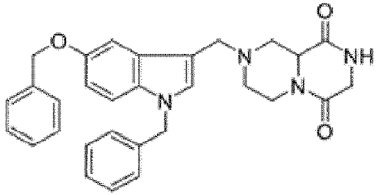 |
| N163 | 84 | 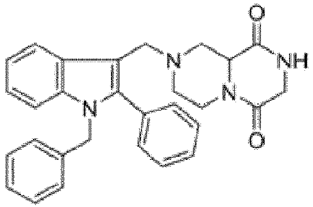 |
| N164 | 85 | 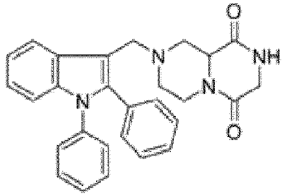 |
| SN611 | 86 | 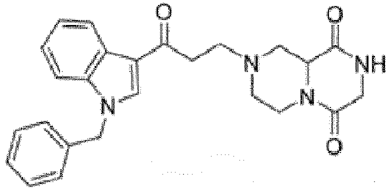 |
| SN612 | 87 | 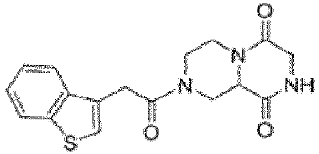 |
| N199 | 88 | 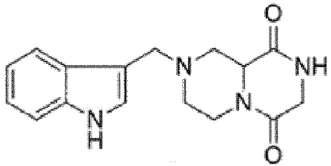 |
| S801 | 89 | 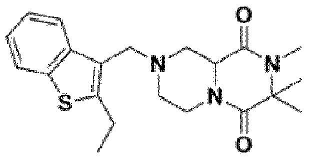 |
| S802 | 90 | 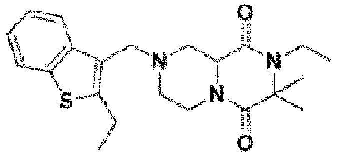 |
| S803 | 91 | 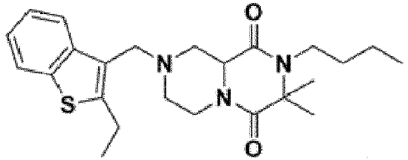 |
| S804 | 92 | 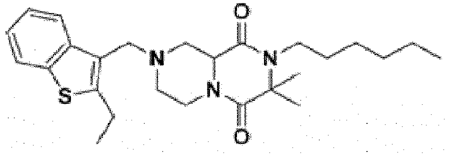 |
| S805 | 93 | 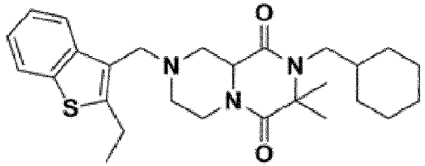 |
| S806 | 94 | 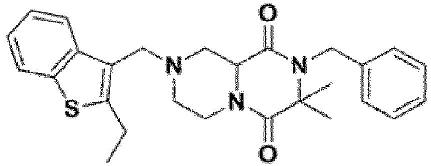 |
| S807 | 95 | 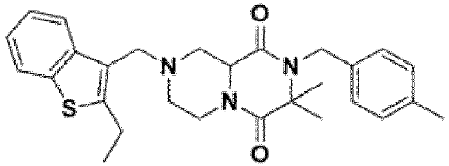 |
| S808 | 96 | 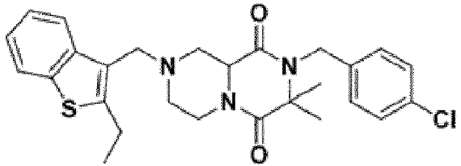 |
| S809 | 97 | 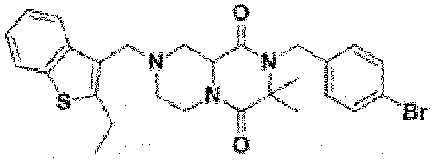 |
| S810 | 98 | 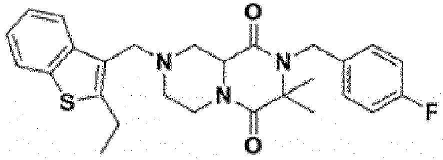 |
| S811 | 99 | 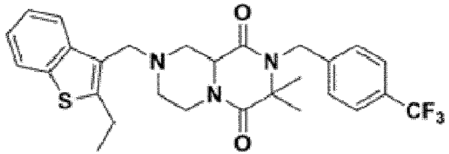 |
| S812 | 100 | 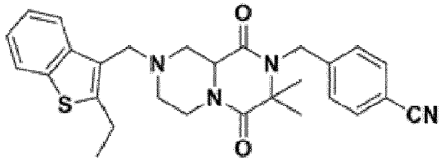 |
| S813 | 101 | 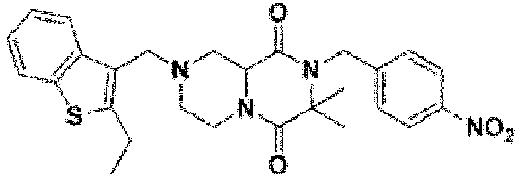 |
| S814 | 102 | 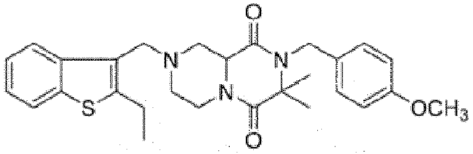 |
| S815 | 103 | 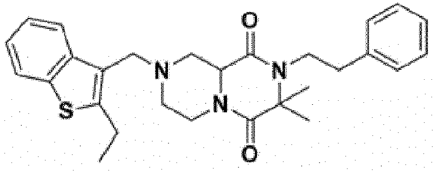 |
| S816 | 104 | 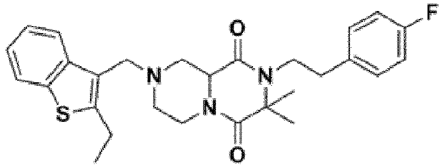 |
| S817 | 105 | 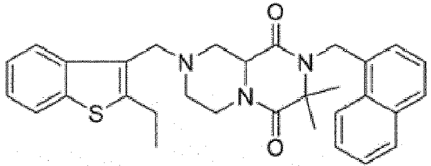 |
| S818 | 106 | 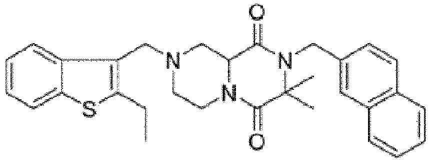 |
| S819 | 107 | 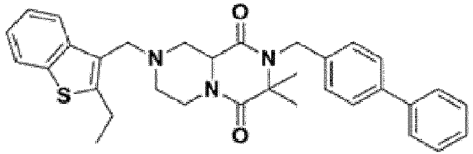 |
| S820 | 108 | 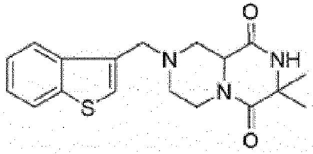 |
| S821 | 109 | 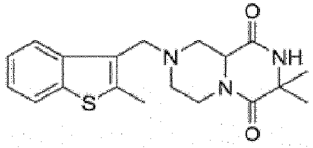 |
| S822 | 110 | 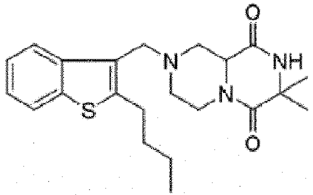 |
| S823 | 111 | 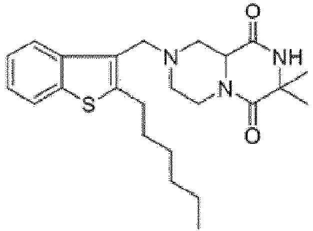 |
| S824 | 112 | 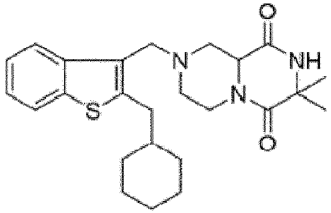 |
| S825 | 113 | 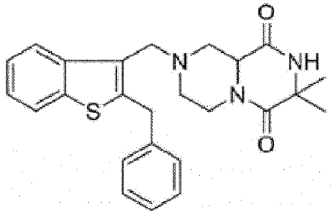 |
| S826 | 114 | 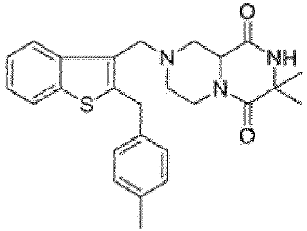 |
| S827 | 115 | 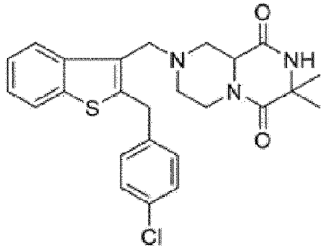 |
| S828 | 116 | 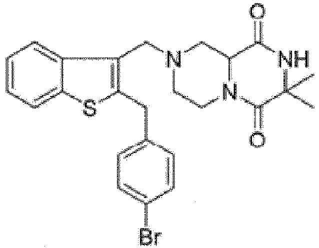 |
| S829 | 117 | 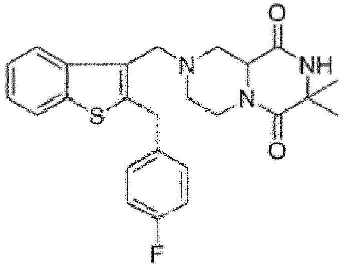 |
| S830 | 118 | 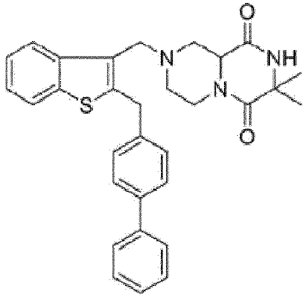 |
| S831 | 119 | 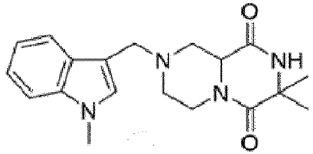 |
| S832 | 120 | 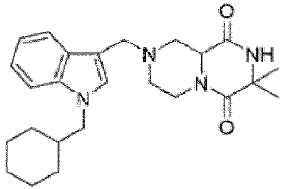 |
| S833 | 121 | 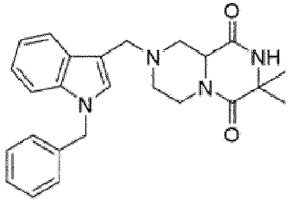 |
| S834 | 122 | 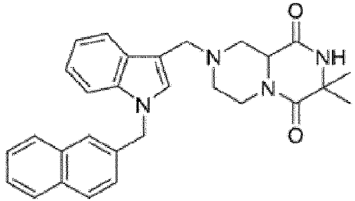 |
| S835 | 123 | 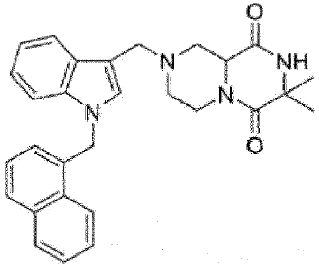 |
| S836 | 124 | 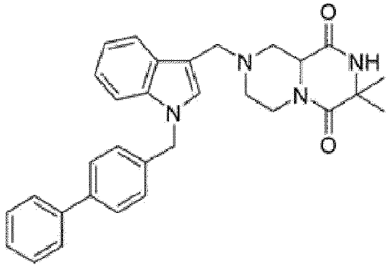 |
| S837 | 125 | 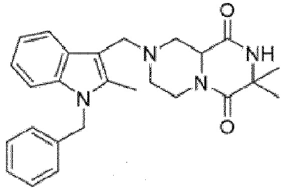 |
| S838 | 126 | 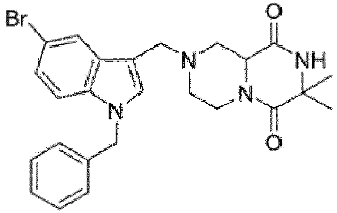 |
| S839 | 127 | 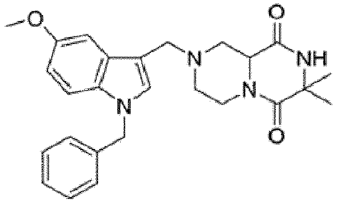 |
| S840 | 128 | 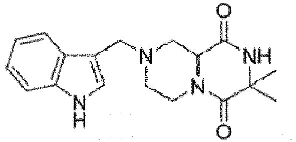 |
| S841 | 129 | 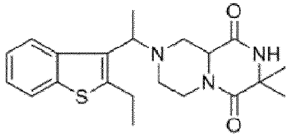 |
| S842 | 130 | 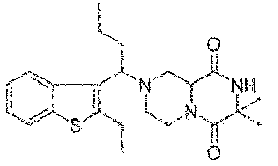 |
| S843 | 131 | 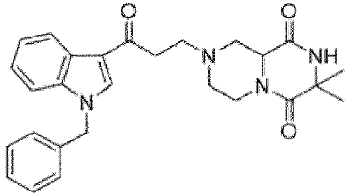 |
| S844 | 132 | 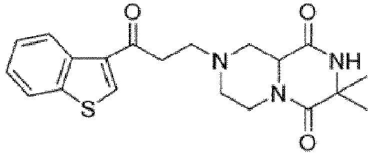 |
| S845 | 133 | 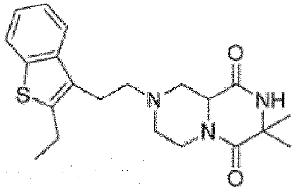 |
| S846 | 134 | 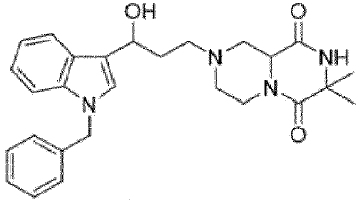 |
| S847 | 134 | 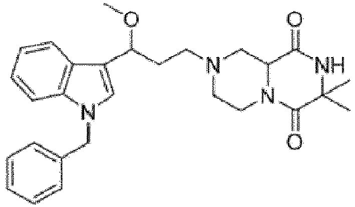 |
| S848 | 135 | 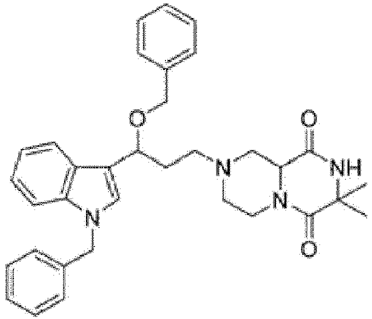 |
| S849 | 136 | 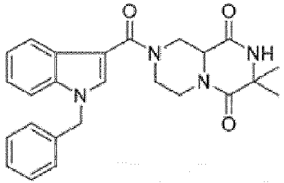 |
| S850 | 137 | 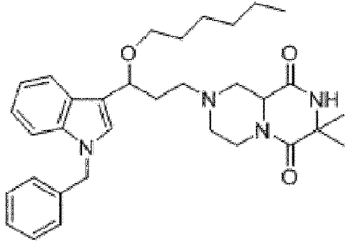 |
| S851 | 138 | 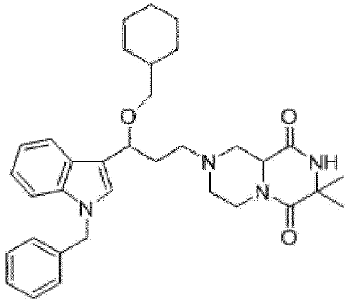 |
| S852 | 139 | 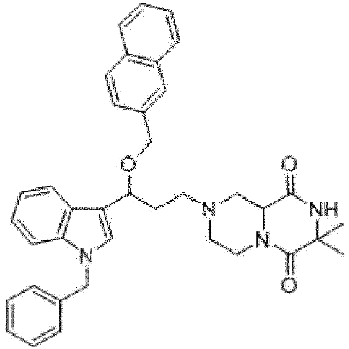 |
| S853 | 140 | 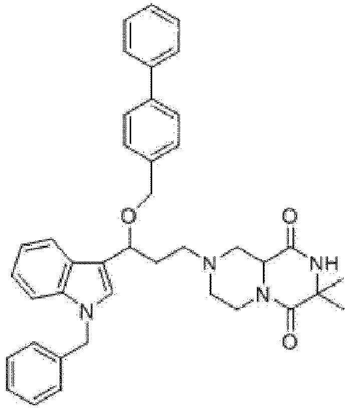 |
| S854 | 141 | 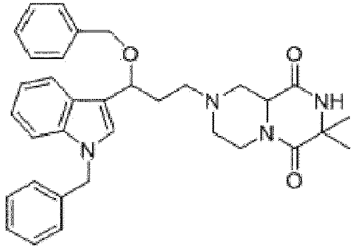 |
| S855 | 142 | 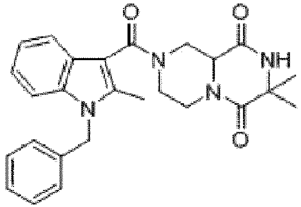 |
| S856 | 143 | 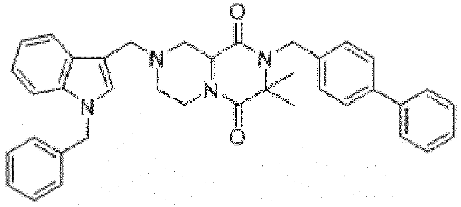 |
| S857 | 144 | 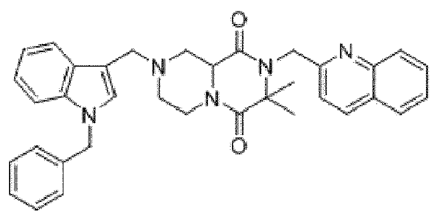 |

The term "treating" or "treatment" used in this specification refers to a therapeutic, prophylactic, or transient remedy, or a prophylactic method. The term "prevention" used in this specification refers to any action of suppressing cancer or delaying the onset of the cancer when the pharmaceutical composition is administered. For the purposes of the present application, beneficial or desired clinical outcomes include, but are not limited to, detectable or undetectable symptom relief, attenuation of the severity of a disease, a stable (i.e., non-attenuated) state of the disease, a slow progression or delay in the progression of the disease, or mitigation, improvement, temporary treatment, or alleviation of the disease or remission (whether partial or total). "Treatment" may also mean prolonging survival as compared to the expected survival without any treatment. The subjects in need of treatment include those already suffering from the condition or disorder, or those in which the condition or disorder has been prevented, as well as those susceptible to suffering the condition or disorder.

The expression "therapeutically effective amount" or "effective amount" used in this specification refers to an amount of a compound of Formula I that is sufficient (i) to treat or prevent a certain disease, condition, or disorder, (ii) to weaken, improve, or alleviate one or more symptoms of the certain disease, condition, or disorder, or (iii) to prevent or delay the onset of one or more symptoms of the certain disease, condition, or disorder as described herein when the compound of Formula I is administered to a mammal in need of such treatment. The amount of the compound corresponding to such an effective amount will depend on factors such as the particular compound, the state of a disease and its severity, a characteristic (e.g., weight) of the mammal in need of treatment. Nevertheless, the amount of the compound may be routinely determined by those skilled in the art.

The terms "cancer" and "cancerous" used in this specification typically refer to or express a physiological condition of a mammal that is characterized by abnormal or uncontrolled cell growth. A "tumor" includes one or more cancerous cells. Examples of the cancer include, but are not limited to, a carcinoma, a lymphoma, a blastoma, a sarcoma, and leukemia, or a malignant lymphoid tumor. More specific examples of such cancer include squamous cell carcinoma (for example, epithelial squamous cell carcinoma), lung cancer (including small-cell lung cancer, non-small cell lung cancer ("NSCLC"), lung adenocarcinoma, and lung squamous carcinoma), peritoneal cancer, hepatocellular cancer, stomach or gastric cancer including stomach cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or nephritic cancer, prostate cancer, vulvar cancer, thyroid cancer, hepatocarcinoma, anal carcinoma, penile carcinoma, skin cancer including melanoma, and head and neck cancer.

The term "pharmaceutically acceptable" used in this specification indicates that the substance or the composition is chemically and/or toxicologically compatible with a mammal treated thereby, and/or other components included in a formulation.

The term "pharmaceutically acceptable salt" used in this specification refers to a pharmaceutically acceptable organic or inorganic salt of the compound according to the present application.

The "pharmaceutically acceptable salt" used in this specification refers to a pharmaceutically acceptable organic or inorganic salt of the compound described in this specification. Exemplary examples of the salt include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate(mesylate), ethanesulfonate, ethanedisulfonate, benzenesulfonate, *p*-toluenesulfonate, and pamoate (i.e., 1,1'-methylene-bis-(2-hydroxy-3-naphthoate)) salts. The pharmaceutically acceptable salt may include an inclusion body of another molecule, for example acetate ions, succinate ions, or other counterions. The counterions may be any organic or inorganic moiety that stabilizes the charge of a parent compound. Furthermore, the pharmaceutically acceptable salt may have more than one charged atom in the structure thereof. When multiple charged atoms are included in a portion of the pharmaceutically acceptable salt, the pharmaceutically acceptable salt may have multiple counterions. Therefore, the pharmaceutically acceptable salt may have one or more charged atoms and/or one or more counterions.

According to one embodiment of the present invention, there is provides a pharmaceutical composition for preventing or treating cancer, which includes the compound represented by Formula 1, or a pharmaceutically acceptable salt, hydrate, solvate, structural isomer, optical isomer, or stereoisomer thereof, or a combination thereof. The compound represented by Formula 1 or a pharmaceutically acceptable salt thereof has an effect of inhibiting cancer activity when administered alone.

According to one embodiment of the present invention, there is provides a pharmaceutical composition for preventing or treating resistant cancer, which includes the compound represented by Formula 1, or a pharmaceutically acceptable salt, hydrate, solvate, structural isomer, optical isomer, or stereoisomer thereof, or a combination thereof. The compound represented by Formula 1 or a pharmaceutically acceptable salt thereof has an effect of inhibiting cancer activity when administered to the resistant cancer in combination with an anticancer drug or radiation therapy.

According to one embodiment of the present invention, the cancer or the resistant cancer may include one or more selected from the group consisting of ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, bile duct cancer, blood cancer, bladder cancer, kidney cancer, melanoma, colon cancer, bone cancer, skin cancer, head and neck cancer, uterine cancer, rectal cancer, brain cancer, anal cancer, fallopian tube carcinoma, endometrial carcinoma, vaginal cancer, vulvar carcinoma, esophagus cancer, small intestine cancer, endocrine carcinoma, adrenal cancer, soft tissue sarcoma, urethral cancer, penile cancer, ureteral cancer, renal cell carcinoma, renal pelvic carcinoma, leukemia, a central nervous system (CNS) tumor, a spinal cord tumor, brainstem glioma, and pituitary adenoma.

The pharmaceutical composition according to one embodiment of the present invention may include the compound represented by Formula 1 or a pharmaceutically acceptable salt thereof and the anticancer drug at a concentration ratio of 1:0.001 to 1:1000 moles, 1:0.01 to 1:100 moles, 1:0.1 to 1:50 moles, or 1:0.1 to 1:20 moles.

The pharmaceutical composition according to one embodiment of the present invention may be in the form of a capsule, a tablet, a granule, an injection, an ointment, a powder, or a beverage.

The pharmaceutical composition according to one embodiment of the present invention may be formulated into the form of an oral formulation (such as a powder, a granule, a capsule, a tablet, an aqueous suspension, and the like), a preparation for external application, a suppository, and an injection, and may be used.

The pharmaceutical composition according to one embodiment of the present invention may include a pharmaceutically acceptable carrier. The pharmaceutically acceptable carrier may be a binder, a lubricant, a disintegrant, an excipient, a solubilizer, a dispersant, a stabilizer, a suspending agent, a pigment or a flavoring agent for oral administration, and in the case of injections, a buffer, a preservative, a pain-relieving agent, a solubilizer, an isotonic agent or a stabilizer may be mixed and used, and for topical administration, a base, an excipient, a lubricant, or a preservative may be used. A formulation of the pharmaceutical composition according to one embodiment of the present invention may be mixed with a pharmaceutically acceptable carrier, and prepared into various forms. For example, for oral administration, the pharmaceutical composition of the present invention may be prepared in the form of a tablet, a troche, a capsule, an elixir, a suspension, a syrup, a wafer, or the like, and may be prepared in a unit dosage ampoule or a multiple dosage form for injection. Also, the formulation of the pharmaceutical composition of the present invention may be prepared as a solution, a suspension, a tablet, a capsule, a sustained-release preparation, and the like.

The carrier, excipient, and diluent for preparation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil, a filler, an anticoagulant, a lubricant, a wetting agent, a flavoring agent, an emulsifier, a preservative, or the like.

One embodiment of the present invention relates to a pharmaceutical composition including the compound represented by Formula 1 of the present invention or a pharmaceutically acceptable salt thereof as an active ingredient in combination with a pharmaceutically applicable adjuvant and/or excipient.

In the present application, the term "adjuvant" refers to a substance that may make, deepen or modify a specific reaction with the active ingredient of the present invention when administered simultaneously or sequentially. For example, known adjuvants for an injectable solution include an aluminum composition (for example, aluminum hydroxide or aluminum phosphate), saponins (for example, QS21), muramyl dipeptide or muramyl tripeptide, proteins (for example, gamma-interferon or TNF), M59, squalene, or a polyol.

The administration route of the pharmaceutical composition according to one embodiment of the present invention may include oral, intravenous, intramuscular, intraarterial, intramedullary, intrathecal, intracardiac, transdermal, subcutaneous, intraperitoneal, intranasal, intestinal, local, sublingual, or rectal administration, but the present invention is not limited thereto.

The pharmaceutical composition according to one embodiment of the present invention may be orally or parenterally administered. For parenteral administration, injection methods such as external use for skin, intraperitoneal injection, rectal injection, subcutaneous injection, intravenous injection, intramuscular injection or intrathoracic injection may be selected.

A dose of the pharmaceutical composition according to one embodiment of the present invention may vary according to a patient's condition and body weight, the severity of a disease, a drug type, a route of administration, and the administration duration, and may be appropriately selected by those of ordinary skill in the art. For example, the pharmaceutical composition according to one embodiment of the present invention may be administered daily at 0.0001 to 1000 mg/kg or 0.001 to 500 mg/kg. The pharmaceutical composition of the present invention may be administered once daily or in divided doses. The dose is not intended to limit the scope of the present invention in any way.

Also, the present application provides a use of the compound represented by Formula 1 or a pharmaceutically acceptable salt thereof for use in the treatment of cancer.

In addition, the present application provides a use of the compound represented by Formula 1 or a pharmaceutically acceptable salt thereof for use in the treatment of resistant cancer.

Additionally, the present application provides a use of the compound represented by Formula 1 or a pharmaceutically acceptable salt thereof for use in the treatment of stem cell-like cancer.

The resistant cancer, and the compound represented by Formula 1 or a pharmaceutically acceptable salt thereof are the same as described above, and thus detailed descriptions thereof will be omitted.

Also, the present application provides a method of treating cancer or resistant cancer, which includes: administering a therapeutically effective amount of the compound represented by Formula 1 or a pharmaceutically acceptable salt, a hydrate, a solvate, a structural isomer, an optical an isomer, or a stereoisomer thereof, or a combination thereof to a subject with resistant cancer. In the case of the administration, chemotherapeutic agents useful for treating cancer or a proliferative disease may be administered simultaneously, separately, or sequentially.

The term "administration" refers to the introduction of a predetermined substance into a subject using a suitable method.

The "subject with resistant cancer" refers to a subject who has developed resistant cancer or has a high possibility of developing resistant cancer and thus is in need of suitable treatment, and may be a subject who has received anticancer therapy, for example, surgical resection therapy, chemotherapy using an anticancer drug, radiation therapy or immunotherapy, but has relapsed due to the resistance thereto.

The subject with resistant cancer may be a human, a cow, a dog, a guinea pig, a rabbit, a chicken, an insect, or the like. Specifically, the subject with resistant cancer may be a mammal such as a human, a cow, a horse, a dog, a sheep, a goat, a cat, or the like. The subject may be a subject who developed cancer or has a high possibility of developing cancer.

Also, the present application provides a radiation treatment method, which includes: administering the above-described compound represented by Formula 1 or a pharmaceutically acceptable salt thereof into a subject with resistant cancer; and irradiating the subject with radiation.

The resistant cancer, the subject with resistant cancer, and the compound represented by Formula 1 or a pharmaceutically acceptable salt thereof are the same as described above, and detailed descriptions thereof will be omitted.

For radiation irradiation, any irradiation method conventionally used for radiation treatment of cancer or a radiation irradiation method, which will be developed in the future, may be applied.

When the administration of the compound represented by Formula 1 according to the present application or a pharmaceutically acceptable salt thereof is used in combination with radiation irradiation, a synergistic effect on the inhibition of the growth of cancer cells or cancer stem cells and/or induction of cell death may be imparted, thereby not only effectively preventing or treating cancer, but also preventing resistance to radiation or the metastasis or recurrence of cancer.

### [Mode for Invention]

### Preparation Example

Compounds listed in Table 1 were prepared using the method described in the following Preparation Example.

### [Formula 7]

### 2-((2-ethylbenzothiophene-3-yl)methyl)hexahydro-2H-pyrazino[1,2-a]pyrazine-6,9-dione was prepared through a 10-step reaction as described below. A synthesis method in each step was described in detail, as follows.

### 1) Step 1

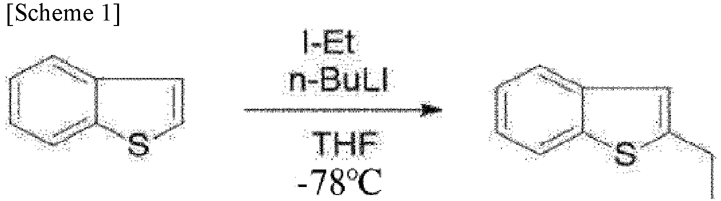

As in Scheme 1, benzothiophene (1.0 equivalent) was cooled to -78°C in the presence of a tetrahydrofuran solvent, and a solution of 2.5M n-butyllithium (1.2 equivalents) was added. The resulting mixture was stirred for an hour while being maintained at -78°C, and ethyl iodide (2.0 equivalents) was added dropwise, and stirred at 0°C for an hour. The completion of the reaction was confirmed, and the reaction was terminated using an aqueous ammonium chloride solution and ethyl acetate. An organic layer was washed with distilled water, dried over anhydrous magnesium sulfate, and then concentrated under reduced pressure. The residue was purified by silica chromatography to obtain 2-ethyl benzothiophene.

### 2) Step 2

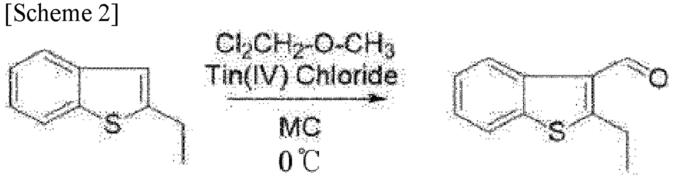

2-ethyl benzothiophene (1.0 equivalent) was added to methylene chloride (MC), and cooled to 0°C, and while maintaining 0°C, tin(IV) chloride (1.5 equivalents) and dichloromethyl methyl ether (1.5 equivalents) were sequentially added, and then stirred for an hour. The completion of the reaction was confirmed, and the reaction was terminated using an aqueous ammonium chloride solution and methylene chloride. An organic layer was washed with distilled water, dried over anhydrous magnesium sulfate, and concentrated under reduced pressure. The residue was purified by silica chromatography to obtain 2-ethyl-1-benzothiophene-3-carbaldehyde.

### 3) Step 3

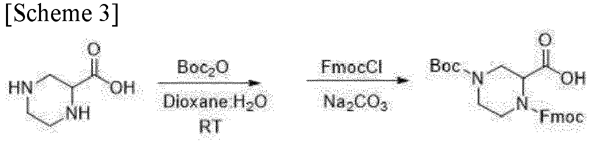

Piperazine-2-carboxylic acid (1.0 equivalent), dioxane, and distilled water were added, and cooled to 5°C or lower. Thereafter, di-tert-butyl dicarbonate (1.1 equivalents) was added while maintaining 10°C or lower. The resulting mixture was stirred at room temperature for 5 hours, and sodium carbonate (1.1 equivalents) was then added thereto, and stirred for 5 minutes. 9-Fluorenylmethyl chloroformate (1.2 equivalents) was added, stirred overnight, and then concentrated under reduced pressure. Ethyl acetate and 1 M hydrochloric acid were added to the residue, and the pH of the resulting mixture was then adjusted to pH 2 to 3. Then, the mixture was vigorously stirred until the residue was completely dissolved. An organic layer was washed with brine, dried over anhydrous magnesium sulfate, and then concentrated under reduced pressure. The organic layer was recrystallized with EA to obtain 1-(((9H-fluoren-9-yl)methoxy)carbonyl)-4-(tert-butoxycarbonyl)piperazine-2-carboxylic acid.

### 4) Step 4

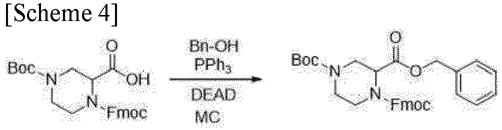

An anhydrous methylene chloride solution of 1-(((9H-fluoren-9-yl)methoxy)carbonyl)-4-(tert-butoxycarbonyl)piperazine-2-carboxylic acid, benzyl alcohol (1.02 equivalents), and triphenylphosphine (1.02 equivalents) was cooled to 0°C in the stream of nitrogen. Diethyl azodicarboxylate (DEAD) (1.02 equivalents) was added and stirred for an hour while maintaining 0°C or lower. Water was added thereto, and the resulting mixture was vigorously stirred for 20 minutes. Thereafter, an organic layer was dried over anhydrous magnesium sulfate, and then concentrated under reduced pressure. The residue was purified by silica chromatography to obtain 1-((9H-fluoren-9-yl)methyl) 2-benzyl 4-(tert-butyl) piperazine-1,2,4-tricarboxylate.

### 5) Step 5

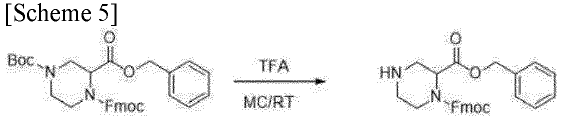

As in Scheme 5, trifluoroacetic acid (TFA) (2.0 volumes) was added to a methylene chloride (5.0 volumes) solution of 1-((9H-fluoren-9-yl)methyl) 2-benzyl 4-(tert-butyl) piperazine-1,2,4-tricarboxylate at room temperature, and stirred at room temperature for 30 minutes. The resulting mixture was neutralized with sodium bicarbonate, and then extracted with methylene chloride, and an organic layer was washed with brine. The organic layer was dried over anhydrous magnesium sulfate, and concentrated to obtain 1-((9H-fluoren-9-yl)methyl) 2-benzyl piperazine-1,2-dicarboxylate.

### 6) Step 6

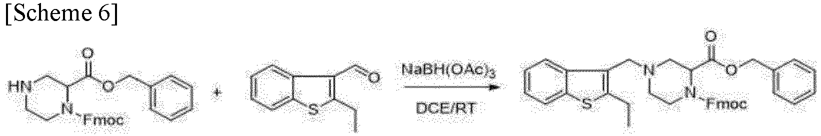

The 2-ethyl-1-benzothiophene-3-carbaldehyde (1.0 equivalent) obtained in Step 2 and the 1-((9H-fluoren-9-yl)methyl) 2-benzyl piperazine-1,2-dicarboxylate(1.2 equivalents) obtained in Step 5 were added to 1,2-dichloroethane (DCE) at room temperature, and then stirred for 30 minutes. Sodium triacetoxyborohydride (3.0 equivalents) was added thereto, and the resulting mixture was stirred overnight at room temperature. Methylene chloride and water were added to the reaction solution, and vigorously stirred for 20 minutes. The separated organic layer was washed with brine, dried over anhydrous magnesium sulfate, and concentrated to obtain 1-((9H-fluoren-9-yl)methyl) 2-benzyl 4-((2-ethylbenzothiophen-3-yl)methyl)piperazine- 1,2-dicarboxylate.

### 7) Step 7

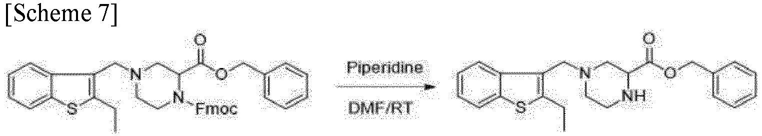

At room temperature, 1-((9H-fluoren-9-yl)methyl) 2-benzyl 4-((2-ethylbenzothiophene-3-yl)methyl)piperazine-1,2-dicarboxylate (1.0 equivalent) was dissolved in dimethylformamide, and piperidine (10 equivalents; 25% piperidine in a solvent) was then added thereto, and stirred for an hour. After ethyl acetate was added to the reaction, piperidine in the solution was washed out using an aqueous ammonium chloride solution. The resulting reaction solution was dried over anhydrous magnesium sulfate, and then concentrated under reduced pressure. Thereafter, the resulting residue was purified by silica chromatography to obtain benzyl 4-((2-ethylbenzothiophene-3-yl)methyl)piperazine-2-carboxylate.

### 8) Step 8

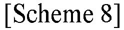

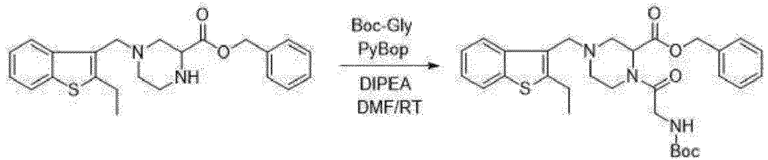

At room temperature, benzyl 4-((2-ethylbenzofuran-3-yl)methyl)piperazine-2-carboxylate (1.0 equivalent), N-(tert-butoxycarbonyl)glycine (1.1 equivalents), and (benzotriazol-1-yloxy)tripyrrolidinophosphonium hexafluorophosphate (1.2 equivalents) were dissolved in dimethylformamide, and N,N-diisopropylethylamine (1.5 equivalents) was added thereto, and stirred overnight at room temperature. The reaction was terminated using an aqueous ammonium chloride solution and ethyl acetate. The resulting organic layer was washed with brine, dried over anhydrous magnesium sulfate, and then concentrated under reduced pressure. The residue was purified by silica chromatography to obtain benzyl 1-((tert-butoxycarbonyl)glycyl)-4-((2-ethylbenzothiophene-3-yl)methyl)piperazine-2-carboxylate.

### 9) Step 9

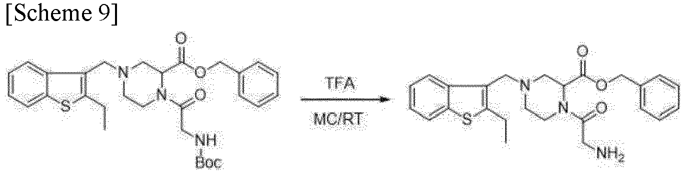

As in Scheme 9, the benzyl 1-((tert-butoxycarbonyl)glycyl)-4-((2-ethylbenzothiophene-3-yl)methyl)piperazine-2-carboxylate obtained in Step 8 was dissolved in methylene chloride (5.0 volumes) at room temperature, and trifluoroacetic acid (2.0 volumes) was then added thereto, and stirred at room temperature for 30 minutes. When the reaction was completed, the reaction solution was neutralized with an aqueous sodium bicarbonate solution, and extracted with methylene chloride. The extract was dried over anhydrous magnesium sulfate, and concentrated under reduced pressure to obtain benzyl 4-((2-ethylbenzothiophene-3-yl)methyl)-1-glycylpiperazine-2-carboxylate.

### 10) Step 10

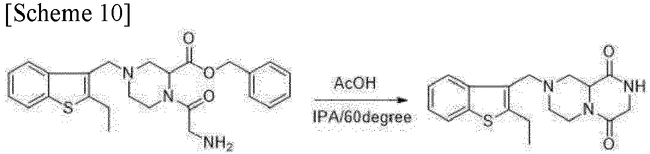

The benzyl 4-((2-ethylbenzothiophene-3-yl)methyl)-1-glycylpiperazine-2-carboxylate obtained in Step 9 was dissolved in isopropanol (5.0 volumes), and acetic acid (1.5 volumes) was then added thereto, warmed, and stirred for an hour. After the reaction was completed, the resulting reaction solution was neutralized with an aqueous sodium bicarbonate solution. The reaction solution was extracted with methylene chloride, and dried over anhydrous magnesium sulfate. After the reaction solution was concentrated under reduced pressure, the residue was purified by silica chromatography to obtain 2-((2-ethylbenzothiophene-3-yl)methyl)hexahydro-2H-pyrazino[1,2-a]pyrazine-6,9-dione (S101).

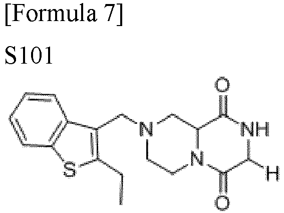

¹H NMR (500 MHz, CDCl₃) δ 7.82 (d, J = 8.0 Hz, 1H), 7.76 (d, J = 8.0 Hz, 1H), 7.34 - 7.31 (m, 1H), 7.29 - 7.25 (m, 1H), 4.50 - 4.46 (m, 1H), 4.07 (d, J = 10.0 Hz, 1H), 4.00 (s, 2H), 3.81 - 3.67 (m, 2H), 3.48 (d, J = 10.5 Hz, 1H), 2.94 (q, J = 7.5 Hz, 2H), 2.86 (d, J = 11.5 Hz, 1H), 2.74 - 2.69 (m, 1H), 2.17 (t, J = 11.0 Hz, 1H), 2.10 - 2.05 (m, 1H), 1.33 (t, J = 7.5 Hz, 3H).

¹³C NMR (126 MHz, CDCl₃) δ 166.10, 161.85, 146.58, 140.52, 138.09, 125.68, 124.02, 123.78, 122.22, 122.19, 56.99, 56.38, 53.28, 51.39, 44.61, 41.59, 22.13, 16.20.

MS (ESI) m/z for C₁₈H₂₁N₃O₂S [M+H]⁺: calcd 344.1427, found 344.1423. 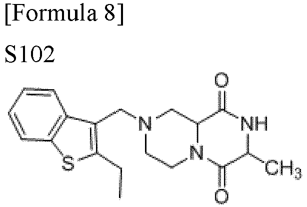 ¹H NMR (500 MHz, CDCl₃) δ 7.83 (d, J = 8.0 Hz, 1H), 7.76 (d, J = 8.0 Hz, 1H), 7.52 - 7.49 (m, 1H), 7.34 - 7.25 (m, 2H), 4.49 - 4.44 (m, 1H), 4.10 - 4.03 (m, 2H), 3.70 (d, J = 13.0 Hz, 1H), 3.68 - 3.65 (m, 1H), 3.50 - 3.46 (m, 1H), 2.94 (q, J = 7.5 Hz, 2H), 2.86 - 2.82 (m, 1H), 2.72 - 2.67 (m, 1H), 2.13 (q, J = 11.0 Hz, 1H), 2.08 - 2.02 (m, 1H), 1.48 (dd, J = 7.0 Hz, 19.0 Hz, 3H), 1.33 (t, J = 7.5 Hz, 3H).

¹³C NMR (126 MHz, CDCl₃) δ 166.06, 165.96, 165.49, 165.41, 146.44, 140.56, 138.09, 125.83, 123.99, 123.75, 122.26, 122.17, 57.45, 57.16, 56.74, 56.53, 53.31, 51.59, 51.46, 50.92, 50.62, 41.75, 22.11, 21.56, 21.48, 16.20.

MS (ESI) m/z for C₁₉H₂₃N₃O₂S [M+H]⁺: calcd 358.1584, found 358.1579.

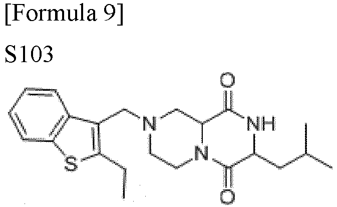

¹H NMR (500 MHz, CDCl₃) δ 7.82 (d, J = 8.0 Hz, 1H), 7.75 (d, J = 8.0 Hz, 1H), 7.33 - 7.30 (m, 1H), 7.28 - 7.25 (m, 1H), 4.78 - 4.44 (m, 1H), 4.05 - 3.98 (m, 2H), 3.80 - 3.66 (m, 2H), 3.50 - 3.48 (m, 1H), 2.94 (q, J = 7.5 Hz, 2H), 2.88 - 2.82 (m, 1H), 2.71 - 2.65 (m, 1H), 2.15 - 2.02 (m, 2H), 1.89 - 1.60 (m, 3H), 1.32 (t, J = 7.5 Hz, 3H), 0.97 - 0.90 (m, 6H).

¹³C NMR (126 MHz, CDCl₃) δ 165.93, 165.71, 165.52, 164.98, 146.45, 140.51, 138.09, 125.81, 124.02, 123.77, 122.21, 122.18, 57.48, 56.65, 56.35, 53.60, 53.23, 51.60, 51.43, 44.21, 44.09, 41.81, 24.13, 23.44, 23.22, 22.14, 21.39, 21.35, 16.18.

MS (ESI) m/z for C₂₂H₂₉N₃O₂S [M+H]⁺: calcd 400.2053, found 400.4056.

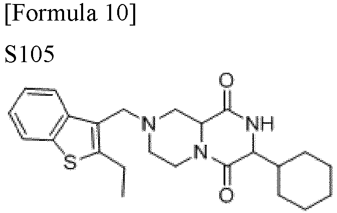

¹H NMR (500 MHz, CDCl₃) δ 7.82 (d, J = 8.0 Hz, 1H), 7.76 (d, J = 8.0 Hz, 1H), 7.35 - 7.25 (m, 2H), 4.52 - 4.48 (m, 1H), 4.07 - 4.01 (m, 1H), 3.89 - 3.75 (m, 2H), 3.69 - 3.62 (m, 1H), 3.55 - 3.49 (m, 1H), 2.94 (q, J = 7.5 Hz, 2H), 2.85 - 2.83 (m, 1H), 2.68 - 2.63 (m, 1H), 2.14 - 2.00 (m, 2H), 1.85 - 1.57 (m, 4H), 1.56 - 1.40 (m, 1H), 1.35 - 1.05 (m, 9H).

¹³C NMR (126 MHz, CDCl₃) δ 166.71, 166.08, 164.96, 163.41, 146.42, 140.54, 138.09, 128.59, 127.62, 127.04, 125.83, 124.00, 123.76, 122.24, 122.17, 60.21, 59.81, 57.40, 57.07, 56.59, 56.38, 53.36, 53.22, 51.80, 51.39, 43.56, 42.00, 41.80, 41.38, 29.36, 29.17, 26.69, 26.46, 26.40, 26.24, 25.96, 25.85, 25.73, 22.12, 16.20.

MS (ESI) m/z for C₂₄H₃₁N₃O₂S [M+H]⁺: calcd 426.2210, found 426.2210.

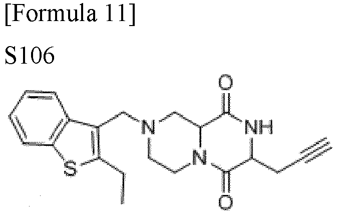

¹H NMR (500 MHz, CDCl₃) δ 7.83 - 7.76 (m, 2H), 7.37 - 7.28 (m, 2H), 6.66 (s, 1H), 4.51 - 4.48 (m, 1H), 4.15 - 4.13 (m, 2H), 3.81 - 3.66 (m, 2H), 3.53 - 3.49 (m, 1H), 2.95 (q, J = 7.5 Hz, 2H), 2.88 - 2.70 (m, 4H), 2.24 - 2.02 (m, 3H), 1.34 (t, J = 7.5 Hz, 3H).

¹³C NMR (126 MHz, CDCl₃) δ 165.73, 165.32, 163.17, 162.57, 146.48, 140.52, 138.09, 128.64, 127.07, 125.76, 124.01, 123.77, 122.19, 78.95, 78.53, 72.91, 57.28, 56.55, 53.75, 53.28, 51.40, 41.74, 31.66, 25.95, 25.45, 22.73, 22.13, 16.18, 14.20.

MS (ESI) m/z for C₂₁H₂₃N₃O₂S [M+H]⁺: calcd 382.1584, found 382.1594.

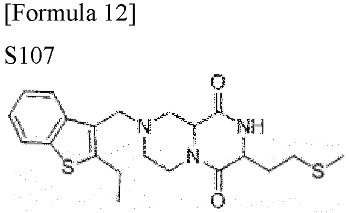

¹H NMR (500 MHz, CDCl₃) δ 7.84 - 7.82 (m, 1H), 7.76 (d, J = 8.0 Hz, 1H), 7.48 - 7.41 (m, 1H), 7.34 - 7.31 (m, 1H), 7.29 - 7.25 (m, 1H), 4.50 - 4.44 (m, 1H), 4.18 - 4.16 (m, 1H), 4.07 - 4.05 (m, 1H), 3.81 - 3.66 (m, 2H), 3.49 (d, J = 10.5 Hz, 1H), 2.95 (q, J = 7.5 Hz, 2H), 2.85 (d, J = 10.5 Hz, 1H), 2.72 - 2.66 (m, 1H), 2.61 (t, J = 7.5 Hz, 1H), 2.60 - 2.55 (m, 1H), 2.25 - 2.11 (m, 3H), 2.09 - 2.02 (m, 4H), 1.33 (t, J = 7.5 Hz, 3H).

¹³C NMR (126 MHz, CDCl₃) δ 166.13, 166.01, 164.70, 164.06, 146.43, 140.53, 138.09, 125.79, 124.01, 123.76, 122.23, 122.17, 57.43, 56.84, 56.71, 56.33, 53.92, 53.82, 53.25, 51.57, 51.35, 41.82, 33.62, 33.31, 29.42, 29.28, 22.13, 16.19, 15.25, 15.09.

MS (ESI) m/z for C₂₁H₂₇N₃O₂S₂ [M+H]⁺: calcd 418.1618, found 418.1618.

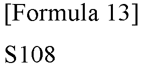

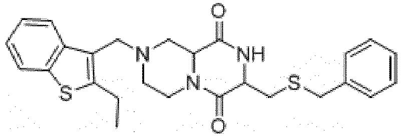

¹H NMR ¹H NMR (500 MHz, CDCl₃) δ 7.82-7.75 (m, 2H), 7.32-7.25 (m, 7H), 6.56-6.52 (m, 1H), 4.46 (t, J = 11.5 Hz, 1H), 4.19-4.02 (m, 2H), 3.80-3.53 (m, 5H), 3.03-2.69 (m, 6H), 2.28-2.06 (m, 2H), 1.34-1.31 (m, 3H).

¹³C NMR (126 MHz, CDCl₃) δ 163.43, 162.88, 140.38, 138.02, 137.42, 128.94, 128.79, 128.77, 128.57, 127.52, 127.46, 127.00, 124.01, 123.77, 122.15, 57.26, 56.59, 54.52, 53.89, 53.18, 51.22, 46.19, 41.58, 37.41, 36.84, 36.78, 22.10, 16.09.

MS (ESI) m/z for C₂₆H₂₉N₃O₂S₂ [M+H]⁺: calcd 480.1774, found 480.1782.

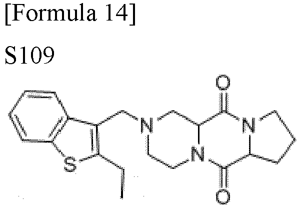

¹H NMR (500 MHz, CDCl₃) δ7.85 - 7.75 (m, 2H), 7.34 - 7.25 (m, 2H), 4.51 - 4.36 (m, 1H), 4.12 - 4.00 (m, 2H), 3.81 - 3.65 (m, 3H), 3.60 - 3.38 (m, 2H), 2.98 - 2.91 (m, 2H), 2.88 - 2.79 (m, 1H), 2.77 - 2.68 (m, 1H), 2.49 - 2.43 (m, 1H), 2.19 - 2.10 (m, 1H), 2.09 - 1.99 (m, 2H), 1.97 - 1.83 (m, 2H), 1.36 - 1.31 (m, 3H)

¹³C NMR (126 MHz, CDCl₃) δ 167.03, 164.35, 163.16, 162.69, 146.41, 146.37, 140.61, 140.54, 138.09, 138.06, 126.04, 125.78, 124.00, 123.93, 123.73, 123.68, 122.33, 122.22, 122.14, 122.11, 61.02, 58.96, 58.59, 56.99, 56.22, 55.84, 53.47, 53.18, 51.81, 51.26, 45.35, 45.28, 41.93, 41.60, 29.99, 29.80, 22.11, 21.90, 21.76, 16.19, 16.16.

MS (ESI) m/z for C₂₁H₂₅N₃O₂S [M+H]⁺: calcd 384.1740, found 384.1746.

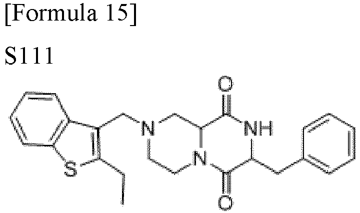

¹H NMR (500 MHz, CDCl₃) δ 7.76 - 7.71 (m, 2H), 7.41 - 7.03 (m, 8H), 4.48 - 4.25 (m, 2H), 3.84 - 3.78 (m, 0.5H), 3.70 (dd, J = 13.0 Hz, 65.5 Hz, 1H),3.44 (dd, J = 13.0 Hz, 27.5 Hz, 1H), 3.35 - 3.27 (m, 1H), 3.21 - 3.16 (m, 0.5H), 3.08 - 2.83 (m, 4H), 2.77 - 2.62 (m, 1H), 2.58 - 2.37 (m, 1H), 2.01 - 1.92 (m, 1H), 1.87 - 1.65 (m, 1H), 1.34 - 1.27 (m, 3H).

¹³C NMR (126 MHz, CDCl₃) δ 166.56, 165.99, 164.52, 162.74, 146.41, 146.22, 140.55, 140.52, 138.07, 135.17, 134.88, 131.05, 130.09, 128.85, 128.82, 127.73, 127.49, 125.84, 123.92, 123.71, 122.23, 122.19, 122.13, 57.51, 56.27, 56.23, 55.98, 55.83, 55.59, 53.25, 52.91, 51.19, 51.14, 41.71, 41.36, 40.76, 22.09, 22.03, 16.18.

MS (ESI) m/z for C₂₅H₂₇N₃O₂S [M+H]⁺: calcd 434.1897, found 434.1895.

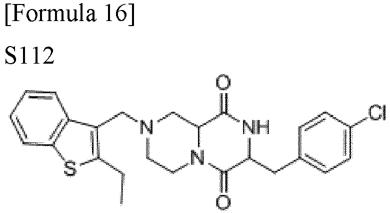

¹H-NMR (500 MHz, CDCl₃) δ 7.78-7.72 (m, 2H), 7.39 (d, J = 8.0 Hz, 1H), 7.34-7.25 (m, 3H), 7.18 (d, J = 8.5 Hz, 1H), 7.09 (d, J = 8.5 Hz, 1H), 4.45-4.28 (m, 2H), 3.83-2.83 (m, 8H), 2.83-2.37 (m, 2H), 2.05-1.68(m, 2H), 1.35-1.27 (m, 3H).

¹³C NMR (126 MHz, CDCl₃) δ 164.18, 162.30, 146.47, 140.48,138.05, 133.81, 133.60, 133.43, 132.54, 131.40, 128.94, 125.75, 124.05, 123.96, 123.76, 122.16, 56.21, 56.07, 55.84, 55.65, 53.17, 53.08, 51.21, 51.05, 41.65, 41.39, 40.57, 39.97, 22.13, 22.06, 16.26, 16.16.

MS (ESI) m/z for C₂₅H₂₆ClN₃O₂S [M+H]⁺: calcd 468.1507, found 468.1507.

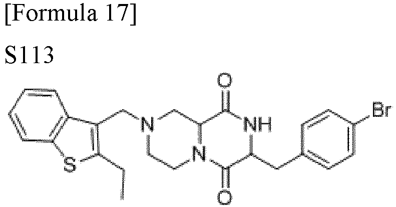

¹H NMR (500 MHz, CDCl₃) δ 7.79-7.72 (m, 2H), 7.54 (d, J = 8.0 Hz, 1H), 7.44 (d, J = 8.5 Hz, 1H), 7.36-7.23 (m, 2H), 7.13 (d, J = 8.0 Hz, 1H), 7.03 (d, J = 8.0 Hz, 1H), 4.45-4.28 (m, 2H), 3.90-2.85 (m, 8H), 2.78-2.41 (m, 2H), 2.05-1.65 (m, 2H), 1.35-1.28 (m, 3H).

¹³C NMR (126 MHz, CDCl₃) δ 164.16, 162.24, 146.43, 140.48, 138.06, 134.87, 134.05, 133.95, 132.93, 131.89, 131.75, 125.75, 124.06, 123.96, 123.76, 122.15, 121.87, 121.70, 57.68, 56.20, 56.15, 56.01, 55.90, 55.58, 53.21, 53.13, 52.47, 51.13, 51.04, 41.68, 41.42, 40.69, 40.11, 22.13, 16.31, 16.18.

MS (ESI) m/z for C₂₅H₂₆BrN₃O₂S [M+H]⁺: calcd 512.1002, found 512.1002.

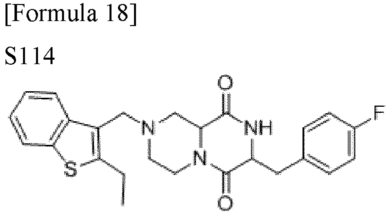

¹H NMR (500 MHz, CDCl₃) δ 7.77-7.72 (m, 2H), 7.37-7.24 (m, 2H), 7.22-7.19 (m, 1H), 7.13-7.08 (m, 2H), 7.00 (t, J = 8.5 Hz, 1H), 4.48-4.27 (m, 2H), 3.84-3.47 (m, 2H), 3.36-3.18 (m, 2H), 3.03-3.86 (m, 4H), 2.77-2.43 (m, 2H), 2.04-1.68 (m, 2H), 1.34-1.28 (m, 3H) .

¹³C NMR (126 MHz, CDCl₃) δ 164.19, 163.33, 162.45, 161.39, 146.43,140.41, 140.36, 137.98, 132.55, 132.49, 131.57, 131.52, 130.81, 130.52, 123.93, 123.88, 123.67, 122.07, 115.72, 115.68, 115.55, 115.52, 56.12, 56.00, 55.69, 55.62, 53.12, 52.97, 51.01, 41.55, 41.28, 40.34, 39.69, 22.04, 21.93, 16.06.

MS (ESI) m/z for C₂₅H₂₆FN₃O₂S [M+H]⁺: calcd 452.1803, found 452.1798.

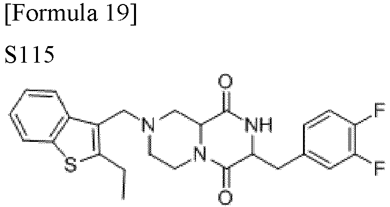

¹H NMR (500 MHz, CDCl₃) δ 7.78-7.72 (m, 2H), 7.37-7.24 (m, 3H), 7.21-7.06 (m, 1H), 6.95-6.86 (m, 1H), 4.49 - 4.27 (m, 2H), 3.86-3.49 (m, 2H), 3.38-3.30 (m, 1H), 3.23-2.98 (m, 2H), 2.94-2.83 (m, 3H), 2.78-2.48 (m, 2H), 2.04-1.68 (m, 2H), 1.33-1.30 (m, 3H).

¹³C NMR (126 MHz, CDCl₃) δ 163.79, 162.28, 146.43, 140.39, 140.35, 137.98, 137.95, 132.03, 131.96, 128.56, 127.64, 127.01, 126.01, 123.96, 123.92, 123.69, 122.07, 119.82, 119.69, 118.95, 118.81, 117.60, 117.44, 117.31, 56.16, 55.88, 55.44, 53.09, 53.02, 51.40, 51.04, 41.55, 41.36, 40.11, 39.54, 22.05, 21.90, 16.05, 16.03.

MS (ESI) m/z for C₂₅H₂₅F₂N₃O₂S [M+H]⁺: calcd 470.1708, found 470.1702.

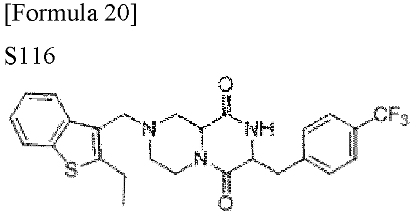

¹H NMR (500 MHz, CDCl₃) δ 7.77 - 7.71 (m, 2H), 7.68 - 7.65 (m, 1H), 7.58 - 7.55 (m, 1H), 7.38 - 7.35 (m, 1H), 7.31 - 7.21 (m, 3H), 4.43 - 4.31 (m, 2H), 3.85 - 3.82 (m, 0.5H), 3.65 (dd, J = 13.0 Hz, 65.0 Hz, 1H), 3.50 - 3.41 (m, 1H), 3.35 - 3.29 (m, 1H), 3.28 - 3.23 (m, 0.5H), 3.14 - 3.09 (m, 0.5H), 3.08 - 3.03 (m, 1H), 3.02 - 2.96 (m, 0.5H), 2.92 - 2.80 (m, 2H), 2.78 - 2.60 (m, 1H), 2.56 - 2.37 (m, 1H), 2.04 - 1.80 (m, 1.5H), 1.53-1.44 (m, 0.5H), 1.31 - 1.26 (m, 3H).

¹³C NMR (126 MHz, CDCl₃) δ 166.34, 165.91, 163.97, 162.11, 146.43, 146.31, 140.48, 140.46, 139.31, 139.26, 138.03, 131.44, 130.46, 128.63, 127.09, 125.70, 125.66, 125.61, 125.58, 123.99, 123.91, 123.75, 123.69, 122.18, 122.12, 57.37, 56.22, 56.18, 56.10, 55.96, 55.53, 53.21, 52.83, 51.10, 51.01, 41.68, 41.51, 40.97, 40.35, 22.07, 21.87, 16.09.

MS (ESI) m/z for C₂₆H₂₆F₃N₃O₂S [M+H]⁺: calcd 502.1771, found 502.1758.

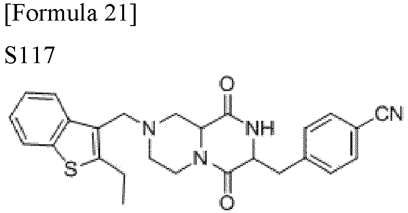

¹H NMR (500 MHz, CDCl₃) δ 7.77 - 7.70 (m, 2H), 7.68 - 7.64 (m, 1H), 7.59 - 7.56 (m, 1H), 7.38 - 7.32 (m, 2H), 7.29 - 7.23 (m, 2H), 4.46 - 4.29 (m, 2H), 3.85 - 3.78 (m, 0.5H), 3.66 (dd, J = 13.0 Hz, 54.0 Hz, 1H), 3.53 - 3.43 (m, 1H), 3.42 - 3.37 (m, 0.5H), 3.32 - 3.24 (m, 1H), 3.13 - 2.96 (m, 2H), 2.93 - 2.86 (m, 2H), 2.81 - 2.68 (m, 1H), 2.61 - 2.36 (m, 1H), 2.02 - 1.92 (m, 1.5H), 1.59-1.51 (m, 0.5H), 1.35 - 1.28 (m, 3H).

¹³C NMR (126 MHz, CDCl₃) δ 166.19, 165.77, 163.62, 162.08, 146.50, 146.44, 140.86, 140.74, 140.46, 140.39, 138.04, 132.39, 132.33, 131.85, 130.89, 128.63, 127.70, 127.09, 125.63, 125.44, 124.07, 123.98, 123.81, 123.75, 122.15, 118.49, 118.45, 111.73, 111.55, 65.30, 57.31, 56.29, 56.15, 56.04, 55.77, 55.37, 53.20, 51.52, 51.15, 41.70, 41.51, 41.09, 40.47, 22.09, 16.23, 16.17.

MS (ESI) m/z for C₂₆H₂₆N₄O₂S [M+H]⁺: calcd 459.1849, found 459.1833.

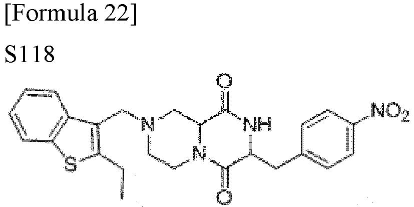

¹H NMR (500 MHz, CDCl₃) δ 8.26 - 8.23 (m, 1H), 8.16 - 8.13 (m, 1H), 7.75 - 7.67 (m, 2H), 7.42 - 7.39 (m, 1H), 7.36 - 7.21 (m, 3H), 4.48 - 4.33 (m, 2H), 3.85 - 3.80 (m, 0.5H), 3.65 (dd, J = 13.0 Hz, 52.5 Hz, 1H), 3.50 - 3.45 (m, 0.5H), 3.44 - 3.26 (m, 2H), 3.22 - 3.10 (m, 1H), 3.08 - 3.01 (m, 1H), 2.89 (q, J = 7.5 Hz, 1H), 2.84 - 2.76 (m, 1.5H), 2.72 - 2.67 (m, 0.5H), 2.60 - 2.42 (m, 1H), 2.03 - 1.89 (m, 1.5H), 1.61-1.51 (m, 0.5H), 1.31 - 1.27 (m, 3H).

¹³C NMR (126 MHz, CDCl₃) δ 166.24, 165.90, 163.51, 162.06, 147.58, 147.54, 146.51, 146.47, 142.89, 140.45, 140.37, 138.04, 138.01, 131.93, 131.00, 128.64, 127.10, 125.61, 125.36, 124.02, 123.97, 123.79, 123.72, 122.13, 122.09, 57.28, 56.36, 56.17, 55.75, 55.35, 53.20, 53.11, 51.45, 51.18, 41.74, 41.54, 40.74, 40.16, 22.09, 21.99, 16.13.

MS (ESI) m/z for C₂₅H₂₆N₄O₄S [M+H]⁺: calcd 479.1748, found 479.1732.

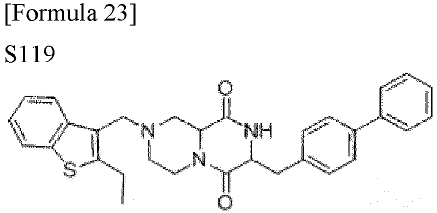

¹H NMR (500 MHz, CDCl₃) δ 7.74 - 7.18 (m, 13H), 4.47 - 4.30 (m, 2H), 3.86 - 3.80 (m, 0.5H), 3.63 (dd, J = 13.0 Hz, 71.0 Hz, 1H), 3.45 - 3.39 (m, 0.5H), 3.36 - 3.15 (m, 2H), 3.13 - 3.07 (m, 0.5H), 3.02 - 2.96 (m, 1.5H), 2.89 - 2.83 (m, 1H), 2.75 - 2.70 (m, 0.5H), 2.57 - 2.47 (m, 2H), 2.46 - 2.38 (m, 0.5H), 2.03 - 1.86 (m, 1.5H), 1.61-1.53 (m, 0.5H), 1.26 (t, J = 7.5 Hz, 1.5H), 1.07 (t, J = 7.5 Hz, 1.5H).

¹³C NMR (126 MHz, CDCl₃) δ 166.68, 166.12, 164.63, 162.50, 146.36, 146.12, 140.75, 140.53, 140.48, 140.41, 138.03, 137.96, 134.07, 133.94, 131.68, 130.57, 129.15, 128.95, 127.79, 127.60, 127.42, 127.32, 127.13, 126.93, 125.83, 125.69, 123.95, 123.84, 123.67, 123.62, 122.22, 122.10, 122.03, 57.47, 56.29, 56.26, 56.09, 55.88, 53.26, 53.04, 51.03, 50.88, 41.68, 41.45, 41.06, 40.45, 22.08, 21.75, 16.14, 16.04.

MS (ESI) m/z for C₃₁H₃₁N₃O₂S [M+H]⁺: calcd 510.2210, found 510.2193.

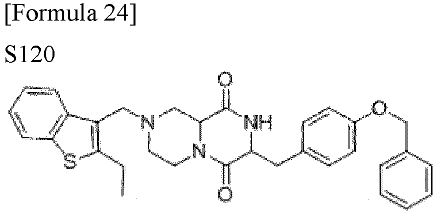

¹H NMR (500 MHz, CDCl₃) δ 7.77 - 7.71 (m, 2H), 7.44 - 7.20 (m, 7H), 7.18 - 7.14 (m, 1H), 7.06 - 7.00 (m, 2H), 6.92 - 6.88 (m, 1H), 5.09 (s, 1H), 5.04 - 4.99 (m, 1H), 4.70 - 4.34 (m, 1H), 4.31 - 4.22 (m, 1H), 3.86 - 3.80 (m, 0.5H), 3.65 (dd, J = 13.0 Hz, 68.5 Hz, 1H), 3.45 (dd, J = 13.0 Hz, 45.5 Hz, 1H), 3.34 - 3.30 (m, 0.5H), 3.29 - 3.24 (m, 0.5H), 3.17 - 3.12 (m, 0.5H), 3.05 - 2.81 (m, 4H), 2.77 - 2.60 (m, 1H), 2.57 - 2.35 (m, 1H), 2.03 - 1.82 (m, 1.5H), 1.73-1.66 (m, 0.5H),1.29 (t, J = 7.5 Hz, 1.5H), 1.25 (t, J = 7.5 Hz, 1.5H).

¹³C NMR (126 MHz, CDCl₃) δ 166.61, 166.00, 164.58, 162.84, 158.44, 146.40, 146.29, 140.57, 140.52, 138.08, 138.05, 136.84, 136.77, 132.07, 131.17, 128.73, 128.68, 128.19, 128.08, 127.51, 127.49, 127.37, 126.97, 125.87, 125.83, 123.97, 123.94, 123.71, 122.22, 122.13, 115.24, 115.20, 70.28, 70.12, 57.49, 56.37, 56.05, 55.98, 55.82, 53.30, 53.08, 51.20, 51.11, 41.68, 41.34, 40.52, 39.97, 22.10, 21.99, 16.17.

MS (ESI) m/z for C₃₂H₃₃N₃O₃S [M+H]⁺: calcd 540.2315, found 540.2292.

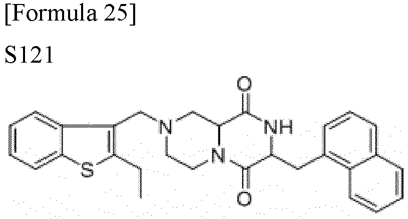

¹H-NMR (500 MHz, CDCl₃) δ 8.18 (m, 1H), 7.93-7.85 (m, 1H), 7.81-7.69 (m, 2H), 7.59-7.35 (m, 4H), 7.33-7.24 (m, 3H), 4.44-4.27 (m, 2H), 3.91-3.77 (m, 1H), 3.73-3.57 (m, 2H), 3.43-3.23 (m, 3H), 2.97-2.74 (m, 3H), 2.50-2.31 (m, 2H), 2.05-1.96 (m, 1H), 1.35-1.27 (m, 3H).

¹³C-NMR (126 MHz, CDCl₃) δ165.62, 165.56, 164.56, 163.23, 146.28, 146.09, 140.45, 140.42,

137.99, 137.94, 134.14, 134.06, 132.44, 131.67, 131.42, 131.30, 129.58, 128.90, 128.75, 128.68,

128.48, 128.36, 126.63, 126.62, 126.13, 126.09, 125.74, 125.73, 125.40, 125.34, 124.32, 123.84,

123.79, 123.64, 123.61, 123.59, 122.14, 122.04, 122.01, 57.45, 56.39, 56.23, 55.56, 55.42, 55.31,53.14, 52.75, 51.08, 50.64, 41.66, 41.55, 38.48, 37.71, 21.99, 21.93, 16.10, 16.08.

MS (ESI) m/z for C₂₉H₂₉N₃O₂S [M+H]⁺: calcd 484.2053, found 484.2047

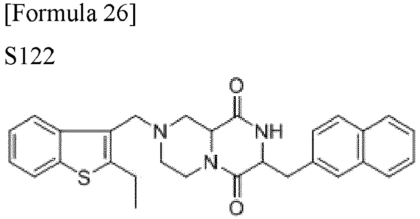

¹H-NMR (500 MHz, CDCl₃) δ 7.94-7.89 (m, 1H), 7.82-7.73 (m, 2H), 7.70-7.65 (m, 1H), 7.60-7.46 (m, 3H), 7.42-7.29 (m, 1H), 7.32-7.12 (m, 3H), 4.45-4.37 (m, 2H), 3.76-3.52 (m, 2H), 3.34-3.12 (m, 2H), 2.98-2.83 (m, 2H), 2.75-2.57 (m, 3H), 2.48-2.38 (m, 2H), 2.00-1.92 (m, 1H), 1.27-1.22 (m, 3H);

¹³C-NMR (126 MHz, CDCl₃) δ 166.20, 165.90, 164.42, 162.50, 146.27, 145.95, 140.42, 140.33, 137.93, 137.85, 133.70, 133.42, 132.68, 132.57, 132.51, 132.42, 129.74, 129.18, 128.90, 128.42, 128.13, 127.93, 127.73, 127.61, 127.60, 126.50, 126.47, 126.11, 125.70, 125.66, 123.83, 123.67, 123.57, 123.50, 122.12, 122.10, 122.00, 121.93, 57.32, 56.27, 56.15, 56.02, 56.01, 55.43, 53.07, 52.40,50.94, 50.64, 41.55, 41.48, 41.36, 40.85, 21.97, 21.70, 16.13, 16.02.

MS (ESI) m/z for C₂₉H₂₉N₃O₂S [M+H]⁺: calcd 484.2053, found 484.2044

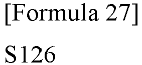

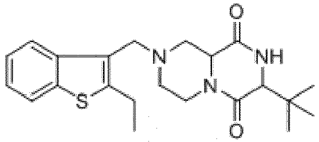

¹H-NMR (500 MHz, CDCl₃) δ 7.83 (d, J = 8.0 Hz, 1H), 7.77 (d, J = 8.0 Hz, 1H), 7.33 (t, J = 7.5 Hz, 1H), 7.27 (t, J = 7.5 Hz, 1H), 7.62 (s, 1H), 4.48 (d, J = 13.0 Hz, 1H), 4.07 (d, J = 11.0 Hz, 1H), 3.80 (d, J = 13.0 Hz, 1H), 3.70-3.3.63 (m, 2H), 3.51 (d, J = 11.0 Hz, 1H), 2.94 (q, J = 7.5 Hz, 2H), 2.85 (d, J = 11.5 Hz, 1H), 2.69-2.62 (m, 1H), 2.18 (t, J = 11.0 Hz, 1H), 2.01-1.95 (m, 1H), 1.33 (t, J = 7.5 Hz, 3H), 1.09 (s, 9H) ;

¹³C-NMR (126 MHz, CDCl₃) δ165.63, 162.11, 146.27, 140.48, 138.01, 125.81, 123.90, 123.65, 122.16, 122.07, 63.52, 57.83, 56.32, 53.18, 51.81, 41.76, 36.62, 29.69, 26.71, 22.02, 16.10.

MS (ESI) m/z for C₂₂H₂₉N₃O₂S [M+H]⁺: calcd 400.2053, found 400.2053.

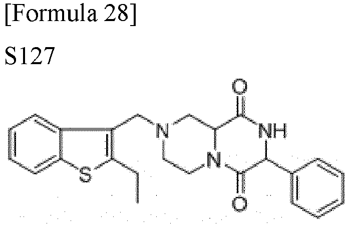

¹H-NMR (500 MHz, CDCl₃) δ 7.81 (d, J = 8.0 Hz, 1H), 7.75 (d, J = 8.0 Hz, 1H), 7.40-7.31 (m, 6H), 7.28-7.24 (m, 1H), 5.00-5.04 (m, 1H), 4.42-4.32 (m, 1H), 4.15-4.09 (m, 1H), 3.78 (d, J = 13.0 Hz, 1H), 3.67-3.59 (m, 1H), 3.56-3.49 (m, 1H), 2.97-2.89 (m, 2H), 2.83-2.75 (m, 1H), 2.69-2.61 (m, 1H), 2.21-2.12 (m, 1H), 2.07-1.90 (m, 1H), 1.34-1.30 (m, 3H) ;

¹³C-NMR (126 MHz, CDCl₃) δ 166.10, 165.36, 163.45, 162.75, 146.35, 140.46, 140.44, 138.01, 137.98, 137.75, 129.00, 128.95, 128.79, 128.75, 128.51, 127.56, 127.08, 126.96, 126.65, 125.72, 125.70, 123.91, 123.89, 123.67, 123.65, 122.17, 122.16, 122.08, 65.21, 59.20, 59.17, 57.68, 57.07, 56.50, 56.48, 53.27, 53.14, 51.37, 51.32, 41.96, 41.80, 22.01, 16.11, 16.10.

MS (ESI) m/z for C₂₄H₂₅N₃O₂S [M+H]⁺: calcd 420.1740, found 420.1740.

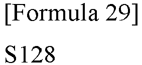

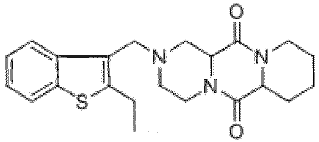

¹H-NMR (500 MHz, CDCl₃) δ 7.82 (d, J = 8.0 Hz, 1H), 7.74 (d, J = 8.0 Hz, 1H), 7.32-7.23 (m, 2H), 4.66 (d, J = 13.0 Hz, 1H), 4.48-4.43 (m, 1H), 4.03 (d, J = 10.5 Hz, 1H), 3.83-3.76 (m, 2H), 3.62 (d, J = 13.0 Hz, 1H), 3.54 (d, J = 11.0 Hz, 1H), 2.95-2.89 (m, 2H), 2.79 (d, J = 11.5 Hz, 1H), 2.68-2.61 (m, 1H), 2.49-2.41 (m, 1H), 2.33 (d, J = 13.0 Hz, 1H), 2.10-1.97 (m, 3H), 1.70 (t, J = 13.0 Hz, 1H), 1.61-1.52 (m, 1H), 1.43-1.37 (m, 2H), 1.32-1.29 (m, 3H) ;

¹³C-NMR (126 MHz, CDCl₃) δ 164.20, 163.98, 162.15, 162.09, 146.28, 146.27, 140.52, 140.49, 138.00, 125.84, 125.79, 123.90, 123.87, 123.63, 123.61, 122.22, 122.20, 122.04, 59.03, 58.78, 57.59, 57.57, 57.19, 57.03, 53.22, 53.20, 51.42, 51.19, 42.60, 42.43, 41.38, 41.34, 32.28, 31.67, 24.69, 24.57, 24.48, 24.44, 22.02, 16.10, 16.09.

MS (ESI) m/z for C₂₂H₂₇N₃O₂S [M+H]⁺: calcd 398.1897, found 398.1891.

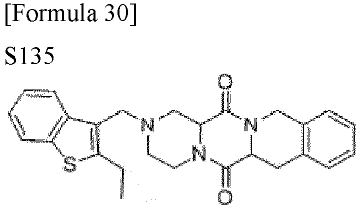

¹H NMR (500 MHz, CDCl₃) δ 7.84 (d, J = 7.5 Hz, 1H), 7.76 (d, J = 8.0 Hz, 1H), 7.34-7.26 (m, 2H), 7.24-7.13 (m, 4H), 5.46 (d, J = 17.0 Hz, 1H), 4.53-4.53 (m, 1H), 4.24-4.19 (m, 2H), 4.16-4.11 (m, 1H), 3.84 (d, J = 13.0 Hz, 1H), 3.67 (d, J = 13.0 Hz, 1H), 3.63-3.59 (m, 1H), 3.45-3.40 (m, 1H), 2.99-2.93 (m, 3H), 2.87-2.84 (m, 1H), 2.77-2.71 (m, 1H), 2.18 (t, J = 11.0 Hz, 1H), 2.10-2.04 (m, 1H), 1.34 (t, J = 7.5 Hz, 3H).

¹³C NMR (126 MHz, CDCl₃) δ 163.56, 162.52, 146.36, 140.49, 138.01, 132.13, 131.00, 128.92, 127.07, 126.95, 126.29, 125.76, 123.90, 123.65, 122.20, 122.07, 57.10, 57.05, 55.51, 53.23, 51.18, 44.07, 41.47, 34.42, 22.04, 16.13.

MS (ESI) m/z for C₂₆H₂₇N₃O₂S [M+H]⁺: calcd 446.1896, found 446.1889.

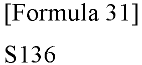

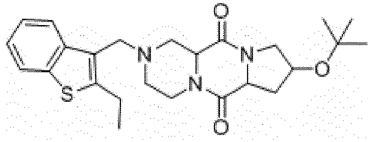

¹H-NMR (500 MHz, CDCl₃) δ 7.83-7.81 (m, 1H), 7.75-7.73 (m, 1H), 7.32-7.29 (m, 1H), 7.26-7.23 (m, 1H), 4.37-4.32 (m, 2H), 4.24-4.20 (m, 1H), 4.04-4.01 (m, 1H), 3.91-3.86 (m, 1H), 3.78 (d, J = 13.0 Hz, 1H), 3.64 (d, J = 13.0 Hz, 1H), 3.57-3.54 (m, 1H), 3.34 (d, J = 13.0 Hz, 1H), 2.96-2.91 (m, 2H), 2.86-2.82 (m, 1H), 2.78-2.72 (m, 1H), 2.36-2.31 (m, 1H), 2.09 (t, J = 11.5 Hz, 1H), 2.04-1.96 (m, 2H), 1.32 (t, J = 7.5 Hz, 3H), 1.17 (s, 9H).

¹³C NMR (126 MHz, CDCl₃) δ 167.28, 162.95, 146.28, 140.52, 138.00, 125.93, 123.87, 123.61, 122.24, 122.03, 74.48, 67.09, 56.97, 56.80, 55.78, 54.49, 53.41, 51.14, 41.52, 38.85, 33.98, 28.23, 25.64, 24.95, 22.03, 16.11.

MS (ESI) m/z for C₂₅H₃₃N₃O₃S [M+H]⁺: calcd 456.2315, found 465.2308.

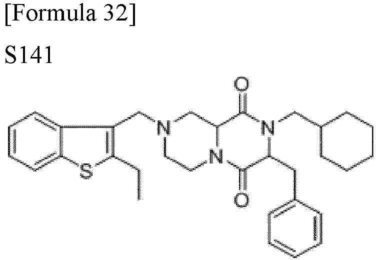

¹H NMR (700 MHz, CDCl₃) δ 7.75-7.68 (m, 2H), 7.30-7.26 (m, 4H), 7.26-7.25 (m, 1H), 7.10-6.99 (m, 2H), 4.43-4.20 (m, 2H), 4.16-4.07 (m, 1H), 3.70-3.49 (m, 1H), 3.38-3.28 (m, 2H), 3.23-3.13 (m, 1H), 3.10-2.95 (m, 1H), 2.89-2.83 (m, 2H), 2.81-2.50 (m, 3H), 2.27-2.17 (m, 1H), 1.89-1.83 (m, 1H), 1.77-1.60 (m, 8H), 1.33-1.26 (m, 3H), 1.24-1.15 (m, 2H), 1.04-0.91 (m, 2H).

¹³C NMR (176 MHz, CDCl₃) δ165.42, 165.21, 164.20, 162.52, 146.16, 145.92, 140.49, 140.45, 137.98, 137.94, 134.83, 134.41, 131.10, 130.02, 128.92, 128.62, 127.83, 127.45, 125.92, 125.91, 123.74, 123.55, 123.52, 122.22, 122.12, 121.98, 60.97, 60.35, 57.62, 56.92, 55.16, 54.78, 53.09, 52.57, 50.71, 50.42, 49.82, 49.62, 41.37, 40.88, 37.39, 36.55, 35.71, 35.26, 31.12, 31.06, 30.53, 30.51, 26.33, 26.28, 25.83, 25.74, 25.65, 25.60, 21.99, 21.93, 16.07, 16.07.

MS (ESI) m/z for C₃₂H₃₉N₃O₂S [M+H]⁺: calcd 530.2836, found 530.2824.

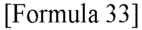

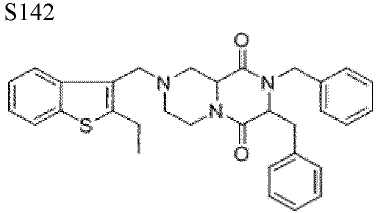

¹H NMR (500 MHz, CDCl₃) δ 7.75-7.68 (m, 2H), 7.40-7.22 (m, 10H), 7.13 (d, J = 8.0 Hz, 1H), 7.02 (d, J = 7.5 Hz, 1H), 5.69 (d, J = 14.5 Hz, 0.5H), 5.53 (d, J = 14.5 Hz, 0.5H), 4.41-4.36 (m, 0.5H), 4.26-4.21 (m, 0.5H), 4.20-4.13 (m, 1H), 4.01-3.93 (m, 1H), 3.87-3.83 (m, 0.5H), 3.60 (dd, J = 13.0 Hz, 81.0 Hz, 1H), 3.39-3.28 (m, 2H), 3.24-3.18 (m, 1H), 3.14-3.09 (m, 0.5H), 2.95-2.82 (m, 3H), 2.66(d, J = 11.5, 0.5H), 2.58-2.47 (m, 1H), 2.31-2.23 (m, 1H), 1.94-1.84 (m, 1H), 1.65-1.58 (m, 0.5H), 1.33-1.26 (m, 3H).

¹³C NMR (176 MHz, CDCl₃) δ165.40, 165.13, 164.12, 162.37, 146.20, 145.97, 140.49, 140.44, 137.99, 137.95, 135.41, 135.05, 134.89, 134.50, 131.01, 129.92, 129.01, 128.97, 128.69, 128.59,128.32, 128.26, 128.14, 127.88, 127.50, 125.89, 125.84, 123.77, 123.75, 123.58, 123.54, 122.19,122.11, 122.00, 59.34, 58.54, 57.85, 56.48, 55.06, 55.03, 53.12, 52.60, 50.75, 50.49, 46.75, 46.39,41.41, 40.97, 37.01, 35.98, 22.01, 21.95, 16.09, 16.07.

MS (ESI) m/z for C₃₂H₃₃N₃O₂S [M+H]⁺: calcd 524.2366, found 524.2364.

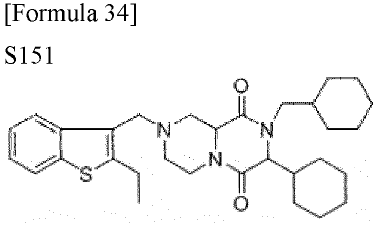

¹H NMR (700 MHz, CDCl₃) δ 7.85 (d, J = 5.5 Hz, 1H), 7.76 (d, J = 6.0 Hz, 1H), 7.36 - 7.31 (m, 1H), 7.28 - 7.25 (m, 1H), 4.44-4.41 (m, 1H), 4.01-3.98 (m, 2H), 3.81 (d, J = 9.5 Hz, 1H), 3.71 (d, J = 3.0 Hz, 1H), 3.64-3.61 (m, 2H), 2.98-2.93 (m, 2H), 2.83-2.80 (m, 1H), 2.70-2.66 (m, 1H), 2.49-2.46 (m, 1H), 2.04-1.99 (m, 2H), 1.82-1.72 (m, 5H), 1.70-1.60 (m, 7H), 1.43-1.38 (m, 1H), 1.33 (t, J = 5.5 Hz, 3H), 1.22 - 1.10 (m, 7H), 0.98 - 0.87 (m, 2H).

¹³C NMR (176 MHz, CDCl₃) δ 165.49, 165.37, 146.23, 140.54, 138.01, 128.51, 127.54, 126.94, 125.95, 123.86, 123.60, 122.27, 122.03, 65.63, 65.25, 57.52, 56.33, 53.37, 51.58, 51.00, 43.22,40.86, 35.78, 30.99, 30.53, 30.05, 28.24, 26.42, 26.35, 26.23, 25.82, 25.77, 25.66, 22.02, 16.10.

MS (ESI) m/z for C₃₁H₄₃N₃O₂S [M+H]⁺: calcd 522.3154, found 522.3143.

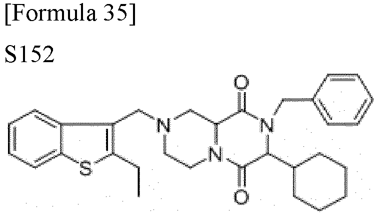

¹H NMR (700 MHz, CDCl₃) δ 7.86-7.83 (m, 1H), 7.77-7.74 (m, 1H), 7.35-7.25 (m, 5H), 7.23- 7.19 (m, 2H), 5.39-5.34 (m, 1H), 4.45-4.40 (m, 1H), 4.16-4.08 (m, 1H), 3.99-3.93 (m, 1H), 3.84-3.81 (m, 1H), 3.75-3.70 (m, 1H), 3.67-3.57 (m, 2H), 2.98-2.93 (m, 2H), 2.83-2.81 (m, 1H), 2.71-2.61 (m, 1H), 2.25-2.06 (m, 1H), 2.05-1.97 (m, 1H), 1.87-1.55 (m, 7H), 1.35-1.32 (m, 3H), 1.21-1.06 (m, 3H), 1.00-0.87 (m, 1H).

¹³C NMR (176 MHz, CDCl₃) δ165.34, 165.00, 164.24, 162.08, 146.27, 146.21, 140.53, 140.48, 138.02, 137.99, 135.77, 135.53, 128.92, 128.87, 128.50, 128.34, 128.01, 127.95, 127.93, 127.54, 126.93, 125.93, 125.88, 123.89, 123.87, 123.63, 123.61, 122.25, 122.21, 122.05, 65.26, 63.77, 63.29, 58.26, 57.16, 56.77, 56.20, 53.39, 53.14, 51.68, 51.03, 47.98, 47.61, 42.84, 41.42, 40.97, 40.86, 30.44, 30.02, 28.30, 28.04, 26.87, 26.50, 26.44, 26.25, 25.92, 25.75, 22.04, 22.02, 16.12, 16.10.

MS (ESI) m/z for C₃₁H₃₇N₃O₂S [M+H]⁺: calcd 516.2679, found 516.2677.

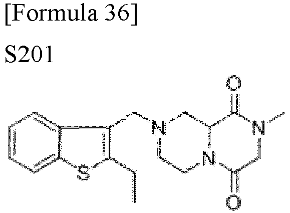

¹H NMR (500 MHz, CDCl₃) δ 7.83 (d, J = 7.5 Hz, 1H), 7.76 (d, J = 8.0 Hz, 1H), 7.32 (t, J = 7.5 Hz, 1H), 7.26 (t, J = 7.5 Hz, 1H), 4.47 (d, J = 13.0 Hz, 1H), 4.05 (d, J = 11.0 Hz, 1H), 3.97 (s, 2H), 3.79 (d, J = 13.0 Hz, 1H), 3.65 (d, J = 13.0 Hz, 1H), 3.54 (d, J = 11.5 Hz, 1H), 2.97-2.91 (m, 5H), 2.82 (d, J = 11.5 Hz, 1H), 2.69 (dt, J = 3.5, 12.5 Hz, 1H), 2.11 (d, J = 11.3 Hz, 1H), 2.03 (dt, J = 3.5, 12.0 Hz, 1H), 1.33 (t, J = 7.5 Hz, 3H).

¹³C NMR (126 MHz, CDCl₃) δ 163.75, 161.59, 146.55, 140.70, 138.21, 125.99, 124.12, 123.86, 122.41, 122.28, 57.51, 57.08, 53.43, 51.46, 51.42, 41.60, 33.62, 22.26, 16.35.

MS (ESI) m/z for C₁₉H₂₃N₃O₂S [M+H]⁺: calcd 358.1584, found 358.1576.

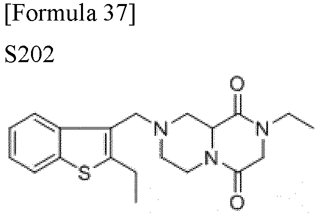

¹H NMR (500 MHz, CDCl₃) δ 7.83 (d, J = 8.0 Hz, 1H), 7.75 (d, J = 8.0 Hz, 1H), 7.32 (t, J = 7.5 Hz, 1H), 7.26 (t, J = 7.5 Hz, 1H), 4.46 (d, J = 13.5 Hz, 1H), 4.04 (d, 11.0 Hz, 1H), 3.96 (d, J = 3.0 Hz, 2H), 3.79 (d, J = 13.0 Hz, 1H), 3.65 (d, J = 13.0 Hz, 1H), 3.54 (d, J = 11.0 Hz, 1H), 3.43 (m, 2H), 2.94 (q, J = 7.5 Hz, 2H), 2.82 (d, J = 11.5 Hz, 1H), 2.69 (dt, J = 3.0, 12.5 Hz, 1H), 2.11 (d, J = 11.5 Hz, 1H), 2.03 (dt, J = 3.0, 11.5 Hz, 1H), 1.33 (t, J = 7.5 Hz, 3H), 1.15 (t, J = 7.3 Hz, 3H).

¹³C NMR (126 MHz, CDCl₃) δ 163.21, 162.04, 146.52, 140.70, 138.21, 126.02, 124.11, 123.85, 122.42, 122.27, 57.55, 57.05, 53.44, 51.37, 48.81, 41.60, 41.12, 22.26, 16.35, 11.84.

MS (ESI) m/z for C₂₀H₂₅N₃O₂S [M+H]⁺: calcd 372.1740, found 372.1734.

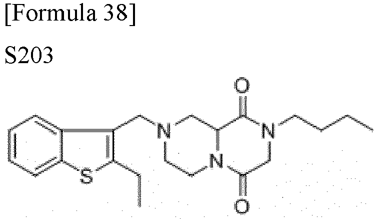

¹H NMR (500 MHz, CDCl₃) δ 7.83 (d, J = 8.0 Hz, 1H), 7.75 (d, J = 7.5 Hz, 1H), 7.32 (t, J = 7.5 Hz, 1H), 7.26 (t, J = 7.5 Hz, 1H), 4.45 (d, J = 13.0 Hz, 1H), 4.05 (d, 11.0 Hz, 1H), 3.96 (s, 2H), 3.80 (d, J = 13.0 Hz, 1H), 3.65 (d, J = 13.0 Hz, 1H), 3.55 (d, J = 11.5 Hz, 1H), 3.37 (t, J = 7.5 Hz, 2H), 2.94 (m, 2H), 2.82 (d, J = 11.0 Hz, 1H), 2.69 (dt, J = 3.5, 12.5 Hz, 1H), 2.11 (d, J = 11.0 Hz, 1H), 2.03 (dt, J = 3.0, 11.0 Hz, 1H), 1.54 (m, J = 3.6 Hz, 2H), 1.33 (t, J = 7.5 Hz, 5H), 0.94 (t, J = 7.5 Hz, 3H).

¹³C NMR (126 MHz, CDCl₃) δ 163.45, 162.09, 146.51, 140.69, 138.21, 126.01, 124.10, 123.85, 122.42, 122.27, 57.53, 57.11, 53.44, 51.35, 49.35, 45.98, 41.61, 28.63, 22.25, 20.13, 16.34, 13.96.

MS (ESI) m/z for C₂₂H₂₉N₃O₂S [M+H]⁺: calcd 400.2053, found 400.2042.

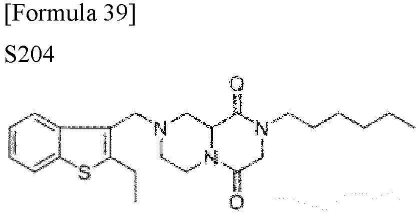

¹H NMR (500 MHz, CDCl₃) δ 7.83 (d, J = 8.0 Hz, 1H), 7.75 (d, J = 8.0 Hz, 1H), 7.32 (t, J = 7.5 Hz, 1H), 7.26 (t, J = 7.5 Hz, 1H), 4.46 (d, J = 13.0 Hz, 1H), 4.04 (d, 11.0 Hz, 1H), 3.96 (s, 2H), 3.79 (d, J = 13.0 Hz, 1H), 3.65 (d, J = 13.0 Hz, 1H), 3.55 (d, J = 11.0 Hz, 1H), 3.39-3.34 (m, 2H), 2.97-2.91 (m, 2H), 2.82 (d, J = 11.5 Hz, 1H), 2.69 (dt, J = 3.5, 12.5 Hz, 1H), 2.11 (t, J = 11.3 Hz, 1H), 2.03 (dt, J = 3.5, 12.0 Hz, 1H), 1.58-1.51 (m, 2H), 1.33 (t, J = 7.5 Hz, 3H), 1.29 (s, 6H) 0.89 (t, J = 6.5 Hz, 3H).

¹³C NMR (126 MHz, CDCl₃) δ 163.42, 162.09, 146.51, 140.69, 138.20, 126.01, 124.10, 123.85, 122.42, 122.26, 57.53, 57.10, 53.43, 51.37, 49.35, 46.24, 41.60, 31.63, 26.55, 26.52, 22.73, 22.25, 16.34, 14.23.

MS (ESI) m/z for C₂₄H₃₃N₃O₂S [M+H]⁺: calcd 428.2366, found 428.2352.

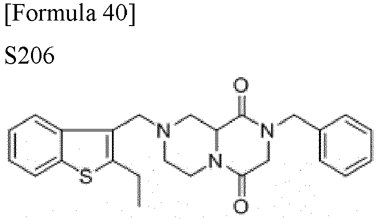

¹H NMR (500 MHz, CDCl₃) δ 7.84 (d, J = 8.0 Hz, 1H), 7.76 (d, J = 8.0 Hz, 1H), 7.37-7.23 (m, 7H), 4.57 (dd, J = 14.5, 32.0 Hz, 2H), 4.43 (d, J = 13.0 Hz, 1H), 4.12 (d, J = 11.0 Hz, 1H), 3.86 (s, 2H), 3.81 (d, J = 13.0 Hz, 1H), 3.66 (d, J = 13.0 Hz, 1H), 3.61 (d, J = 11.0 Hz, 1H), 2.94 (q, J = 7.5 Hz, 2H), 2.81 (d, J = 11.0 Hz, 1H), 2.68 (dt, J = 3.5, 12.5 Hz, 1H), 2.16 (t, J = 11.0 Hz, 1H), 2.03 (dt, J = 3.0, 11.5 Hz, 1H), 1.33 (t, J = 7.5 Hz, 3H).

¹³C NMR (126 MHz, CDCl₃) δ 163.70, 161.90, 146.55, 140.70, 138.24, 135.16, 129.19, 128.73, 128.45, 126.00, 124.13, 123.89, 122.43, 122.31, 57.61, 57.14, 53.46, 51.33, 49.55, 48.77, 41.62, 22.28, 16.37.

MS (ESI) m/z for C₂₅H₂₇N₃O₂S [M+H]⁺: calcd 434.1897, found 434.1892.

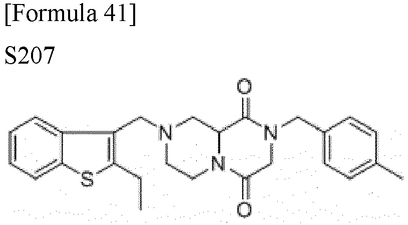

¹H NMR (500 MHz, CDCl₃) δ 7.84 (d, J = 8.0 Hz, 1H), 7.76 (d, J = 8.0 Hz, 1H), 7.33 (t, J = 7.5 Hz, 1H), 7.27 (t, J = 7.5 Hz, 1H), 7.14 (s, 4H), 4.53 (dd, J = 14.5, 35.5 Hz, 2H), 4.43 (d, J = 13.0 Hz, 1H), 4.11 (d, J = 11 Hz, 1H), 3.84 (s, 2H), 3.81 (d, J = 13.0 Hz, 1H), 3.66 (d, J = 13.0 Hz, 1H), 3.60 (d, J = 11.5 Hz, 1H), 2.94 (q, J = 7.5 Hz, 2H), 2.81 (d, J = 10.0 Hz, 1H), 2.67 (dt, J = 3.5, 12.5 Hz, 1H), 2.33 (s, 3H), 2.15 (t, J = 11.0 Hz, 1H), 2.02 (dt, J = 3.5, 11.5 Hz, 1H), 1.33 (t, J = 7.5 Hz, 3H).

¹³C NMR (126 MHz, CDCl₃) δ 163.61, 161.97, 146.54, 140.70, 138.24, 138.23, 132.11, 129.84, 128.77, 126.01, 124.13, 123.88, 122.43, 122.30, 57.62, 57.14, 53.46, 51.34, 49.28, 48.67, 41.60, 22.27, 21.37, 16.37.

MS (ESI) m/z for C₂₆H₂₉N₃O₂S [M+H]⁺: calcd 448.2053, found 448.2055.

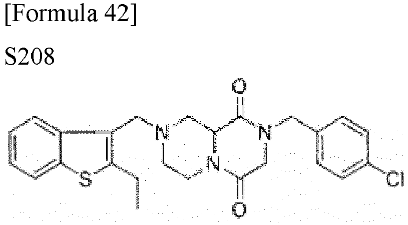

¹H NMR (500 MHz, CDCl₃) δ 7.84 (d, J = 8.0 Hz, 1H), 7.77 (d, J = 8.0 Hz, 1H), 7.35-7.30 (m,3H), 7.27 (t, J = 7.5 Hz, 1H), 7.20 (d, J = 8.5 Hz, 2H), 4.53 (dd, J = 14.5, 27.0 Hz, 2H), 4.44 (d, J = 13.0 Hz, 1H), 4.12 (d, J = 11.0 Hz, 1H), 3.86 (s, 2H), 3.81 (d, J = 13.0 Hz, 1H), 3.67 (d, J = 13.0 Hz, 1H), 3.59 (d, J = 11.5 Hz, 1H), 2.98-2.91 (m, 2H), 2.82 (d, J = 11.5 Hz, 1H), 2.69 (dt, J = 3.0, 12.5 Hz, 1H), 2.15 (t, J = 11.5 Hz, 1H), 2.04 (dt, J = 3.0, 11.5 Hz, 1H), 1.34 (t, J = 7.5 Hz, 3H).

¹³C NMR (126 MHz, CDCl₃) δ 163.79, 161.68, 146.57, 140.67, 138.24, 134.42, 133.72, 130.09, 129.38, 125.93, 124.13, 123.89, 122.40, 122.31, 57.57, 57.07, 53.44, 51.32, 48.97, 48.83, 41.65, 22.27, 16.36.

MS (ESI) m/z for C₂₅H₂₆ClN₃O₂S [M+H]⁺: calcd 468.1507, found 468.1477.

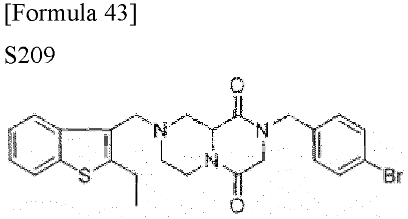

¹H NMR (500 MHz, CDCl₃) δ 7.83 (d, J = 8.0 Hz, 1H), 7.76 (d, J = 8.0 Hz, 1H), 7.47 (d, J = 8.0 Hz, 2H), 7.33 (t, J = 7.5 Hz, 1H), 7.27 (t, J = 7.5 Hz, 1H), 7.13 (d, J = 8.0 Hz, 2H), 4.51 (dd, J = 14.5, 24.0 Hz, 2H), 4.43 (d, J = 13.5 Hz, 1H), 4.11 (d, J = 10.5 Hz, 1H), 3.85 (s, 2H), 3.81 (d, J = 13.0 Hz, 1H), 3.67 (d, J = 13.0 Hz, 1H), 3.59 (d, J = 11.0 Hz, 1H), 2.98-2.91 (m, 2H), 2.82 (d, J = 11.5 Hz, 1H),2.68 (dt, J = 3.0, 12.5 Hz, 1H), 2.14 (t, J = 11.5 Hz, 1H), 2.03 (dt, J = 3.0, 11.5 Hz, 1H), 1.33 (t, J = 7.5 Hz, 3H).

¹³C NMR (126 MHz, CDCl₃) δ 163.80, 161.66, 146.57, 140.67, 138.23, 134.25, 132.33, 130.41, 125.94, 124.13, 123.89, 122.52, 122.40, 122.31, 57.56, 57.06, 53.44, 51.32, 49.03, 48.84, 41.65, 22.27, 16.37.

MS (ESI) m/z for C₂₅H₂₆BrN₃O₂S [M+H]⁺: calcd 512.1002, found 512.0997.

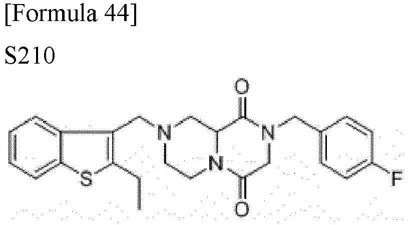

¹H NMR (500 MHz, CDCl₃) δ 7.84 (d, J = 8.0 Hz, 1H), 7.76 (d, J = 8.0 Hz, 1H), 7.33 (t, J = 7.5 Hz, 1H), 7.29-7.22 (m, 3H), 7.03 (t, J = 8.5 Hz, 2H), 4.53 (dd, J = 14.5, 36.0 Hz, 2H), 4.43 (d, J = 13.0 Hz, 1H), 4.11 (d, J = 11.0 Hz, 1H), 3.85 (s, 2H), 3.81 (d, J = 13.0 Hz, 1H), 3.67 (d, J = 13.0 Hz, 1H), 3.59 (d, J = 11.5 Hz, 1H), 2.94 (q, J = 7.0 Hz, 2H), 2.82 (d, J = 11.5 Hz, 1H), 2.68 (dt, J = 3.0, 12.5 Hz, 1H), 2.15 (t, J = 11.0 Hz, 1H), 2.03 (dt, J = 3.5, 12.0 Hz, 1H), 1.33 (t, J = 7.5 Hz, 3H).

¹³C NMR (126 MHz, CDCl₃) δ163.79, 163.73, 161.83, 161.76, 146.57, 140.68, 138.24, 131.07, 131.04, 130.56, 130.50, 125.96, 124.13, 123.89, 122.41, 122.31, 116.21, 116.04, 57.58, 57.07, 53.44, 51.32, 48.88, 48.74, 41.64, 22.27, 16.36.

MS (ESI) m/z for C₂₅H₂₆FN₃O₂S [M+H]⁺: calcd 452.1803, found 452.1781.

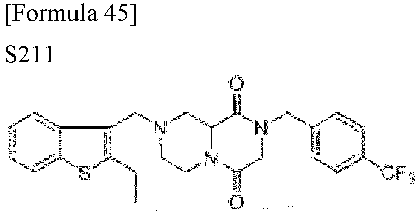

¹H NMR (500 MHz, CDCl₃) δ 7.84 (d, J = 7.5 Hz, 1H), 7.76 (d, J = 8.0 Hz, 1H), 7.61 (d, J = 8.0 Hz, 2H), 7.38 (d, J = 8.0 Hz, 2H), 7.33 (t, J = 7.5 Hz, 1H), 7.27 (t, J = 7.5 Hz, 1H), 4.62 (s, 2H), 4.44 (d, J = 13.0 Hz, 1H), 4.14 (d, J = 11.0 Hz, 1H), 3.87 (s, 2H), 3.81 (d, J = 13.0 Hz, 1H), 3.68 (d, J = 13.0 Hz, 1H), 3.60 (d, J = 11.0 Hz, 1H), 2.98-2.92 (m, 2H), 2.83 (d, J = 11.5 Hz, 1H), 2.60 (dt, J = 3.0, 12.5 Hz, 1H), 2.16 (t, J = 11.0 Hz, 1H), 2.04 (dt, J = 3.0, 12.0 Hz, 1H), 1.34 (t, J = 7.5 Hz, 3H).

¹³C NMR (126 MHz, CDCl₃) δ 163.94, 161.55, 146.60, 140.67, 139.29, 138.24, 131.13, 130.87, 130.61, 130.35, 128.92, 127.38, 126.22, 126.19, 126.16, 126.13, 125.92, 125.21, 124.13, 123.90, 123.05, 122.40, 122.32, 57.54, 57.04, 53.43, 51.32, 49.20, 49.03, 41.68, 22.27, 16.35.

MS (ESI) m/z for C₂₆H₂₆F₃N₃O₂S [M+H]⁺: calcd 502.1771, found 502.1766.

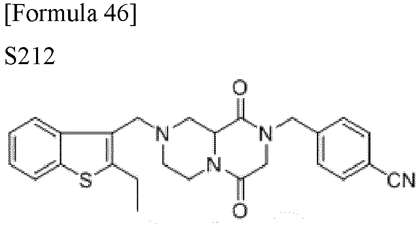

¹H NMR (500 MHz, CDCl₃) δ 7.83 (d, J = 8.0 Hz, 1H), 7.76 (d, J = 8.0 Hz, 1H), 7.64 (d, J = 8.5 Hz, 2H), 7.36 (d, J = 8.5 Hz, 2H), 7.33 (t, J = 7.5 Hz, 1H), 7.30-7.25 (m, 1H), 4.61 (d, J = 3.0 Hz, 2H), 4.44 (d, J = 13.5 Hz, 1H), 4.14 (d, J = 8.5 Hz, 1H), 3.88 (s, 2H), 3.81 (d, J = 13.0 Hz, 1H), 3.68 (d, J = 13.0 Hz, 1H), 3.58 (d, J = 11.0 Hz, 1H), 2.98-2.92 (m, 2H), 2.84 (d, J = 11.0 Hz, 1H), 2.71 (dt, J = 3.0, 12.5 Hz, 1H), 2.16 (t, J = 11.0 Hz, 1H), 2.06 (dt, J = 3.0, 12.5 Hz, 1H), 1.34 (t, J = 7.5 Hz, 3H).

¹³C NMR (126 MHz, CDCl₃) δ 164.06, 161.42, 146.61, 140.65, 140.64, 138.24, 132.98, 129.16, 125.87, 124.13, 123.91, 122.38, 122.32, 118.57, 112.43, 57.50, 56.98, 53.42, 51.31, 49.34, 49.20, 41.71, 22.27, 16.37.

MS (ESI) m/z for C₂₆H₂₆N₄O₂S [M+H]⁺: calcd 459.1849, found 459.1828.

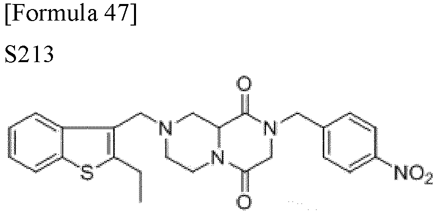

¹H NMR (500 MHz, CDCl₃) δ 8.20 (d, J = 8.5 Hz, 2H), 7.84 (d, J = 8.0 Hz, 1H), 7.76 (d, J = 7.5 Hz, 1H), 7.43 (d, J = 8.5 Hz, 2H), 7.33 (t, J = 7.5 Hz, 1H), 7.28 (t, J = 7.5 Hz, 1H), 4.66 (s, 2H), 4.45 (d, J = 13.0 Hz, 1H), 4.16 (d, J = 11.0 Hz, 1H), 3.91 (s, 2H), 3.82 (d, J = 13.0 Hz, 1H), 3.69 (d, J = 13.0 Hz, 1H), 3.59 (d, J = 11.0 Hz, 1H), 2.98-2.92 (m, 2H), 2.85 (d, J = 11.5 Hz, 1H), 2.71 (dt, J = 3.0, 12.5 Hz, 1H), 2.17 (t, J = 11.5 Hz, 1H), 2.06 (dt, J = 3.0, 12.0 Hz, 1H), 1.34 (t, J = 7.5 Hz, 3H).

¹³C NMR (126 MHz, CDCl₃) δ 164.11, 161.36, 148.01, 146.62, 142.63, 140.65, 138.24, 129.30, 125.87, 124.39, 124.13, 123.91, 122.38, 122.32, 57.50, 56.98, 53.42, 51.32, 49.27, 49.10, 41.72, 22.27, 16.36.

MS (ESI) m/z for C₂₅H₂₆N₄O₄S [M+H]⁺: calcd 479.1748, found 479.1727.

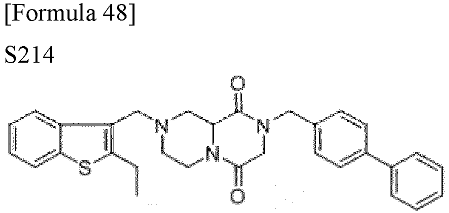

¹H NMR (500 MHz, CDCl₃) δ 7.84 (d, J = 8.0 Hz, 1H), 7.76 (d, J = 8.0 Hz, 1H), 7.56 (d, J = 8.0 Hz, 4H), 7.43 (t, J = 7.5 Hz, 2H), 7.37-7.24(m, 5H), 4.61 (dd, J = 14.5, 31.5 Hz, 2H), 4.44 (d, J = 13.0 Hz, 1H), 4.13 (d, J = 11.0 Hz, 1H), 3.90 (s, 2H), 3.82 (d, J = 13.0 Hz, 1H), 3.67 (d, J = 13.0 Hz, 1H), 3.62 (d, J = 11.0 Hz, 1H), 2.98-2.92 (m, 2H), 2.82 (d, J = 11.0 Hz, 1H), 2.68 (dt, J = 3.0, 12.5 Hz, 1H), 2.18 (t, J = 11.3 Hz, 1H), 2.04 (dt, J = 3.0, 12.0 Hz, 1H), 1.33 (t, J = 7.5 Hz, 3H).

¹³C NMR (126 MHz, CDCl₃) δ 163.75, 161.91, 146.56, 141.47, 140.71, 138.25, 134.14, 129.22, 129.08, 127.93, 127.76, 127.33, 126.01, 124.15, 123.90, 122.44, 122.32, 57.64, 57.14, 53.47, 51.35, 49.30, 48.85, 41.64, 22.29, 16.38.

MS (ESI) m/z for C₃₁H₃₁N₃O₂S [M+H]⁺: calcd 510.2210, found 510.2210.

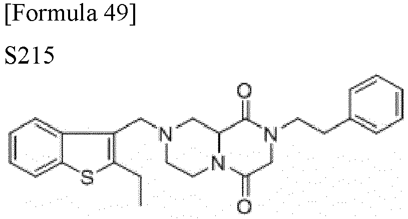

¹H NMR (500 MHz, CDCl₃) δ 7.83 (d, J = 8.0 Hz, 1H), 7.76 (d, J = 7.5 Hz, 1H), 7.35-7.21 (m, 5H), 7.19 (d, J = 7.0 Hz, 2H), 4.42 ( d, J = 13.0, 1H), 4.01 (d, J = 11.0 Hz, 1H), 3.81-3.75 (m, 3H), 3.66-3.55 (m, 4H), 3.51 (d, J = 11.0 Hz, 1H), 2.97-2.86 (m, 3H), 2.81 (d, J = 11.5 Hz, 1H), 2.67 (dt, J = 3.0, 12.5 Hz, 1H), 2.04-1.97 (m, 2H), 1.33 (t, J = 7.5 Hz, 3H).

¹³C NMR (126 MHz, CDCl₃) δ 163.54, 161.88, 146.54, 140.71, 138.23, 138.15, 128.98, 128.94, 127.06, 126.00, 124.12, 123.88, 122.42, 122.30, 57.49, 57.01, 53.45, 51.41, 50.14, 48.13, 41.62, 33.09, 22.28, 16.37.

MS (ESI) m/z for C₂₆H₂₉N₃O₂S [M+H]⁺: calcd 448.2053, found 448.2026.

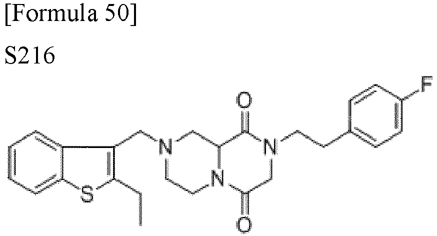

¹H NMR (500 MHz, CDCl₃) δ 7.82 (d, J = 8.0 Hz, 1H), 7.76 (d, J = 8.0 Hz, 1H), 7.33 (t, J = 7.5 Hz, 1H), 7.27 (q, J = 7.5 Hz, 1H), 7.15 (dd, J = 5.5, 8.0 Hz, 2H), 6.99 (t, J = 8.5 Hz, 2H), 4.43 (d, J = 13.0 Hz, 1H), 4.01 (d, J = 11.0 Hz, 1H), 3.83 (s, 2H), 3.78 (d, J = 13.5 Hz, 1H), 3.65 (d, J = 13.5 Hz, 1H), 3.63-3.52 (m, 2H), 3.50 (d, J = 11.5 Hz, 1H), 2.97-2.91 (m, 2H), 2.88-2.80 (m, 3H), 2.68 (dt, J = 3.5, 12.5 Hz, 1H), 2.05-1.98 (m, 2H), 1.33 (t, J = 7.5 Hz, 3H).

¹³C NMR (126 MHz, CDCl₃) δ 163.58, 162.96, 161.78, 161.01, 146.57, 140.69, 138.23, 133.74, 133.72, 130.41, 130.35, 125.96, 124.12, 123.87, 122.40, 122.30, 115.88, 115.72, 57.45, 56.96, 53.43, 51.41, 50.00, 47.93, 41.63, 32.21, 22.26, 16.35.

MS (ESI) m/z for C₂₆H₂₈FN₃O₂S [M+H]⁺: calcd 466.1959, found 466.1935.

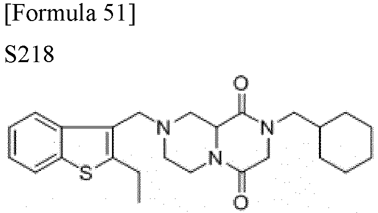

¹H NMR (500 MHz, CDCl₃) δ 7.83 (d, J = 8.0 Hz, 1H), 7.76 (d, J = 8.0 Hz, 1H), 7.34-7.25 (m, 2H), 4.45 (d, J = 13.0 Hz, 1H), 4.07 (d, J = 11.0 Hz, 1H), 3.96 (s, 2H), 3.80 (d, J = 13.0 Hz, 1H), 3.65 (d, J = 13.0 Hz, 1H), 3.56 (d, J = 11.0 Hz, 1H), 3.30-3.15 (m, 2H), 2.94 (q, J = 7.5 Hz, 2H), 2.82 (d, J = 11.5 Hz, 1H), 2.72-2.67 (m, 1H), 2.12 (t, J = 11.5 Hz, 1H), 2.06-2.01 (m, 1H), 1.74-1.60 (m, 6H), 1.32 (t, J = 7.5 Hz, 3H), 1.26-1.14 (m, 3H), 1.00-0.93 (m, 2H).

¹³C NMR (176 MHz, CDCl₃) δ 163.61, 161.95, 146.27, 140.48, 138.01, 125.81, 123.88, 123.63, 122.21, 122.04, 57.29, 57.01, 53.22, 52.01, 51.08, 49.91, 41.40, 35.17, 30.60, 30.57, 26.24, 25.67, 22.02, 16.09.

MS (ESI) m/z for C₂₅H₃₃N₃O₂S [M+H]⁺: calcd 440.2366, found 440.2360.

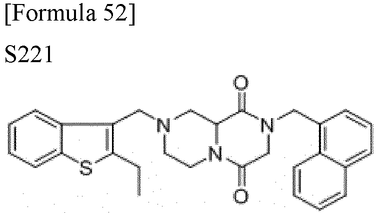

¹H NMR (500 MHz, CDCl₃) δ 8.06-8.03 (m, 1H), 7.91-7.89(m, 1H), 7.85 (t, J = 8.0 Hz, 2H), 7.78 (d, J = 8.0 Hz, 1H), 7.76-7.52 (m, 2H), 7.45-7.39 (m, 2H), 7.34 (t, J = 7.2 Hz, 1H), 7.29 (t, J = 7.2 Hz, 1H), 5.12 (d, J = 14.5, 1H), 5.06 (d, J = 14.5, 1H), 4.40 (d, J = 13.0 Hz, 1H), 4.15 (d, J = 11.0 Hz, 1H), 3.82 (d, J = 13.0 Hz, 1H), 3.73 (s, 2H), 3.68-3.65 (m, 2H), 2.98-2.93 (m, 2H), 2.80 (d, J = 11.5 Hz, 1H), 2.69-2.63 (m, 1H), 2.15 (t, J = 11.0 Hz, 1H), 2.02-1.97 (m, 1H), 1.35 (t, J = 7.5 Hz, 3H).

¹³C NMR (176 MHz, CDCl₃) δ 163.04, 161.51, 146.32, 140.49, 138.02, 133.99, 131.50, 130.12, 129.48, 128.88, 128.56, 126.90, 126.25, 125.77, 125.13, 123.90, 123.65, 123.60, 122.18, 122.07, 57.47, 56.89, 53.21, 51.09, 48.16, 47.25, 41.28, 22.02, 16.11.

MS (ESI) m/z for C₂₉H₂₉N₃O₂S [M+H]⁺: calcd 484.2053, found 484.2053.

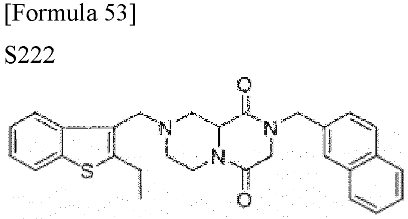

¹H NMR (500 MHz, CDCl₃) δ 7.86-7.80 (m, 4H), 7.77 (d, J = 8.0 Hz, 1H), 7.71 (s, 1H), 7.54-7.47 (m, 2H), 7.38-7.32 (m, 2H), 7.28 (t, J = 7.5 Hz, 1H), 4.80 (d, J = 14.5, 1H), 4.70 (d, J = 14.5, 1H), 4.44 (d, J = 13.5 Hz, 1H), 4.16 (d, J = 9.0 Hz, 1H), 3.91 (s, 2H), 3.83 (d, J = 13.0 Hz, 1H), 3.68 (d, J = 13.0 Hz, 1H), 3.64 (d, J = 11.0 Hz, 1H), 2.98-2.93 (m, 2H), 2.83 (d, J = 11.0 Hz, 1H), 2.72-2.67 (m, 1H), 2.19 (t, J = 11.3 Hz, 1H), 2.06-2.01 (m, 1H), 1.34 (t, J = 7.5 Hz, 3H).

¹³C NMR (176 MHz, CDCl₃) δ 163.56, 161.64, 146.32, 140.49, 138.03, 133.23, 133.04, 132.34, 129.02, 127.77, 127.75, 127.72, 126.54, 126.36, 125.99, 125.77, 123.90, 123.65, 122.20, 122.07, 57.43, 56.92, 53.23, 51.10, 49.52, 48.58, 41.38, 22.03, 16.11.

MS (ESI) m/z for C₂₉H₂₉N₃O₂S [M+H]⁺: calcd 484.2053, found 484.2041.

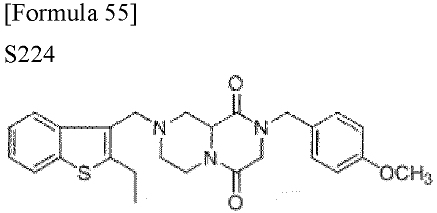

¹H NMR (500 MHz, CDCl₃) δ 7.84 (d, J = 8.0 Hz, 1H), 7.77 (d, J = 8.0 Hz, 1H), 7.33 (t, J = 7.5 Hz, 1H), 7.29 (d, J = 7.5 Hz, 1H), 7.19 (d, J = 8.5 Hz, 2H), 6.87 (d, J = 8.5 Hz, 2H), 4.57 (d, J = 14.5 Hz, 1H), 4.47 (d, J = 14.5 Hz, 1H), 4.44 (d, J = 16.0 Hz, 1H), 4.11 (d, J = 8.5 Hz, 1H), 3.85 (s, 2H), 3.82 (d, J = 13.0 Hz, 1H), 3.80 (s, 3H), 3.66 (d, J = 13.0 Hz, 1H), 3.60 (d, J = 11.0 Hz, 1H), 2.98-2.93 (m, 2H), 2.82 (d, J = 11.5 Hz, 1H), 2.71-2.65 (m, 1H), 2.15 (t, J = 11.3 Hz, 1H), 2.05-2.00 (m, 1H), 1.34 (t, J = 7.5 Hz, 3H).

¹³C NMR (176 MHz, CDCl₃) δ 163.35, 161.75, 159.56, 146.31, 140.49, 138.02, 129.96, 127.01, 125.79, 123.89, 123.64, 122.20, 122.07, 114.32, 57.41, 56.90, 55.31, 53.23, 51.11, 48.75, 48.36, 41.36, 22.03, 16.10.

MS (ESI) m/z for C₂₆H₂₉N₃O₃S [M+H]⁺: calcd 464.2002, found 464.1991.

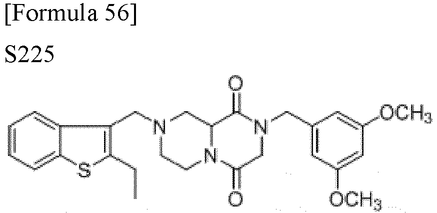

¹H NMR (500 MHz, CDCl₃) δ 7.84 (d, J = 8.0 Hz, 1H), 7.77 (d, J = 8.0 Hz, 1H), 7.33 (t, J = 7.5 Hz, 1H), 7.28 (t, J = 8.0 Hz, 1H), 6.39 (s, 3H), 4.56 (d, J = 14.5 Hz, 1H), 4.48-4.42 (m, 2H), 4.13 (d, J = 11.0 Hz, 1H), 3.88 (s, 2H), 3.81 (d, J = 13.0 Hz, 1H), 3.78 (s, 6H), 3.67 (d, J = 13.0 Hz, 1H), 3.60 (d, J = 11.0 Hz, 1H), 2.97-2.93 (m, 2H), 2.83 (d, J = 11.5 Hz, 1H), 2.72-2.66 (m, 1H), 2.16 (t, J = 11.3 Hz, 1H), 2.07-2.02 (m, 1H), 1.34 (t, J = 7.8 Hz, 3H).

¹³C NMR (176 MHz, CDCl₃) δ 163.46, 161.66, 161.24, 146.32, 140.48, 138.01, 137.17, 125.77, 123.89, 123.64, 122.18, 122.06, 106.46, 99.89, 57.39, 56.88, 55.36, 53.21, 51.13, 49.39, 48.57, 41.37, 22.02, 16.10.

MS (ESI) m/z for C₂₇H₃₁N₃O₄S [M+H]⁺: calcd 494.2108, found 494.2101.

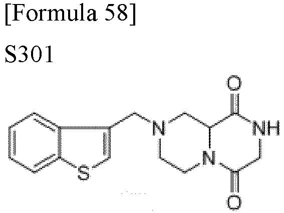

¹H NMR (500 MHz, CDCl₃) δ 7.95 (d, J = 7.0 Hz, 1H), 7.86 (d, J = 7.0 Hz, 1H), 7.40-7.35 (m, 2H), 7.30 (s, 1H), 6.25 (s, 1H), 4.52 (d, J = 13.0 Hz, 1H),4.12 (d, J = 12.5 Hz, 1H), 4.06 (s, 2H), 3.89 (d, J = 13.5 Hz, 1H), 3.77 (d, J = 13.5 Hz, 1H), 3.53 (d, J = 11.0 Hz, 1H), 2.93 (d, J = 11.5 Hz, 1H), 2.82-2.76 (m, 1H), 2.17 (t, J = 11.3 Hz, 1H), 2.12-2.07 (m, 1H).

¹³C NMR (126 MHz, CDCl₃) δ 166.12, 161.93, 140.93, 138.87, 132.20, 125.27, 124.72, 124.23, 123.02, 122.83, 57.12, 56.55, 56.49, 51.81, 44.81, 41.75.

MS (ESI) m/z for C₁₆H₁₇N₃O₂S [M+H]⁺: calcd 316.112, found 316.1115.

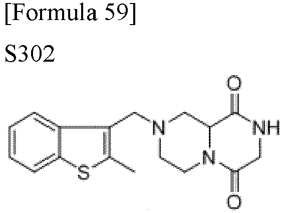

¹H NMR (500 MHz, CDCl₃) δ 7.81 (d, J = 7.5 Hz, 1H), 7.75 (d, J = 8.0 Hz, 1H), 7.33 (t, J = 7.5 Hz, 1H), 7.28 (d, J = 7.5 Hz, 1H), 6.21 (s, 1H), 4.49 (d, J = 13.0 Hz, 1H), 4.08 (d, J = 11.0 Hz, 1H), 4.06 (s, 2H), 3.79 (d, J = 13.0 Hz, 1H), 3.67 (d, J = 13.0 Hz, 1H), 3.50 (d, J = 10.5 Hz, 1H), 2.86 (d, J = 11.5 Hz, 1H), 2.76-2.70 (m, 1H), 2.54 (s, 3H), 2.18 (t, J = 11.3 Hz, 1H), 2.11-2.06 (m, 1H).

¹³C NMR (176 MHz, CDCl₃) δ 166.05, 161.70, 140.47, 138.58, 137.99, 126.71, 123.94, 123.69, 121.99, 121.89, 56.96, 56.29, 53.25, 51.33, 44.54, 41.53, 14.07.

MS (ESI) m/z for C₁₇H₁₉N₃O₂S [M+H]⁺: calcd 330.1276, found 330.1273.

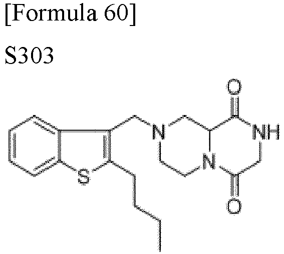

¹H NMR (500 MHz, CDCl₃) δ 7.84 (d, J = 8.0 Hz, 1H), 7.76 (d, J = 7.5 Hz, 1H), 7.33 (t, J = 7.2 Hz, 1H), 7.28 (d, J = 7.0 Hz, 1H), 6.23 (s, 1H), 4.49 (d, J = 13.0 Hz, 1H), 4.09 (d, J = 11.0 Hz, 1H), 4.06 (s, 2H), 3.80 (d, J = 13.0 Hz, 1H), 3.67 (d, J = 13.0 Hz, 1H), 3.51 (d, J = 12.0 Hz, 1H), 2.91 (t, J = 7.8 Hz, 2H), 2.85 (d, J = 11.5 Hz, 1H), 2.74-2.68 (m, 1H), 2.18 (t, J = 11.3 Hz, 1H), 2.09-2.04 (m, 1H), 1.72-1.66 (m, 2H), 1.46-1.38 (m, 2H), 0.95 (t, J = 7.5 Hz, 3H).

¹³C NMR (176 MHz, CDCl₃) δ 166.09, 161.72, 144.96, 140.42, 138.11, 126.12, 123.86, 123.65, 122.16, 122.01, 56.97, 56.38, 53.31, 51.28, 44.53, 41.56, 33.70, 28.31, 22.46, 13.91.

MS (ESI) m/z for C₂₀H₂₅N₃O₂S [M+H]⁺: calcd 372.1746, found 372.1748.

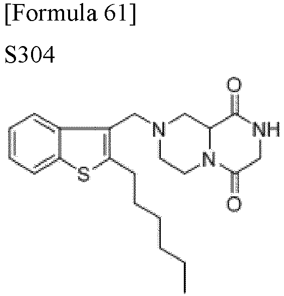

¹H NMR (500 MHz, CDCl₃) δ 7.84 (d, J = 8.0 Hz, 1H), 7.76 (d, J = 8.0 Hz, 1H), 7.32 (t, J = 7.5 Hz, 1H), 7.27 (t, J = 6.8 Hz, 1H), 6.71 (s, 1H), 4.49 (d, J = 13.0 Hz, 1H), 4.08 (d, J = 11.0 Hz, 1H), 4.05 (s, 2H), 3.80 (d, J = 13.0 Hz, 1H), 3.66 (d, J = 13.0 Hz, 1H), 3.50 (d, J = 11.0 Hz, 1H), 2.90 (t, J = 7.5 Hz, 2H), 2.84 (d, J = 11.5 Hz, 1H), 2.73-2.68 (m, 1H), 2.18 (t, J = 11.0 Hz, 1H), 2.09-2.03 (m, 1H), 1.73-1.67 (m, 2H), 1.40-1.37 (m, 2H), 1.33-1.31 (m, 4H), 0.89 (t, J = 7.0 Hz, 3H).

¹³C NMR (176 MHz, CDCl₃) δ 166.06, 161.69, 145.03, 140.44, 138.13, 126.11, 123.86, 123.65, 122.17, 122.02, 56.99, 56.41, 53.32, 51.27, 44.56, 41.57, 31.61, 31.58, 29.06, 28.63, 22.56, 14.08.

MS (ESI) m/z for C₂₂H₂₉N₃O₂S [M+H]⁺: calcd 400.2059, found 400.2070.

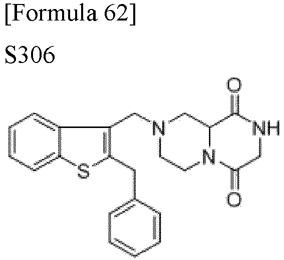

¹H NMR (500 MHz, CDCl₃) δ 7.86 (d, J = 8.0 Hz, 1H), 7.73 (d, J = 8.0 Hz, 1H), 7.35-7.31 (m, 2H), 7.30-7.26 (m, 3H), 7.25-7.22 (m, 2H), 4.45 (d, J = 13.0 Hz, 1H), 4.26 (d, J = 3.5 Hz, 2H), 4.02-4.01 (m, 1H), 4.00 (s, 2H), 3.83 (d, J = 13.0 Hz, 1H), 3.71 (d, J = 13.0 Hz, 1H), 3.46 (d, J = 10.5 Hz, 1H), 2.82 (d, J = 11.5 Hz, 1H), 2.68-2.62 (m, 1H), 2.12 (t, J = 11.3 Hz, 1H), 2.04-1.99 (m, 1H).

¹³C NMR (176 MHz, CDCl₃) δ 166.07, 161.80, 142.67, 140.37, 139.55, 138.66, 128.63, 128.60, 127.25, 126.72, 124.06, 124.01, 122.31, 122.16, 56.94, 56.34, 53.42, 51.35, 44.57, 41.52, 34.54.

MS (ESI) m/z for C₂₃H₂₃N₃O₂S [M+H]⁺: calcd 406.1589, found 406.1605.

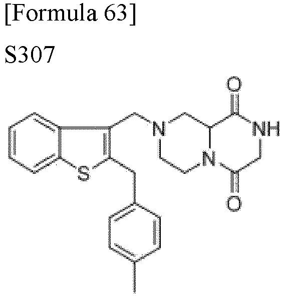

¹H NMR (500 MHz, CDCl₃) δ 7.85 (d, J = 8.0 Hz, 1H), 7.72 (d, J = 8.0 Hz, 1H), 7.35-7.30 (m, 2H), 7.27 (t, J = 7.2 Hz, 1H), 7.11 (q, J = 7.7 Hz, 3H), 4.46 (d, J = 13.0 Hz, 1H), 4.22 (d, J = 4.0 Hz, 2H), 4.03 (d, J = 11.0 Hz, 1H), 4.00 (s, 2H), 3.83 (d, J = 13.0 Hz, 1H), 3.71 (d, J = 13.0 Hz, 1H), 3.47 (d, J = 11.0 Hz, 1H), 2.83 (d, J = 11.5 Hz, 1H), 2.70-2.64 (m, 1H), 2.31 (s, 3H), 2.13 (t, J = 11.3 Hz, 1H), 2.05-2.00 (m, 1H).

¹³C NMR (176 MHz, CDCl₃) δ 166.05, 161.77, 143.19, 140.39, 138.65, 136.48, 136.31, 129.32, 128.50, 127.02, 124.01, 123.94, 122.29, 122.15, 56.96, 56.35, 53.42, 51.36, 44.57, 41.54, 34.15, 21.08.

MS (ESI) m/z for C₂₄H₂₅N₃O₂S [M+H]⁺: calcd 420.1746, found 420.1740.

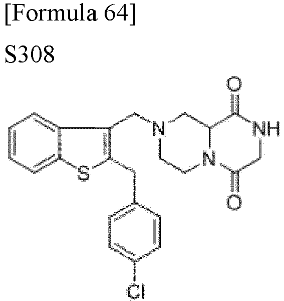

¹H NMR (500 MHz, CDCl₃) δ 7.86 (d, J = 8.0 Hz, 1H), 7.74 (d, J = 8.0 Hz, 1H), 7.34 (t, J = 7.2 Hz, 1H), 7.29 (t, J = 7.2 Hz, 1H), 7.24 (d, J = 7.0 Hz, 2H), 7.16 (d, J = 8.0 Hz, 2H), 4.47 (d, J = 13.0 Hz, 1H), 4.23 (d, J = 3.5 Hz, 2H), 4.01 (s, 2H), 3.99 (d, J = 2.5 Hz, 1H), 3.80 (d, J = 13.0 Hz, 1H), 3.71 (d, J = 13.5 Hz, 1H), 3.43 (d, J = 10.5 Hz, 1H), 2.84 (d, J = 11.5 Hz, 1H), 2.70-2.64 (m, 1H), 2.11-2.02 (m, 2H).

¹³C NMR (176 MHz, CDCl₃) δ 165.81, 162.58, 161.80, 141.87, 140.23, 138.57, 137.99, 132.47, 129.90, 128.71, 127.48, 124.14, 122.30, 122.15, 56.89, 56.20, 53.36, 51.47, 44.54, 41.45, 33.82.

MS (ESI) m/z for C₂₃H₂₂ClN₃O₂S [M+H]⁺: calcd 440.12, found 440.1199.

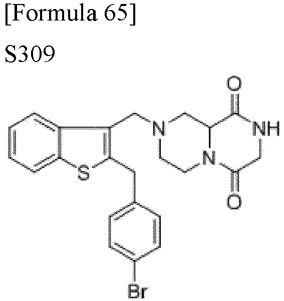

¹H NMR (500 MHz, CDCl₃) δ 7.86 (d, J = 7.5 Hz, 1H), 7.74 (d, J = 7.5 Hz, 1H), 7.40 (d, J = 8.0 Hz, 2H), 7.34 (t, J = 7.2 Hz, 1H), 7.29 (t, J = 7.2 Hz, 1H), 7.11 (d, J = 8.5 Hz, 2H), 4.47 (d, J = 13.0 Hz, 1H), 4.22 (d, J = 3.5 Hz, 2H), 4.03-3.97 (m, 3H), 3.79 (d, J = 13.0 Hz, 1H), 3.71 (d, J = 13.0 Hz, 1H), 3.42 (d, J = 11.0 Hz, 1H), 2.84 (d, J = 11.5 Hz, 1H), 2.70-2.64 (m, 1H), 2.10-2.02 (m, 2H).

¹³C NMR (176 MHz, CDCl₃) δ 165.79, 162.57, 161.79, 141.72, 140.22, 138.56, 138.51, 131.65, 130.28, 127.52, 124.14, 122.30, 122.15, 120.51, 56.88, 56.19, 53.36, 51.48, 44.53, 41.45, 33.88.

MS (ESI) m/z for C₂₃H₂₂BrN₃O₂S [M+H]⁺: calcd 484.0694, found 484.0660.

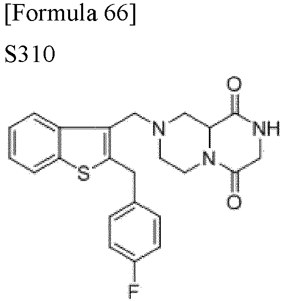

¹H NMR (500 MHz, CDCl₃) δ 7.87 (d, J = 8.0 Hz, 1H), 7.74 (d, J = 8.0 Hz, 1H), 7.36-7.28 (m, 2H), 7.20 (dd, J = 8.5, 5.5 Hz, 2H), 6.98 (t, J = 8.5 Hz, 2H), 4.48 (d, J = 13.0 Hz, 1H), 4.24 (d, J = 3.5 Hz, 2H), 4.01 (s, 3H), 3.82 (d, J = 13.0 Hz, 1H), 3.72 (d, J = 13.0 Hz, 1H), 3.45 (d, J = 11.0 Hz, 1H), 2.84 (d, J = 11.5 Hz, 1H), 2.70-2.65 (m, 1H), 2.12 (t, J = 11.5 Hz, 1H), 2.08-2.02 (m, 1H).

¹³C NMR (176 MHz, CDCl₃) δ 165.94, 162.35, 161.74, 160.96, 142.42, 140.27, 138.56, 135.20, 135.19, 130.04, 129.99, 127.28, 124.93, 124.10, 124.09, 122.28, 122.15, 115.46, 115.34, 56.89, 56.24, 53.36, 51.42, 44.51, 41.47, 33.68.

MS (ESI) m/z for C₂₃H₂₂FN₃O₂S [M+H]⁺: calcd 424.1495, found 424.1490.

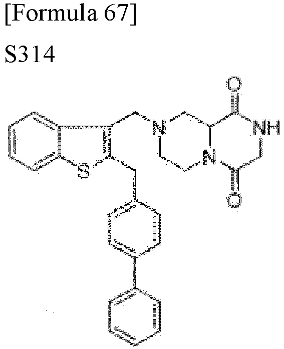

¹H NMR (500 MHz, CDCl₃) δ 7.87 (d, J = 8.0 Hz, 1H), 7.74 (d, J = 8.0 Hz, 1H), 7.56 (d, J = 7.0 Hz, 2H), 7.52 (d, J = 8.0 Hz, 2H), 7.41 (t, J = 7.8 Hz, 2H), 7.36-7.27 (m, 5H), 4.47 (d, J = 13.0 Hz, 1H), 4.30 (d, J = 3.0 Hz, 2H), 4.02-3.96 (m, 3H), 3.84 (d, J = 13.0 Hz, 1H), 3.74 (d, J = 13.5 Hz, 1H), 3.45 (d, J = 11.0 Hz, 1H), 2.88-2.83 (m, 1H), 2.70-2.65 (m, 1H), 2.12-2.03 (m, 2H).

¹³C NMR (176 MHz, CDCl₃) δ 165.92, 162.59, 161.72, 142.46, 140.68, 140.33, 139.57, 138.62, 138.57, 128.97, 128.74, 127.27, 127.22, 126.96, 124.04, 124.01, 122.29, 122.14, 56.88, 56.20, 53.40, 51.43, 44.50, 41.47, 34.13.

MS (ESI) m/z for C₂₉H₂₇N₃O₂S [M+H]⁺: calcd 482.1902, found 482.1898.

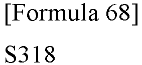

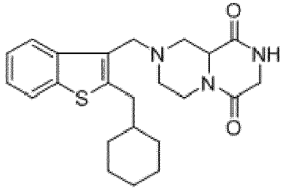

¹H NMR (500 MHz, CDCl₃) δ 7.85 (d, J = 8.0 Hz, 1H), 7.75 (d, J = 8.0 Hz, 1H), 7.32 (t, J = 7.5 Hz, 1H), 7.27 (t, J = 7.5 Hz, 1H), 4.48 (d, J = 13.0 Hz, 1H), 4.06 (d, J = 10.0 Hz, 1H), 4.02 (s, 2H), 3.79 (d, J = 13.0 Hz, 1H), 3.66 (d, J = 13.0 Hz, 1H), 3.48 (d, J = 10.0 Hz, 1H), 2.84 (d, J = 11.5 Hz, 1H), 2.78 (d, J = 7.5 Hz, 2H), 2.72-2.67 (m, 1H), 2.17 (t, J = 11.0 Hz, 1H), 2.08-2.03 (m, 1H), 1.79-1.64 (m, 5H), 1.27-1.14 (m, 4H), 2.08-2.03 (m, 2H).

¹³C NMR (126 MHz, CDCl₃) δ 166.33, 161.93, 143.80, 140.62, 138.50, 127.14, 124.04, 123.91, 122.49, 122.16, 57.21, 56.64, 53.66, 51.50, 44.78, 41.80, 40.54, 36.52, 33.53, 33.51, 26.61, 26.44.

MS (ESI) m/z for C₂₃H₂₉N₃O₂S [M+H]⁺: calcd 412.2059, found 412.2052.

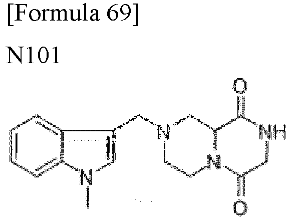

¹H NMR (500 MHz, CDCl₃) δ 7.68 (d, J = 8.0 Hz, 1H), 7.31 (d, J = 8.0 Hz, 1H), 7.22 (t, J = 7.5 Hz, 1H), 7.11 (t, J = 7.5 Hz, 1H), 6.96 (s, 1H), 4.47 (d, J = 13.0 Hz, 1H), 4.05 (d, J = 9.5 Hz, 1H), 3.94 (s, 2H), 3.81 (d, J = 13.5 Hz, 1H), 3.74-3.72 (m, 4H), 3.48 (d, J = 10.5 Hz, 1H), 2.94 (d, J = 11.5 Hz, 1H), 2.79-2.73 (m, 1H), 2.15-2.10 (m, 1H), 2.07-2.02 (m, 1H).

¹³C NMR (126 MHz, CDCl₃) δ 166.49, 162.08, 137.36, 128.84, 128.41, 122.01, 119.74, 119.42, 109.97, 109.53, 57.20, 56.17, 53.44, 51.60, 44.71, 41.80, 32.97.

MS (ESI) m/z for C₁₇H₂₀N₄O₂ [M+H]⁺: calcd 313.1659, found 313.1651. [M+Na]⁺: calcd 335.1478, found 335.1473.

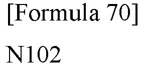

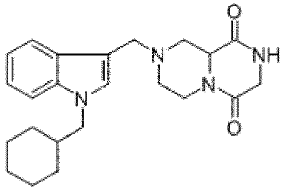

¹H NMR (500 MHz, CDCl₃) δ 7.69 (d, J = 8.0 Hz, 1H), 7.31 (d, J = 8.0 Hz, 1H), 7.21 (t, J = 7.5 Hz, 1H), 7.10 (t, J = 7.5 Hz, 1H), 6.98 (s, 1H), 6.60 (s, 1H), 4.49 (d, J = 13.0 Hz, 1H), 4.13-4.08 (m, 1H), 4.03-4.00 (m, 1H), 3.90 (d, J = 7.5 Hz, 2H), 3.86 (d, J = 13.5 Hz, 1H), 3.72 (d, J = 13.5 Hz, 1H), 3.53 (d, J = 11.5 Hz, 1H), 2.92-2.98 (m, 1H), 2.81-2.75 (m, 1H), 2.12 (t, J = 11.3 Hz, 1H), 2.07-2.02 (m, 1H), 1.87-1.80 (m,1H) 1.73-1.59 (m, 5H), 1.28-1.13 (m, 1H), 1.17 (m, 3H), 0.99 (m, 2H).

¹³C NMR (126 MHz, CDCl₃) δ 166.44, 161.99, 137.00, 128.67, 128.37, 121.79, 119.75, 119.27,109.98, 109.48, 57.14, 56.25, 53.50, 53.14, 51.43, 44.76, 41.77, 38.97, 31.33, 26.53, 25.95.

MS (ESI) m/z for C₂₃H₃₀N₄O₂ [M+H]⁺: calcd 395.2441, found 395.2438. [M+Na]⁺: calcd 417.2261, found 417.2272.

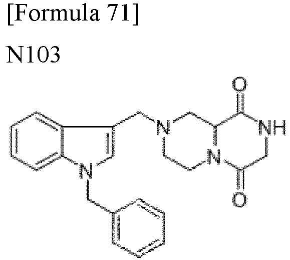

¹H NMR (500 MHz, CDCl₃) δ 7.72 (d, J = 7.5 Hz, 1H), 7.32-7.20 (m, 4H), 7.17 (t, J = 7.5 Hz, 1H), 7.13-7.09 (m, 3H), 7.03 (s, 1H), 5.27 (s, 2H), 4.49 (d, J = 13.0 Hz, 1H), 4.07 (d, J = 11.0 Hz, 1H), 3.96 (s, 2H), 3.84 (d, J = 13.5 Hz, 1H), 3.73 (d, J = 13.5 Hz, 1H), 3.51 (d, J = 11.0 Hz, 1H), 2.95 (d, J = 11.5 Hz, 1H), 2.79-2.74 (m, 1H), 2.10 (t, J = 11.5 Hz, 1H), 2.07-2.01 (m, 1H).

¹³C NMR (126 MHz, CDCl₃) δ 166.45, 162.00, 137.64, 137.09, 129.03, 128.63, 128.17, 127.90, 127.06, 122.25, 119.96, 119.70, 110.84, 110.03, 57.23, 56.34, 53.59, 51.57, 50.22, 44.77, 41.84.

MS (ESI) m/z for C₂₃H₂₄N₄O₂ [M+H]⁺: calcd 389.1972, found 389.1971. [M+Na]⁺: calcd 411.1791, found 411.1798.

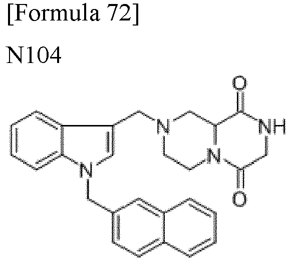

¹H NMR (500 MHz, CDCl₃) δ 7.80-7.72 (m, 3H), 7.54 (s, 1H), 7.46-7.42 (m, 2H), 7.30 (d, J = 8.0 Hz, 1H), 7.22 (d, J = 8.5 Hz, 1H), 7.18-7.11 (m, 2H), 7.12 (t, J = 7.5 Hz, 1H), 7.06 (s, 1H), 5.41 (s, 2H), 4.48 (d, J = 13.5 Hz, 1H), 4.06 (d, J = 11.0 Hz, 1H), 3.94 (s, 2H), 3.84 (d, J = 13.5 Hz, 1H), 3.73 (d, J = 13.5 Hz, 1H), 3.52 (d, J = 10.0 Hz, 1H), 2.95 (d, J = 11.5 Hz, 1H), 2.79-2.73 (m, 1H), 2.10 (t, J = 11.5 Hz, 1H), 2.06-2.01 (m, 1H).

¹³C NMR (126 MHz, CDCl₃) δ 166.46, 162.02, 137.19, 135.08, 133.55, 133.09, 128.96, 128.70, 128.22, 128.07, 127.97, 126.66, 126.35, 125.93, 125.12, 122.32, 120.01, 119.77, 110.95, 110.08, 57.23, 56.35, 53.60, 51.60, 50.43, 44.77, 41.84.

MS (ESI) m/z for C₂₇H₂₆N₄O₂ [M+H]⁺: calcd 439.2128, found 439.2129. [M+Na]⁺: calcd 461.1948, found 461.1957.

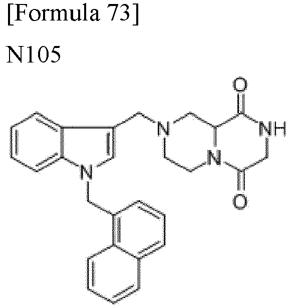

¹H NMR (500 MHz, CDCl₃) δ 7.91-7.74 (m, 2H), 7.77 (dd J = 8.0 Hz, 19.5 Hz, 2H), 7.52-7.48 (m, 2H), 7.34-7.30 (m, 2H), 7.18-7.23 (m, 2H), 7.15 (t, J = 7.5 Hz, 1H), 6.92 (s, 1H), 5.70 (s, 2H), 4.46 (d, J = 12.0 Hz, 1H), 4.04 (d, J = 11.0 Hz, 1H), 3.95 (s, 2H), 3.81 (d, J = 13.5 Hz, 1H), 3.68 (d, J = 13.0 Hz, 1H), 3.49 (d, J = 10.0 Hz, 1H), 2.91 (d, J = 11.5 Hz, 1H), 2.76-2.70 (m, 1H), 2.17 (t, J = 11.5 Hz, 1H), 2.02-1.97 (m, 1H).

¹³C NMR (126 MHz, CDCl₃) δ 166.31, 161.95, 137.25, 133.92, 132.50, 131.12, 129.18, 128.74, 128.65, 128.12, 126.91, 126.28, 125.79, 125.45, 122.83, 122.35, 120.04, 119.85, 110.81, 109.97, 57.15, 56.26, 53.54, 51.47, 48.04, 44.79, 41.76.

MS (ESI) m/z for C₂₇H₂₆N₄O₂ [M+H]⁺: calcd 439.2128, found 439.2128. [M+Na]⁺: calcd 461.1948, found 461.1957.

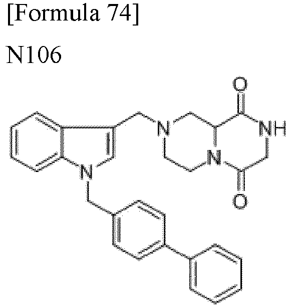

¹H NMR (500 MHz, CDCl₃) δ 7.73 (d, J = 8.0 Hz, 1H), 7.52 (dd, J = 8.0 Hz, 11.0 Hz, 4H), 7.40 (t, J = 7.5 Hz, 2H), 7.28-7.33 (m, 2H), 7.15-7.22 (m, 3H), 7.13 (t, J = 7.5 Hz, 1H), 7.05 (s, 1H), 5.30 (s, 2H), 4.49 (d, J = 13.0 Hz, 1H), 4.07 (d, J = 9.0 Hz, 1H), 3.95 (s, 2H), 3.85 (d, J = 13.5 Hz, 1H), 3.74 (d, J = 13.5 Hz, 1H), 3.52 (d, J = 11.0 Hz, 1H), 2.95 (d, J = 10.0 Hz, 1H), 2.79-2.73 (m, 1H), 2.11 (t, J = 11.3 Hz, 1H), 2.07-2.02 (m, 1H).

¹³C NMR (126 MHz, CDCl₃) δ 166.33, 161.96, 140.87, 140.76, 137.09, 136.61, 129.04, 128.67, 128.20, 127.75, 127.64, 127.52, 127.27, 122.31, 119.98, 119.77, 110.84, 110.06, 57.19, 56.31, 53.58, 51.55, 49.97, 44.79, 41.81.

MS (ESI) m/z for C₂₉H₂₈N₄O₂ [M+H]⁺: calcd 465.2285, found 465.2291. [M+Na]⁺: calcd 487.2104, found 487.2109.

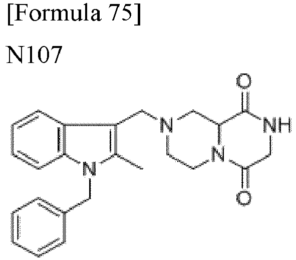

¹H NMR (500 MHz, CDCl₃) δ 7.66-7.64 (m, 1H), 7.27-7.20 (m, 4H), 7.13-7.08 (m, 2H), 6.95 (d, J = 7.0 Hz, 2H), 5.30 (s, 2H), 4.48 (d, J = 11.0 Hz, 1H), 4.08-4.04 (m, 1H), 3.97 (s, 2H), 3.83 (d, J = 13.0 Hz, 1H), 3.70 (d, J = 13.0 Hz, 1H), 3.51-3.49 (m, 1H), 2.94-2.89 (m, 1H), 2.77-2.71 (m, 1H), 2.32 (s, 3H), 2.13 (t, J = 11.3 Hz, 1H), 2.07-2.01 (m, 1H);

¹³C NMR (126 MHz, CDCl₃) δ 166.33, 161.82, 137.87, 136.61, 135.44, 128.90, 128.57, 127.41,126.05, 121.25, 119.58, 118.71, 109.19, 107.21, 57.19, 56.34, 52.51, 51.36, 46.65, 44.64, 41.74,10.59.

MS (ESI) m/z for C₂₄H₂₆N₄O₂ [M+Na]⁺: calcd 425.1948, found 425.1949.

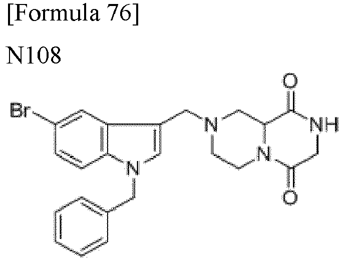

¹H NMR (500 MHz, CDCl₃) δ 7.85 (d, J = 2.0 Hz, 1H), 7.32-7.23 (m, 4H), 7.12-7.04 (m, 4H), 5.25 (s, 2H), 4.51 (d, J = 13.5 Hz, 1H), 4.10 (d, J = 10.5 Hz, 1H), 4.01 (s, 2H), 3.77-3.66 (m, 2H), 3.49 (d, J = 11.0 Hz, 1H), 2.94 (d, J = 11.5 Hz, 1H), 2.82-2.77 (m, 1H), 2.10-2.02 (m, 2H);

¹³C NMR (126 MHz, CDCl₃) NMR (126 MHz, ) δ 166.09, 161.79, 136.91, 135.58, 130.01, 129.02, 128.91, 127.88, 126.75, 124.95, 122.39, 112.92, 111.37, 110.45, 56.90, 56.03, 53.31, 51.49, 50.22, 44.60, 41.56.

MS (ESI) m/z for C₂₃H₂₃BrN₄O₂ [M+H]⁺: calcd 467.1077, found 467.1079.

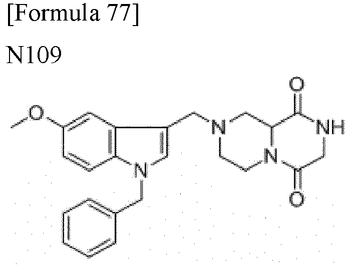

¹H NMR (500 MHz, CDCl₃) δ 7.31-7.26 (m, 2H), 7.17-7.08 (m, 5H), 7.02 (s, 1H), 6.85-6.82 (m, 1H), 5.24 (s, 2H), 4.52-4.48 (m, 1H), 4.11 (m, 1H), 3.99 (s, 2H), 3.85 (s, 3H), 3.83 (d, J = 13.0 Hz, 1H), 3.69 (d, J = 13.5 Hz, 1H), 3.55-3.52 (m, 1H), 2.97 (d, J = 11.5 Hz, 1H), 2.82-2.76 (m, 1H), 2.13 (t, J = 11.5 Hz, 1H), 2.07-2.02 (m, 1H);

¹³C NMR (126 MHz, CDCl₃) δ 166.18, 161.83, 154.13, 137.55, 132.25, 128.87, 128.85, 128.74, 127.74, 126.83, 112.11, 110.68, 109.97, 101.78, 57.02, 56.21, 56.06, 53.50, 51.31, 50.28, 44.65, 41.66.

MS (ESI) m/z for C₂₄H₂₆N₄O₃ [M+H]⁺: calcd 419.2078, found 419.2070.

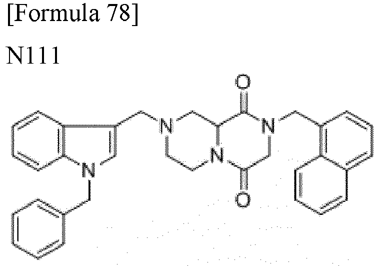

¹H NMR (500 MHz, CDCl₃) δ 8.05 - 8.02 (m, 1H), 7.90 - 7.87 (m, 1H), 7.85 (d, J = 8.5 Hz, 1H), 7.76 - 7.74 (m, 1H), 7.53 - 7.49 (m, 2H), 7.44 - 7.37 (m, 2H), 7.32 - 7.26 (m, 4H), 7.22 - 7.19 (m, 1H), 7.16 - 7.12 (m, 3H), 7.06 (s, 1H), 5.30 (s, 2H), 5.11 (d, J = 14.5 Hz, 1H), 5.03 (d, J = 14.5 Hz, 1H), 4.43 - 4.40 (m, 1H), 4.20 - 4.17 (m, 1H), 3.89 (d, J = 13.0 Hz, 1H), 3.75 (d, J = 13.5 Hz, 1H), 3.72 - 3.69 (m, 3H), 2.93 - 2.90 (m, 1H), 2.76 - 2.70 (m, 1H), 2.11 (t, J = 11.0 Hz, 1H), 2.02 - 1.96 (m, 1H).

¹³C NMR (126 MHz, CDCl₃) NMR (126 MHz, ) δ 163.31, 161.65, 137.51, 136.97, 134.08, 131.62, 130.25, 129.56, 128.98, 128.91, 128.62, 128.54, 128.08, 128.03, 127.98, 127.77, 127.02, 126.94, 126.36, 125.26, 123.72, 122.12, 119.83, 119.59, 110.73, 109.90, 57.62, 56.76, 53.40, 51.17, 50.10, 48.28, 47.33, 41.42.

MS (ESI) m/z for C₁₈H₂₁N₃O₂S [M+H]⁺: calcd 529.2598, found 529.2598.

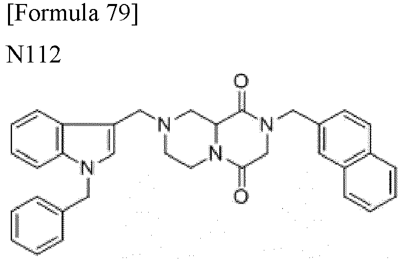

¹H NMR (500 MHz, CDCl₃) δ 7.82 - 7.78 (m, 3H), 7.74 (d, J = 8.0 Hz, 1H), 7.68 (s, 1H), 7.50 - 7.45 (m, 2H), 7.37 - 7.35 (m, 1H), 7.30 - 7.23 (m, 4H), 7.20 - 7.17 (m, 1H), 7.14 - 7.10 (m, 3H), 7.04 (s, 1H), 5.28 (s, 2H), 4.71 (dd, J = 14.5 Hz, 37.0 Hz, 2H), 4.46 - 4.43 (m, 1H), 4.19 - 4.16 (m, 1H), 3.90-85 (m, 3H), 3.74 (d, J = 13.0 Hz, 1H), 3.68 - 3.65 (m, 1H), 2.94 - 2.92 (m, 1H), 2.78 - 2.72 (m, 1H), 2.13 (t, J = 11.0 Hz, 1H), 2.05 - 1.99 (m, 1H).

¹³C NMR (126 MHz, CDCl₃) δ 163.80, 161.73, 137.52, 136.98, 133.33, 133.13, 132.48, 129.12, 128.90, 128.52, 128.09, 128.04, 127.98, 127.88, 127.83, 126.95, 126.91, 126.63, 126.44, 126.14, 126.11, 122.13, 119.85, 119.58, 110.76, 109.90, 57.58, 56.77, 53.44, 51.26, 50.09, 49.59, 48.68, 41.52.

MS (ESI) m/z for C₁₈H₂₁N₃O₂S [M+H]⁺: calcd 529.2598, found 529.2605.

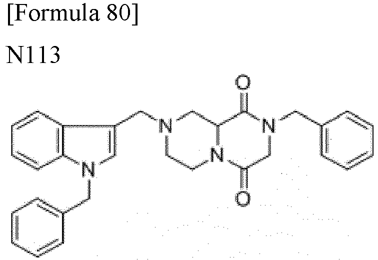

¹H NMR (500 MHz, CDCl₃) δ 7.74 - 7.72 (m, 1H), 7.35 - 7.22 (m, 9H), 7.19 - 7.16 (m, 1H), 7.14 - 7.10 (m, 3H), 7.04 (s, 1H), 5.28 (s, 2H), 4.56 (dd, J = 14.5 Hz, 27.5 Hz, 2H), 4.47 - 4.43 (m, 1H), 4.16 - 4.13 (m, 1H), 3.87 (d, J = 13.5 Hz, 1H), 3.84 - 3.83 (m, 2H), 3.73 (d, J = 13.5 Hz, 1H), 3.66 - 3.63 (m, 1H), 2.95 - 2.92 (m, 1H), 2.78 - 2.72 (m, 1H), 2.11 (t, J = 11.0 Hz, 1H), 2.05 - 1.99 (m, 1H).

¹³C NMR (126 MHz, CDCl₃) NMR (126 MHz, ) δ 163.70, 161.76, 137.52, 136.98, 135.07, 129.05, 129.02, 128.94, 128.67, 128.57, 128.51, 128.29, 128.10, 127.96, 127.81, 126.99, 126.88, 122.13, 119.85, 119.62, 110.77, 109.91, 57.54, 56.78, 53.44, 51.32, 50.09, 49.39, 48.65, 41.53.

MS (ESI) m/z for C₁₈H₂₁N₃O₂S [M+H]⁺: calcd 479.2442, found 479.2446.

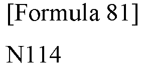

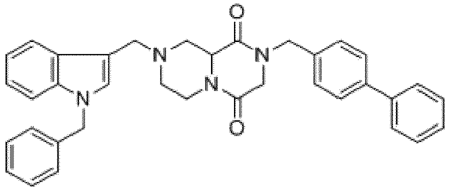

¹H NMR (500 MHz, CDCl₃) δ 7.75 (d, J = 8.0 Hz, 1H), 7.58 - 7.76 (m, 4H), 7.45 (t, J = 8.0 Hz, 2H), 7.38 - 7.25 (m, 7H), 7.21 - 7.18 (m, 1H), 7.16 - 7.12 (m, 3H), 7.06 (s, 1H), 5.30 (s, 2H), 4.61 (dd, J = 14.5 Hz, 23.5 Hz, 2H), 4.50 - 4.46 (m, 1H), 4.20 - 4.17 (m, 1H), 3.90 - 3.88 (m, 3H), 3.76 (d, J = 13.0 Hz, 1H), 3.69 - 3.66 (m, 1H), 2.97 - 2.94 (m, 1H), 2.81 - 2.75 (m, 1H), 2.15 (t, J = 11.0 Hz, 1H), 2.08 - 2.02 (m, 1H).

¹³C NMR (126 MHz, CDCl₃) δ 163.68, 161.77, 154.49, 141.31, 140.60, 137.48, 136.96, 134.02, 129.09, 128.94, 128.91, 128.51, 128.16, 127.79, 127.61, 127.20, 126.93, 122.14, 119.79, 119.61, 110.47, 109.91, 57.44, 56.64, 53.39, 51.20, 50.11, 49.14, 48.71, 41.43.

MS (ESI) m/z for C₁₈H₂₁N₃O₂S [M+H]⁺: calcd 555.2755, found 555.2752.

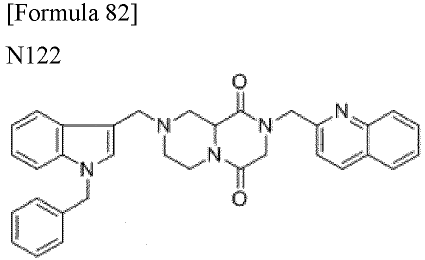

¹H NMR (500 MHz, CD₃OD) δ 8.12 (d, J = 8.5 Hz, 1H), 8.02 (d, J = 8.5 Hz, 1H), 7.80 - 7.78 (m, 1H), 7.75 - 7.73 (m, 1H), 7.72 - 7.69 (m, 1H), 7.55 - 7.51 (m, 1H), 7.37 (d, J = 8.5 Hz, 1H), 7.31 - 7.25 (m, 4H), 7.20 - 7.17 (m, 1H), 7.14 - 7.10 (m, 3H), 7.05 (s, 1H), 5.28 (s, 2H), 4.85 (dd, J = 14.5 Hz, 25.5 Hz, 2H), 4.51 - 4.48 (m, 1H), 4.22 - 4.15 (m, 3H), 3.88 (d, J = 13.5 Hz, 1H), 3.75 (d, J = 13.5 Hz, 1H), 3.66 - 3.63 (m, 1H), 2.97 - 2.94 (m, 1H), 2.82 - 2.76 (m, 1H), 2.17 (t, J = 11.0 Hz, 1H), 2.09 - 2.03 (m, 1H).

¹³C NMR (126 MHz, CDCl₃) δ 164.01, 161.98, 155.41, 147.76, 137.45, 137.41, 136.93, 129.94, 129.31, 128.89, 128.49, 128.21, 127.75, 127.68, 127.54, 126.92, 126.80, 122.13, 120.18, 119.75, 119.62, 110.32, 109.91, 57.33, 56.55, 53.36, 51.64, 51.19, 50.10, 49.92, 41.40.

MS (ESI) m/z for C₁₈H₂₁N₃O₂S [M+H]⁺: calcd 530.2551, found 530.2549.

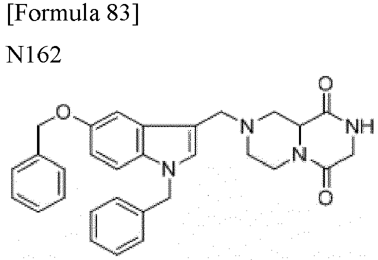

¹H NMR (500 MHz, CDCl₃) δ 7.49-7.46 (m,2H), 7.39-7.36 (m, 2H), 7.31-7.27 (m, 3H), 7.25-7.24 (m, 1H), 7.15 - 7.09 (m, 4H), 7.00 (s, 1H), 6.92 (dd, J = 2.5 Hz, 9.0 Hz, 1H),5.23 (s, 2H), 5.12 (s, 2H), 4.47-4.44 (m, 1H), 4.02-3.94 (m, 3H), 3.80 (d, J = 13.5 Hz, 1H), 3.65 (d, J = 13.0 Hz, 1H), 3.50-3.47 (m, 1H), 2.92-2.88 (m, 1H), 2.71-2.65 (m, 1H), 2.08 (t, J = 11.0 Hz, 1H), 2.01-1.95 (m, 1H).

¹³C NMR (126 MHz, CDCl₃) δ 166.11, 161.72, 153.11, 137.77, 137.44, 132.32, 128.77, 128.68, 128.50, 127.69, 127.64, 127.41, 126.76, 112.75, 110.54, 110.16, 103.36, 70.83, 56.90, 56.05, 53.46, 51.19, 50.15, 44.53, 41.56.

MS (ESI) m/z for C₃₀H₃₀N₄O₃ [M+H]⁺: calcd 495.2390, found 495.2386.

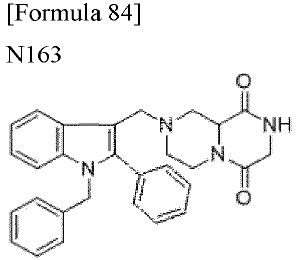

¹H NMR (500 MHz, CDCl₃)δ 7.84 - 7.82 (m, 1H), 7.40 - 7.39 (m, 3H), 7.33 - 7.32 (m, 2H), 7.25 - 7.15 (m, 5H), 6.93 (d, J = 7.5 Hz, 2H), 6.65 (s, 1H), 5.22 (s, 2H), 4.46 - 4.44 (m, 1H), 4.10 - 4.08 (m, 1H), 4.03 - 3.95 (m, 2H), 3.85 - 3.83 (m, 1H), 3.69 - 3.67 (m, 1H), 3.49 (d, J = 10.5 Hz, 1H), 2.87 (d, J = 11.0 Hz, 1H), 2.75 - 2.73 (m, 1H), 2.07 - 1.96 (m, 2H).

¹³C NMR (126 MHz, CDCl₃) δ 165.99, 161.61, 140.37, 138.16, 136.90, 131.44, 130.83, 128.70, 128.53, 128.43, 127.22, 126.13, 122.28, 119.99, 119.96, 110.41, 109.20, 57.20, 56.32, 52.74, 51.17, 47.68, 44.69, 41.80.

MS (ESI) m/z for C₁₈H₂₁N₃O₂S [M+H]⁺: calcd 465.2285, found 465.2281.

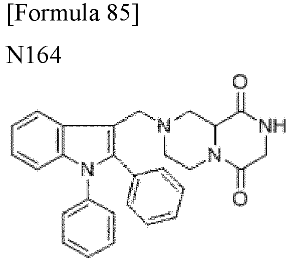

¹H NMR (500 MHz, DMSO) δ 8.13 (s, 1H), 7.88 - 7.86 (m, 1H), 7.41 (t, J = 11.0 Hz, 2H), 7.34 - 7.28 (m, 5H), 7.23 (t, J = 7.5 Hz, 2H), 7.19 - 7.15 (m, 3H), 4.27 (d, J = 12.5 Hz, 1H), 3.95 - 3.92 (m, 1H), 3.86 - 3.77 (m, 2H), 3.69 (s, 2H), 3.28 - 3.26 (m, 1H), 2.89 (d, J = 11.5 Hz, 1H),2.68 - 2.62 (m, 1H), 1.99 - 1.86 (m, 2H).

¹³C NMR (126 MHz, DMSO) δ 165.32, 162.97, 139.54, 138.21, 137.84, 131.56, 131.26, 131.13, 131.04, 129.82, 128.75, 128.51, 128.27, 127.74, 123.19, 123.01, 120.94, 120.76, 120.44, 111.18, 110.78, 110.59, 56.92, 55.99, 52.77, 52.00, 44.41, 41.38.

MS (ESI) m/z for C₁₈H₂₁N₃O₂S [M+H]⁺: calcd 451.2129, found 451.2129.

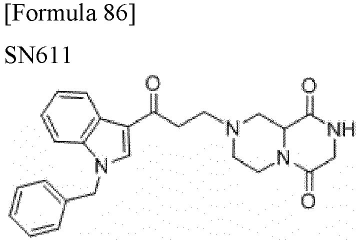

¹H NMR (500 MHz, CDCl₃) δ 8.39 (d, J = 7.5 Hz, 1H), 7.81 (s, 1H), 7.35 - 7.24 (m, 5H), 7.18 - 7.15 (m, 3H), 5.34 (s, 2H), 4.52 - 4.48 (m, 1H), 4.01 - 3.99 (m, 3H), 3.48 - 3.46 (m, 1H), 3.07 (t, J = 7.5 Hz, 2H), 3.02 - 2.96 (m, 1H), 2.94 - 2.92 (m, 1H), 2.89 - 2.83 (m, 1H), 2.79 - 2.73 (m, 1H), 2.17 - 2.08 (m, 2H).

¹³C NMR (126 MHz, CDCl₃) δ 193.68, 165.94, 161.82, 137.19, 135.81, 134.79, 129.16, 128.36, 127.14, 126.50, 123.72, 122.93, 122.72, 117.11, 110.30, 56.84, 55.95, 53.27, 52.27, 50.84, 44.64, 41.46, 37.31.

MS (ESI) m/z for C₁₈H₂₁N₃O₂S [M+H]⁺: calcd 431.2078, found 431.2072.

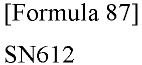

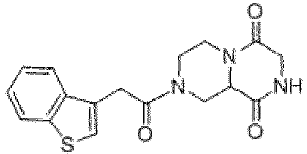

¹H NMR (500 MHz, CDCl₃) δ 7.89-7.77 (m, 2H), 7.41-7.20 (m, 3H), 4.66-4.44 (m, 2H), 4.09-3.85 (m, 5H), 3.68-3.56 (m, 1H), 3.18-3.02 (m, 1H), 2.74-2.63 (m, 1H), 2.62-2.38 (m, 1H).

¹³C NMR (126 MHz, CDCl₃) δ 169.05, 164.35, 162.19, 140.32, 138.48, 128.60, 124.76, 124.49, 122.91, 122.17, 121.71, 56.38, 54.61, 48.89, 44.59, 41.00, 34.82.

MS (ESI) m/z for C₁₇H₁₇N₃O₃S [M+H]⁺: calcd 344.1063, found 344.1061.

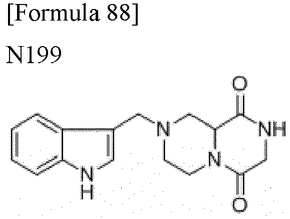

¹H NMR (500 MHz, CDCl₃) δ 7.68 - 7.66 (m, 1H), 7.39 - 7.37 (m, 1H), 7.19 - 7.13 (m, 2H), 7.12 - 7.07 (m, 1H), 4.45 (d, J = 13.5 Hz, 1H), 4.08 (d, J = 11.0 Hz, 1H), 3.96 - 3.77 (m, 4H), 3.49 (d, J = 11.0 Hz, 1H), 2.98 (d, J = 11.5 Hz, 1H), 2.79 (t, J = 12.5 Hz, 1H), 2.15 - 2.04 (m, 2H).

¹³C NMR (126 MHz, CDCl₃) δ 169.79, 166.51, 140.46, 131.63, 128.55, 125.70, 123.22, 122.92, 115.31, 113.92, 60.80, 59.65, 57.06, 55.17, 48.11, 45.41.

MS (ESI) m/z for C₁₆H₁₆N₄O₂ [M+H]⁺: calcd 299.1502, found 299.1502.

### Experimental Examples

### 1. Preparation of SKOV3 and SKOV3-TR

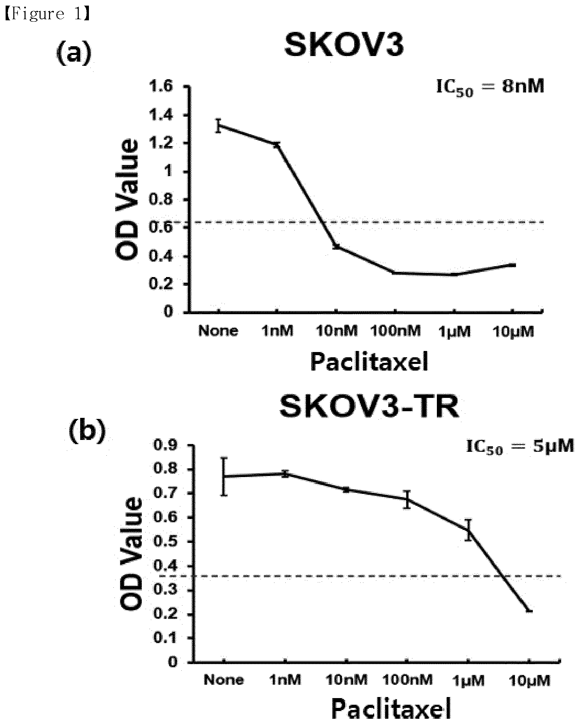

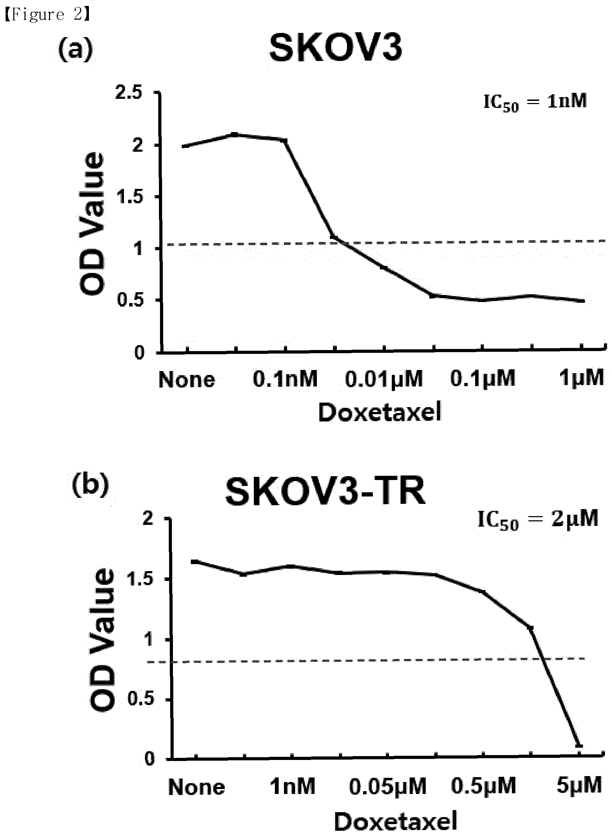

A cell experiment was performed on an epithelial ovarian cancer cell line SKOV3 and SKOV3-TR which is derived therefrom and produced as a resistant cancer cell line to having resistance to the anticancer drug paclitaxel. Here, FIG. 1A shows an IC50 value of the anticancer drug paclitaxel with respect to SKOV3, and FIG. 1B shows an IC50 value of the anticancer drug paclitaxel with respect to SKOV3-TR. FIG. 2A shows an IC50 value of the anticancer drug doxetaxel with respect to SKOV3, and FIG. 2B shows an IC50 value of the anticancer drug doxetaxel with respect to SKOV3-TR.

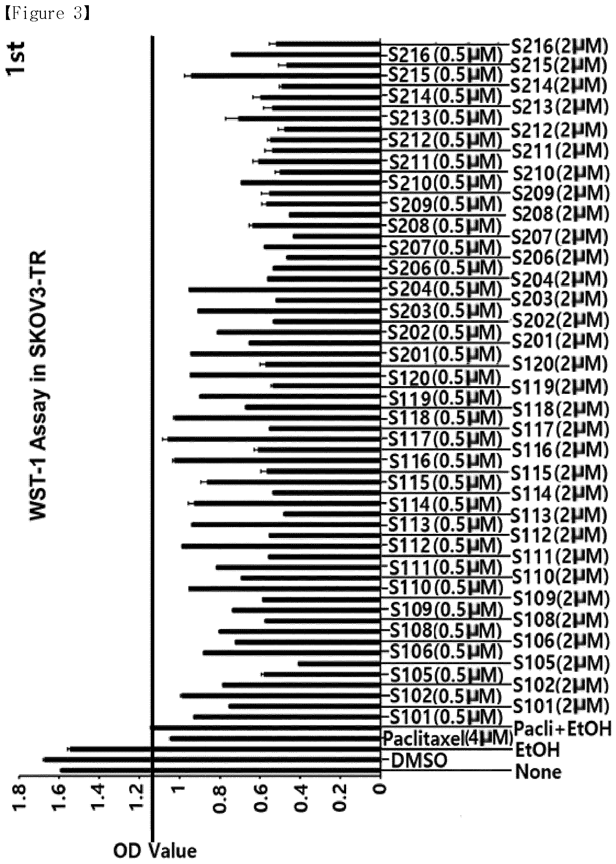

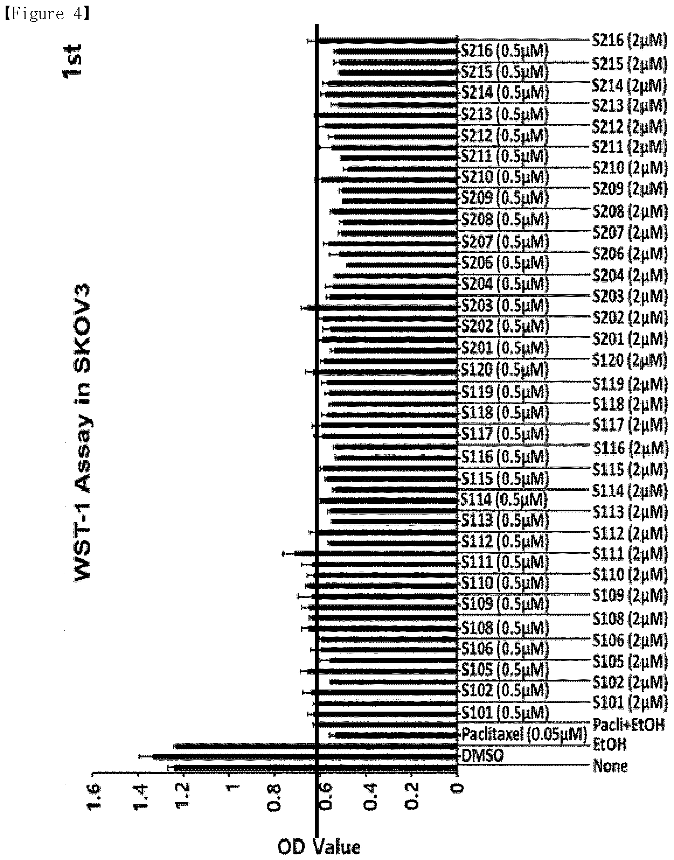

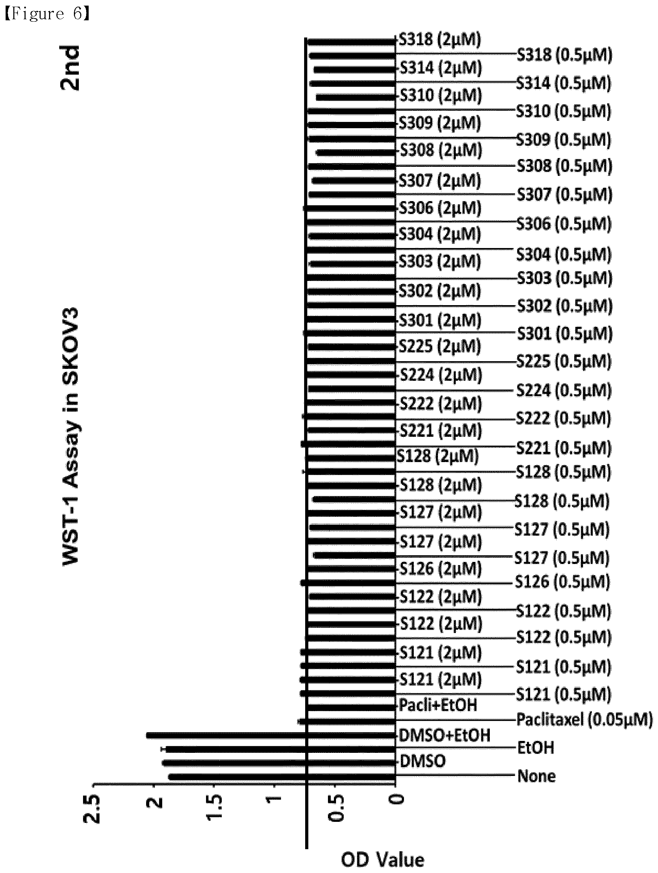

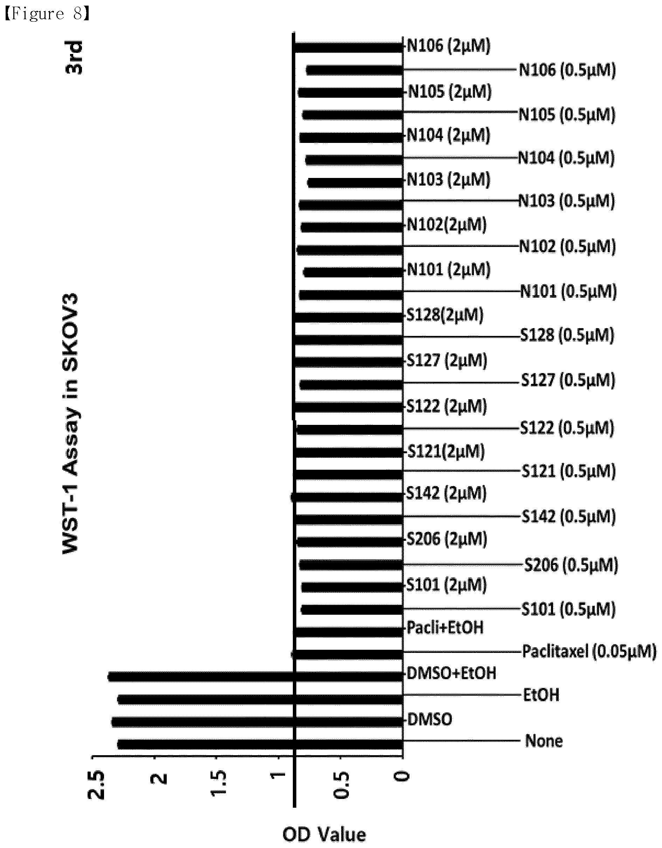

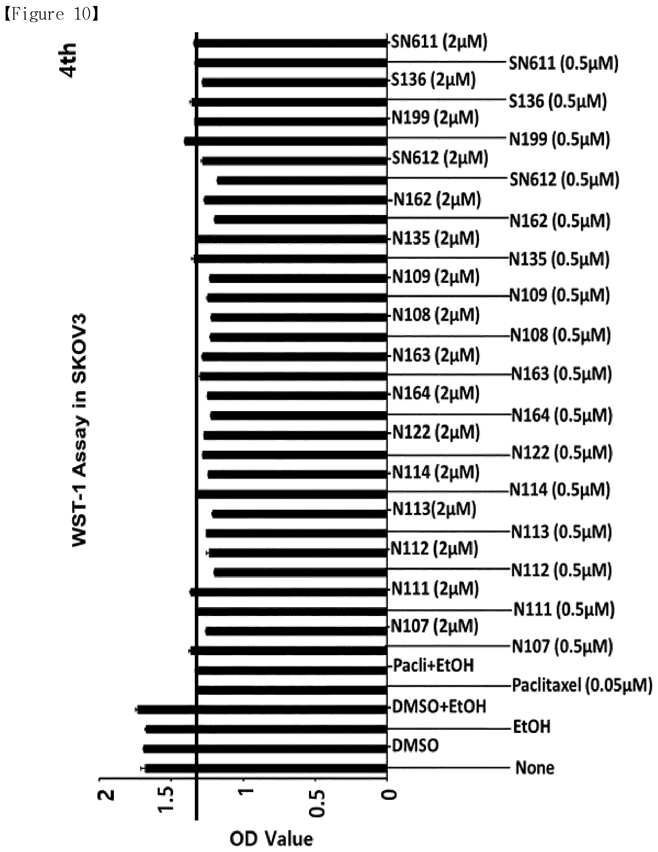

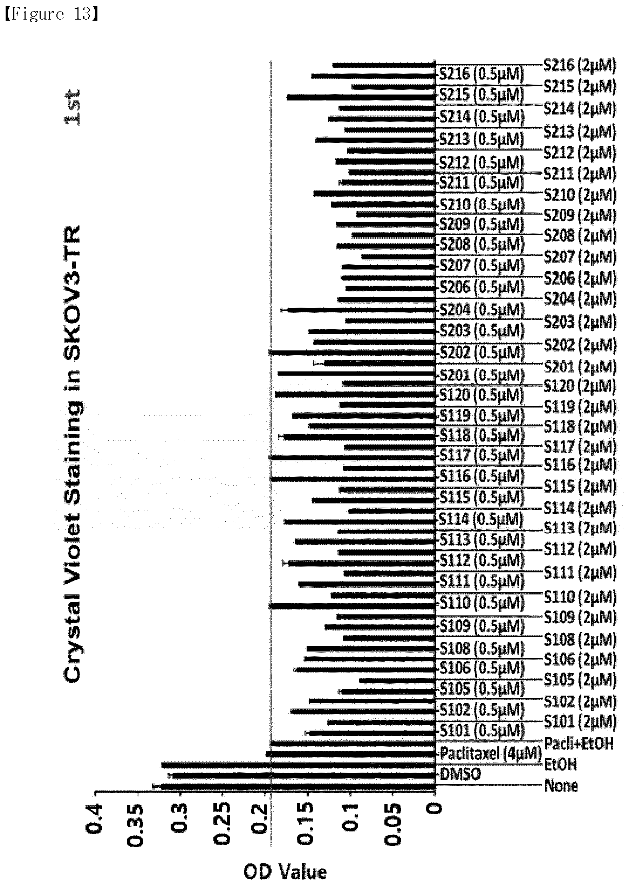

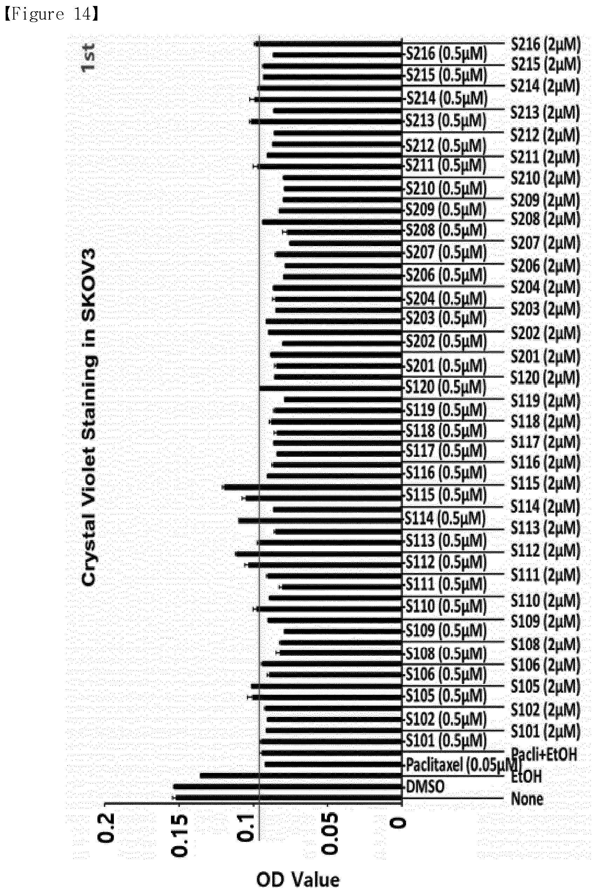

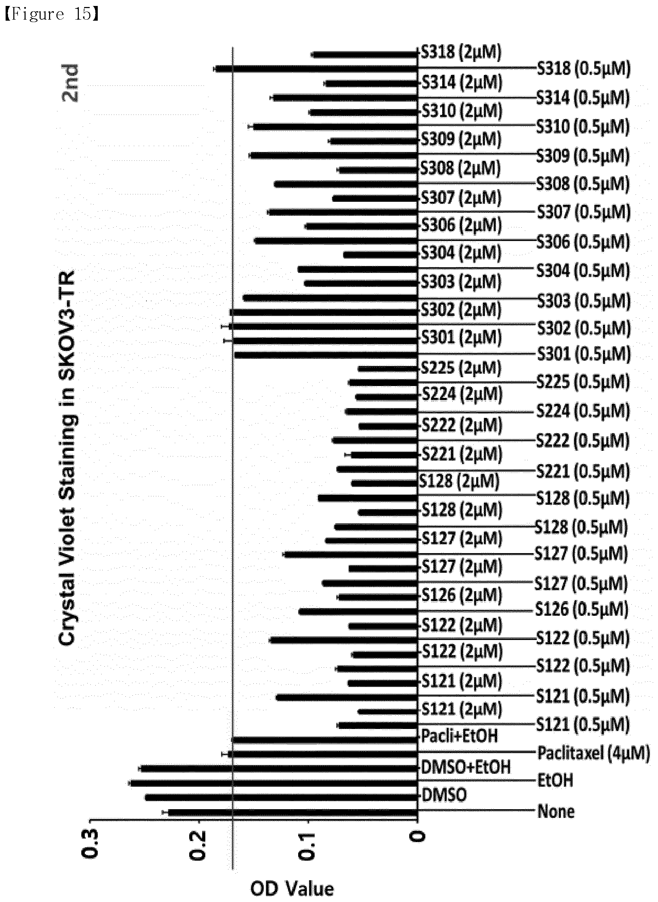

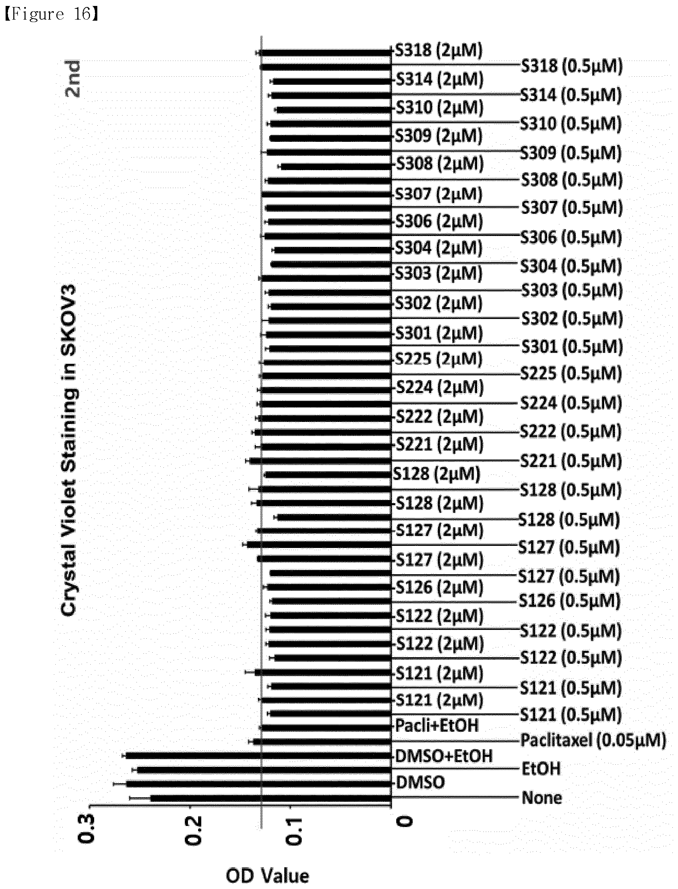

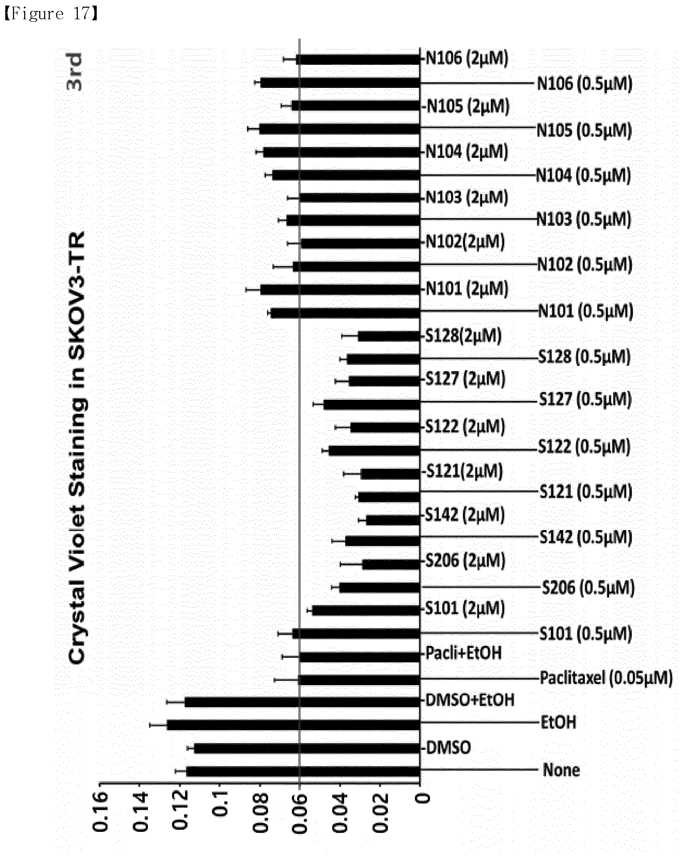

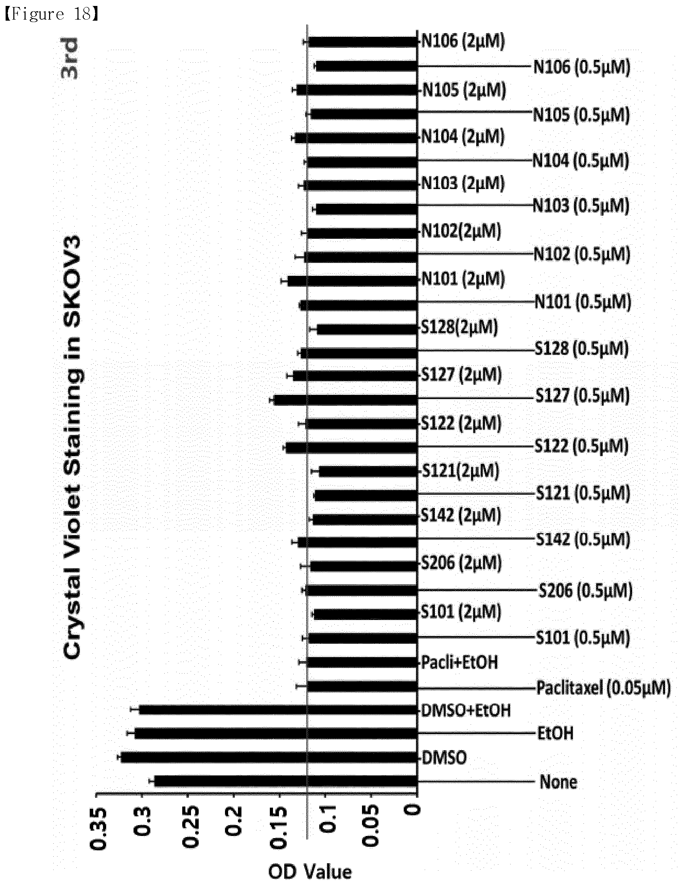

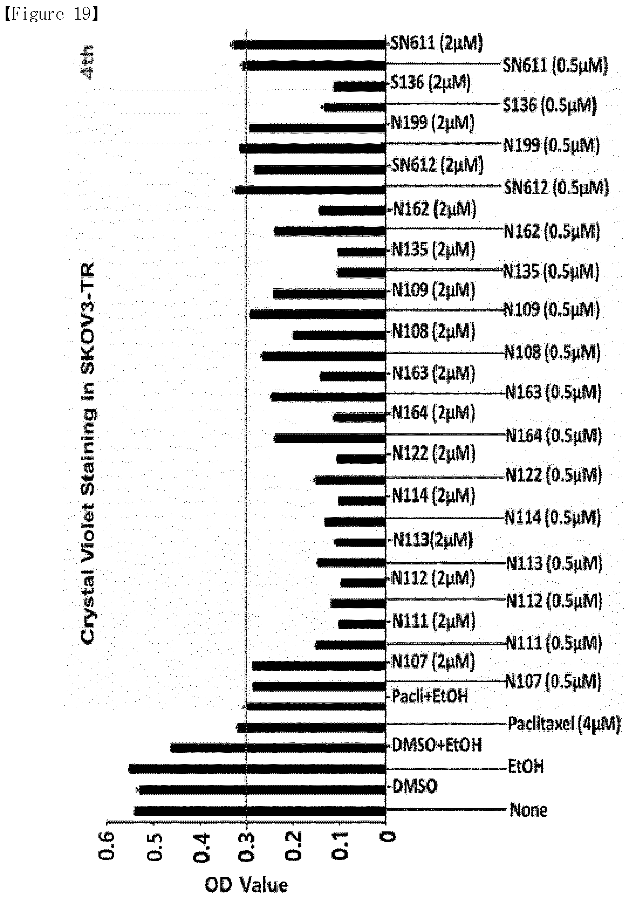

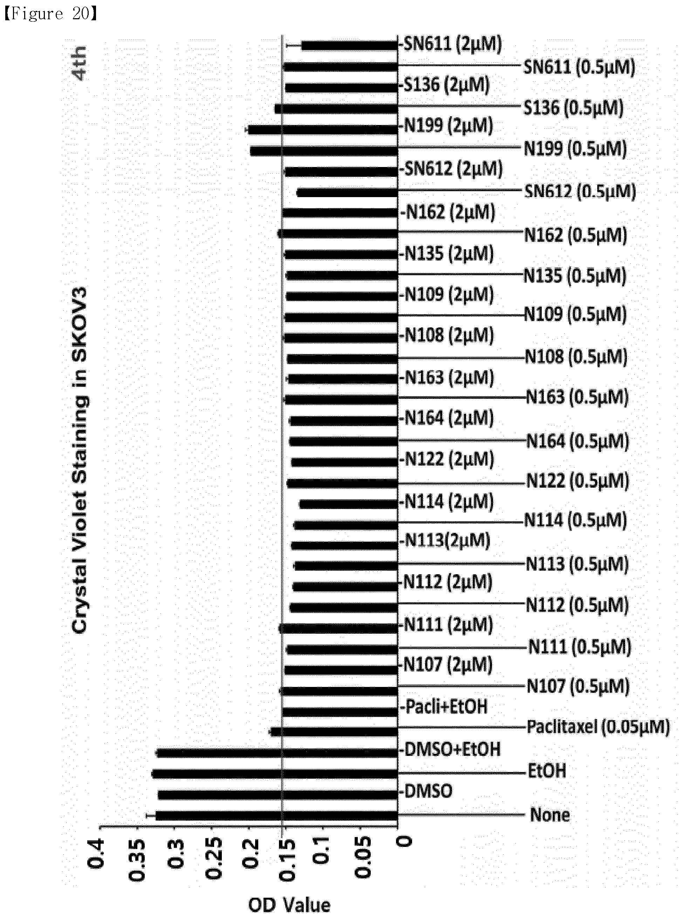

The two cancer cell lines growing on a 12-well plate were pretreated with 102 S-forms at a low concentration (0.5 µM) and a high concentration (2 µM) for 4 hours, and the SKOV3-TR cell line was then treated with 4 µM paclitaxel, and the SKOV3 cell line was treated with 0.05 µM paclitaxel. For the morphological analysis of the cells, the cells treated with None (a group that was treated with nothing), DMSO (a paclitaxel solvent), ethanol (EtOH) (an S-form solvent), DMSO+EtOH, paclitaxel, paclitaxel+ethanol (Pacli+EtOH), and 102 S-forms were analyzed after 1, 2, and 3 days, and images were captured. For cellular metabolism analysis, WST-1 analyses were performed after 3 days of the combined treatment with paclitaxel and S-forms. The results are shown in FIGS. 3 to 10. The cell survival rates were analyzed on the same experimental groups using a crystal violet staining method. The results are shown in FIGS. 13 to 20.

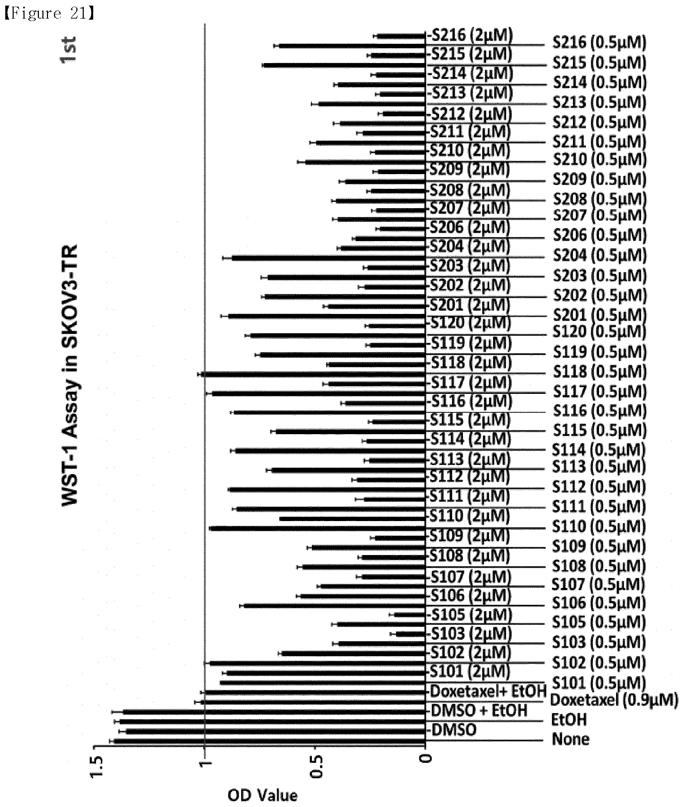

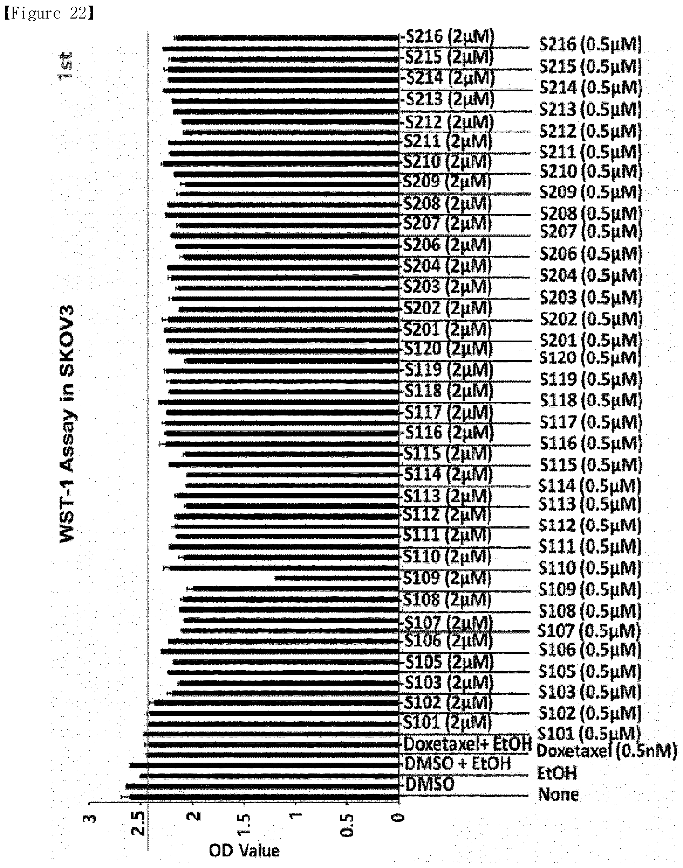

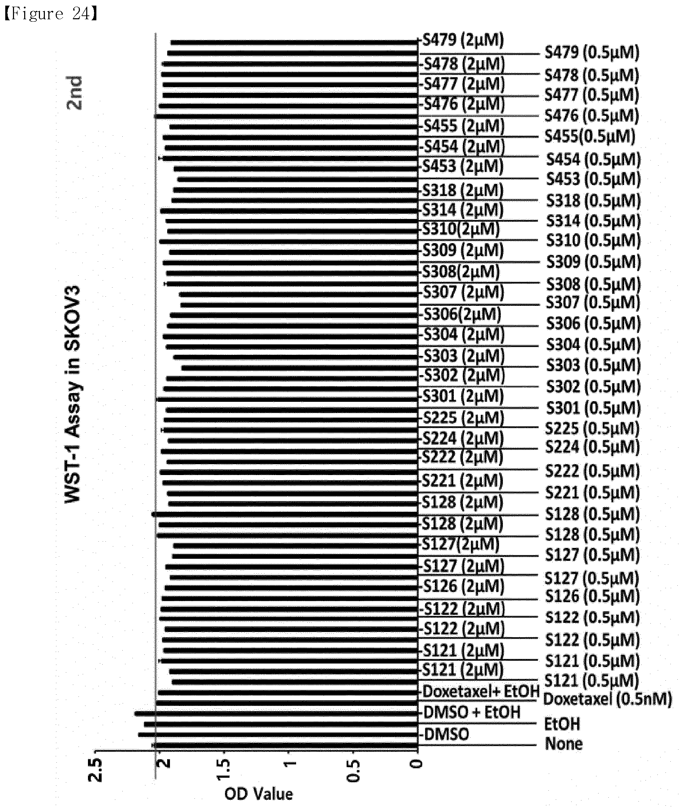

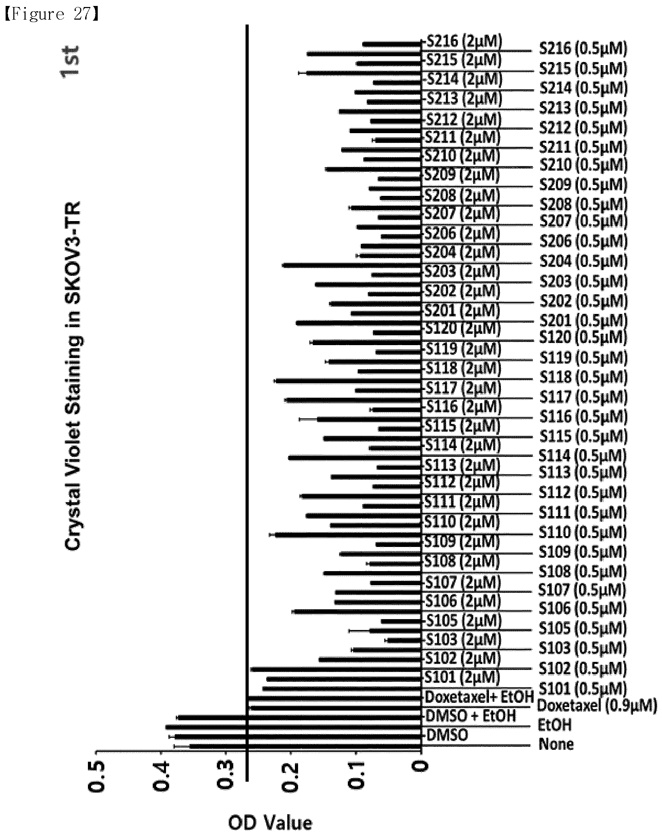

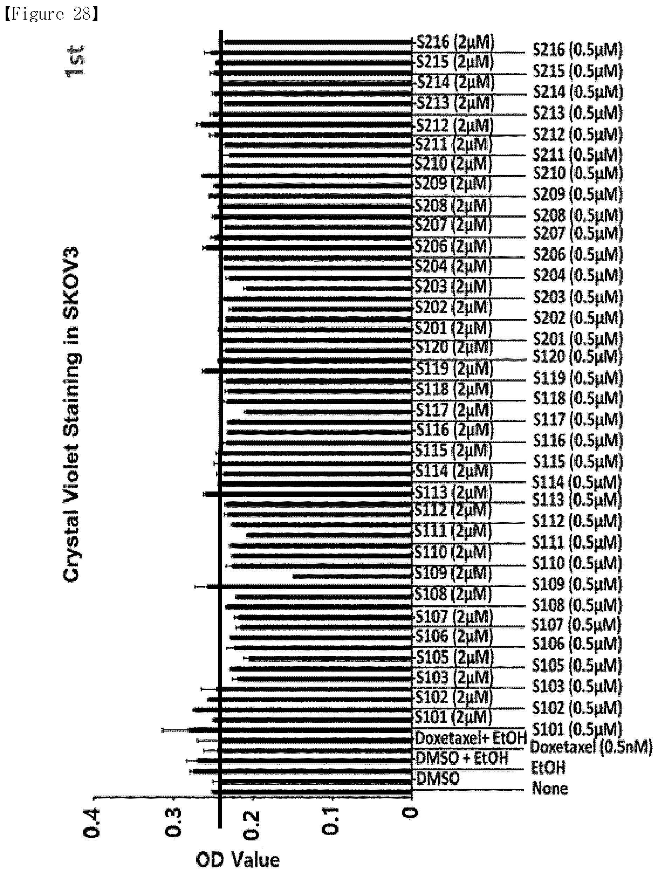

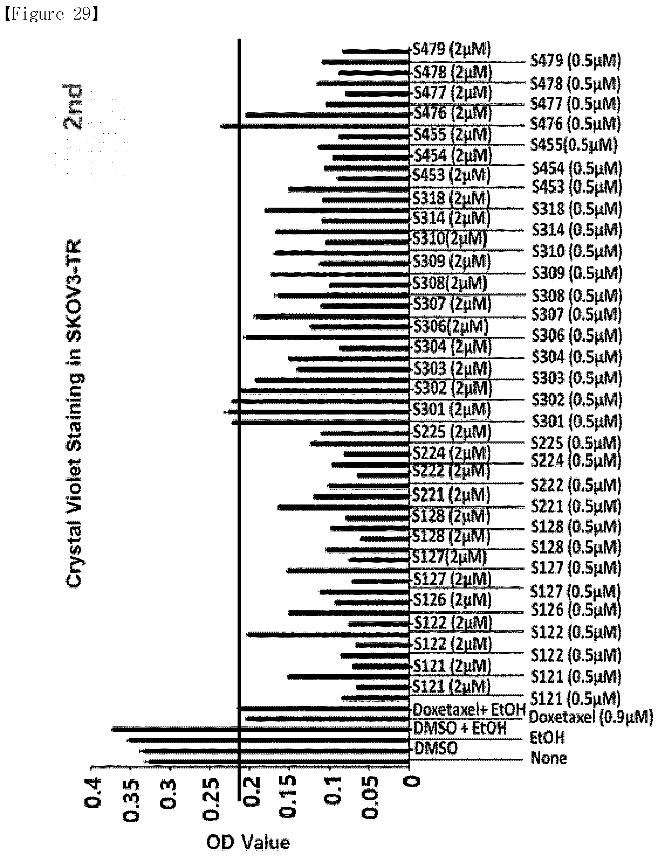

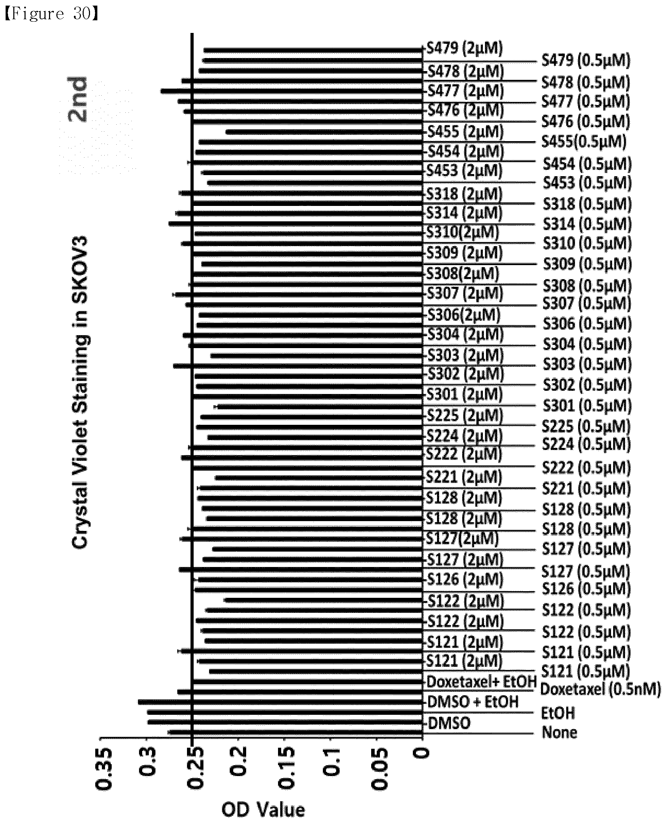

The two cell lines growing on a 12-well plate were pretreated with an S-form at a low concentration (0.5 µM) and a high concentration (2 µM) for 4 hours, and the SKOV3-TR cell line was then treated with 0.9 µM doxetaxel, and the SKOV3 cell line was treated with 0.5 µM doxetaxel. For the morphological analysis of the cells, the cells treated with None (a group that was treated with nothing), DMSO (a doxetaxel solvent), ethanol (EtOH) (an S-form solvent), DMSO+EtOH, doxetaxel, doxetaxel+ethanol (Doxetaxel+EtOH), and S-forms were analyzed after 1, 2, and 3 days, and images were captured. For cellular metabolism analysis, WST-1 analyses were performed after 3 days of the combined treatment with doxetaxel and S-forms. The results are shown in FIGS. 21 to 24. The cell survival rates were analyzed on the same experimental groups using a crystal violet staining method. The results are shown in FIGS. 27 to 30.

FIGS. 3, 4, 13, and 14 show None, DMSO, ethanol (EtOH), DMSO+ethanol, paclitaxel, paclitaxel+ethanol, S101 (0.5 µM), S101 (2 µM), S102 (0.5 µM), S102 (2 µM), S105 (0.5 µM), S105 (2 µM), S106 (0.5 µM), S106 (2 µM), S108 (0.5 µM), S108 (2 µM), S109 (0.5 µM), S109 (2 µM), S110 (0.5 µM), S110 (2 µM), S111 (0.5 µM), S111 (2 µM), S112 (0.5 µM), S112 (2 µM), S113 (0.5 µM), S113 (2 µM), S114 (0.5 µM), S114 (2 µM), S115 (0.5 µM), S115 (2 µM), S116 (0.5 µM), S116 (2 µM), S117 (0.5 µM), S117 (2 µM), S118 (0.5 µM), S118 (2 µM), S119 (0.5 µM), S119 (2 µM), S120 (0.5 µM), S120 (2 µM), S201 (0.5 µM), S201 (2 µM), S202 (0.5 µM), S202 (2 µM), S203 (0.5 µM), S203 (2 µM), S204 (0.5 µM), S204 (2 µM), S206 (0.5 µM), S206 (2 µM), S207 (0.5 µM), S207 (2 µM), S208 (0.5 µM), S208 (2 µM), S209 (0.5 µM), S209 (2 µM), S210 (0.5 µM), S210 (2 µM), S211 (0.5 µM), S211 (2 µM), S212 (0.5 µM), S212 (2 µM), S213 (0.5 µM), S213 (2 µM), S214 (0.5 µM), S214 (2 µM), S215 (0.5 µM), S215 (2 µM), S216 (0.5 µM), and S216 (2 µM) in this order.

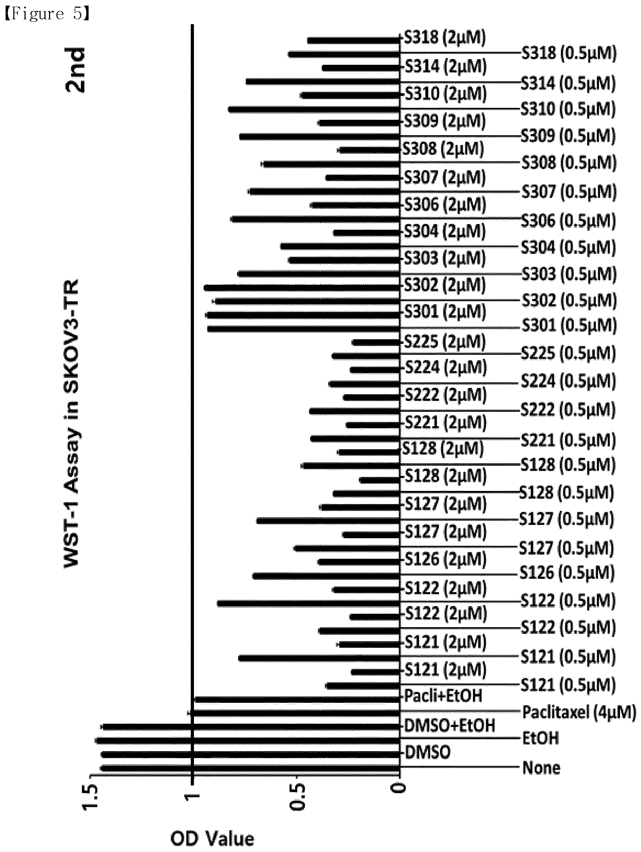

FIGS. 5, 6, 15, and 16 show None, DMSO, ethanol (EtOH), DMSO+ethanol, paclitaxel, paclitaxel+ethanol, S121 (0.5 µM), S121 (2 µM), S122 (0.5 µM), S122 (2 µM), S126 (0.5 µM), S126 (2 µM), S127 (0.5 µM), S127 (2 µM), S128 (0.5 µM), S128 (2 µM), S221 (0.5 µM), S221 (2 µM), S222 (0.5 µM), S222 (2 µM), S224 (0.5 µM), S224 (2 µM), S225 (0.5 µM), S225 (2 µM), S301 (0.5 µM), S301 (2 µM), S302 (0.5 µM), S302 (2 µM), S303 (0.5 µM), S303 (2 µM), S304 (0.5 µM), S304 (2 µM), S306 (0.5 µM), S306 (2 µM), S307 (0.5 µM), S307 (2 µM), S309 (0.5 µM), S309 (2 µM), S310 (0.5 µM), S310 (2 µM), S314 (0.5 µM), S314 (2 µM), S318 (0.5 µM), and S318 (2 µM) in this order.

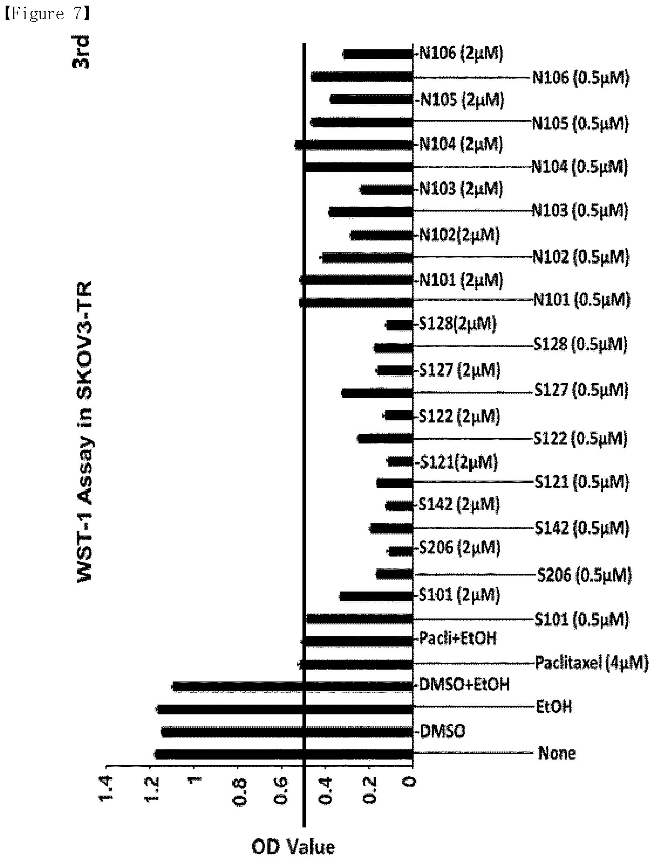

FIGS. 7, 8, 17, and 18 show None, DMSO, ethanol (EtOH), DMSO+ethanol, paclitaxel, paclitaxel+ethanol, S101 (0.5 µM), S101 (2 µM), S206 (0.5 µM), S206 (2 µM), S142 (0.5 µM), S142 (2 µM), S121 (0.5 µM), S121 (2 µM), S122 (0.5 µM), S122 (2 µM), S127 (0.5 µM), S127 (25 µM), S128 (0.5 µM), S128 (2 µM), N101 (0.5 µM), N101 (2 µM), N102 (0.5 µM), N102 (2 µM), N103 (0.5 µM), N103 (2 µM), N104 (0.5 µM), N104 (2 µM), N105 (0.5 µM), N105 (2 µM), N106 (0.5 µM), N106 (2 µM) in this order.

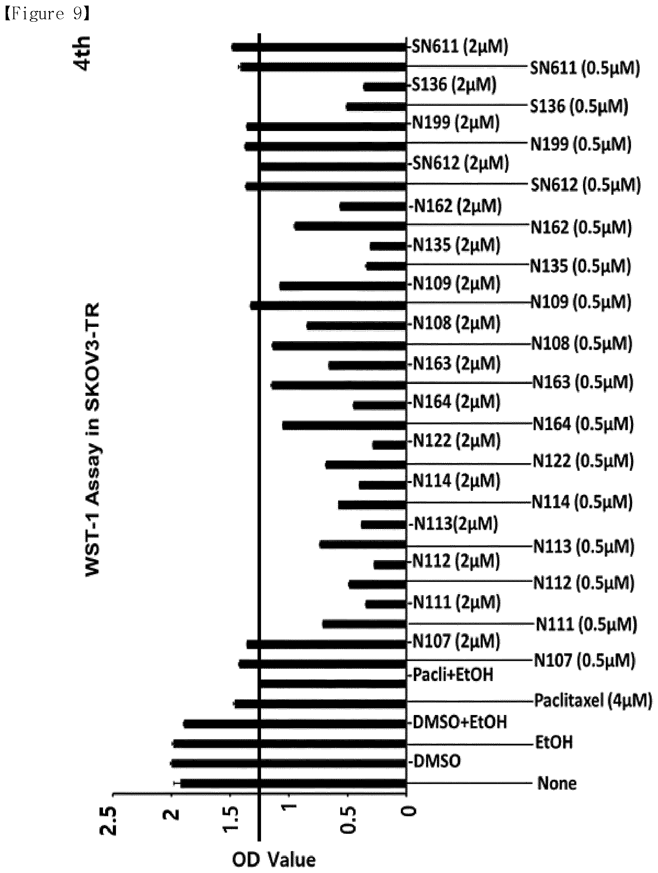

FIGS. 9, 10, 19, and 20 show None, DMSO, ethanol (EtOH), DMSO+ethanol, paclitaxel, paclitaxel+ethanol, N107 (0.5 µM), N107 (2 µM), N111 (0.5 µM), N111 (2 µM), N112 (0.5 µM), N112 (2 µM), N113 (0.5 µM), N113 (2 µM), N114 (0.5 µM), N114 (2 µM), N122 (0.5 µM), N122 (2 µM), N164 (0.5 µM), N164 (2 µM), N163 (0.5 µM), N163 (2 µM), N108 (0.5 µM), N108 (2 µM), N109 (0.5 µM), N109 (2 µM), S135 (0.5 µM), S135 (2 µM), N162 (0.5 µM), N162 (2 µM), SN612 (0.5 µM), SN612 (2 µM), N199 (0.5 µM), N199 (2 µM), S136 (0.5 µM), S136 (2 µM), SN611 (0.5 µM), SN611 (2 µM) in this order.

FIGS. 21, 22, 27, and 28 show None, DMSO, ethanol (EtOH), DMSO+ethanol, doxetaxel, doxetaxel+ethanol, S101 (0.5 µM), S101 (2 µM), S102 (0.5 µM), S102 (2 µM), S103 (0.5 µM), S103 (2 µM), S105 (0.5 µM), S105 (2 µM), S106 (0.5 µM), S106 (2 µM), S107 (2 µM), S107 (0.5 µM), S108 (0.5 µM), S108 (2 µM), S109 (0.5 µM), S109 (2 µM), S110 (0.5 µM), S110 (2 µM), S111 (0.5 µM), S111 (2 µM), S112 (0.5 µM), S112 (2 µM), S113 (0.5 µM), S113 (2 µM), S114 (0.5 µM), S114 (2 µM), S115 (0.5 µM), S115 (2 µM), S116 (0.5 µM), S116 (2 µM), S117 (0.5 µM), S117 (2 µM), S118 (0.5 µM), S118 (2 µM), S119 (0.5 µM), S119 (2 µM), S120 (0.5 µM), S120 (2 µM), S201 (0.5 µM), S201 (2 µM), S202 (0.5 µM), S202 (2 µM), S203 (0.5 µM), S203 (2 µM), S204 (0.5 µM), S204 (2 µM), S206 (0.5 µM), S206 (2 µM), S207 (0.5 µM), S207 (2 µM), S208 (0.5 µM), S208 (2 µM), S209 (0.5 µM), S209 (2 µM), S210 (0.5 µM), S210 (2 µM), S211 (0.5 µM), S211 (2 µM), S212 (0.5 µM), S212 (2 µM), S213 (0.5 µM), S213 (2 µM), S214 (0.5 µM), S214 (2 µM), S215 (0.5 µM), S215 (2 µM), S216 (0.5 µM), S216 (2 µM) in this order.

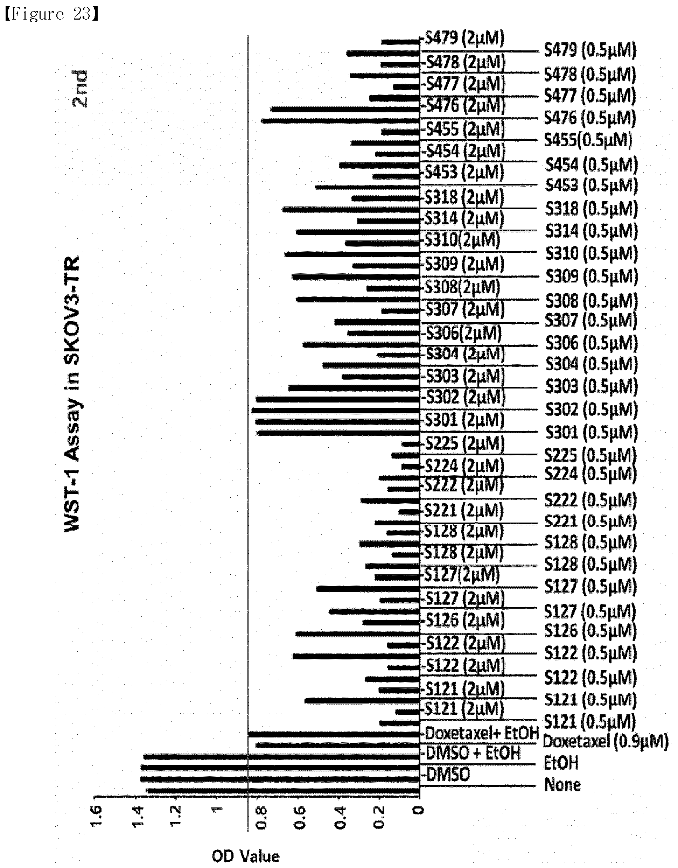

FIGS. 23, 24, 29, and 30 show None, DMSO, ethanol (EtOH), DMSO+ethanol, doxetaxel, doxetaxel+ethanol, S121 (0.5 µM), S121 (2 µM), S122 (0.5 µM), S122 (2 µM), S126 (0.5 µM), S126 (2 µM), S127 (0.5 µM), S127 (2 µM), S128 (0.5 µM), S128 (2 µM), S221 (0.5 µM), S221 (2 µM), S222 (0.5 µM), S222 (2 µM), S224 (0.5 µM), S224 (2 µM), S225 (0.5 µM), S225 (2 µM), S301 (0.5 µM), S301 (2 µM), S302 (0.5 µM), S302 (2 µM), S303 (0.5 µM), S303 (2 µM), S304 (0.5 µM), S304 (2 µM), S306 (0.5 µM), S306 (2 µM), S307 (0.5 µM), S307 (2 µM), S308 (0.5 µM), S308 (2 µM), S309 (0.5 µM), S309 (2 µM), S310 (0.5 µM), S310 (2 µM), S314 (0.5 µM), S314 (2 µM), S318 (0.5 µM), S318 (2 µM) in this order.

### 2. WST-1 analysis

Based on the results of WST-1 analysis in which cellular metabolism was analyzed, a decrease in metabolism in SKOV3-TR and SKOV3 by treatment with paclitaxel was induced. The WST-1 analysis results of 102 S-forms with respect to SKOV3-TR and the WST-1 analysis results of 102 S-forms with respect to SKOV3 in the presence of paclitaxel are shown in FIGS. 3 to 10.

That is, the optical densities at 450 nm were reduced in the paclitaxel-treated groups due to the phenomena such as the induction of cell death and the inhibition of cell growth in SKOV3-TR and SKOV3, compared to the None or DMSO-treated groups. When the cell lines were treated with the S-forms in combination with paclitaxel, the S-forms enhanced the anticancer effect of paclitaxel on SKOV3-TR, which is a resistant cancer cell line, to exhibit lower optical densities as compared to the paclitaxel+ethanol (Pacli+EtOH)-treated group, but did not enhance the anticancer effect of paclitaxel on SKOV3. Also, it can be seen that the dose-response relationship was satisfied because a decrease in metabolism by paclitaxel in SKOV3-TR was further promoted in the presence of the 2 µM S-forms as compared to the 0.5 µM S-forms. As a comparative example, the baseline was applied to the optical density of the paclitaxel+ethanol (Pacli+EtOH)-treated group. For the anticancer-enhancing effect of paclitaxel, the optical densities (OD values at 450 nm) were significantly reduced in the WST-1 analysis when the cell lines were treated with paclitaxel+S-forms, compared to the paclitaxel+ethanol-treated group (Pacli+EtOH).

Based on the results of WST-1 analysis in which the cellular metabolism was analyzed, a decrease in metabolism in SKOV3-TR and SKOV3 by treatment with doxetaxel was induced. The WST-1 analysis results of the S-forms with respect to SKOV3-TR and the WST-1 analysis results of the S-forms with respect to SKOV3 in the presence of paclitaxel are shown in FIGS. 21 to 24.

That is, the optical densities at 450 nm were reduced in the doxetaxel-treated groups due to the phenomena such as the induction of cell death and the inhibition of cell growth in SKOV3-TR and SKOV3, compared to the None or DMSO-treated groups. When the cell lines were treated with the S-forms in combination with paclitaxel, the S-forms enhanced the anticancer effect of doxetaxel on SKOV3-TR, which is a resistant cancer cell line, to exhibit lower optical densities as compared to the doxetaxel+ethanol (Doxetaxel+EtOH)-treated group, but did not enhance the anticancer effect of paclitaxel on SKOV3. Also, it can be seen that the dose-response relationship was satisfied because a decrease in metabolism by doxetaxel in SKOV3-TR was further promoted in the presence of the 2 µM S-forms as compared to the 0.5 µM S-forms. As a comparative example, the baseline was applied to the optical density of the doxetaxel+ethanol (Doxetaxel +EtOH)-treated group. For the anticancer-enhancing effect of doxetaxel, the optical densities (OD values at 450 nm) were significantly reduced in the WST-1 analysis when the cell lines were treated with the doxetaxel+S-forms, compared to the doxetaxel+ethanol-treated group (Doxetaxel +EtOH).

### 3. Cell survival rate and cell line analysis

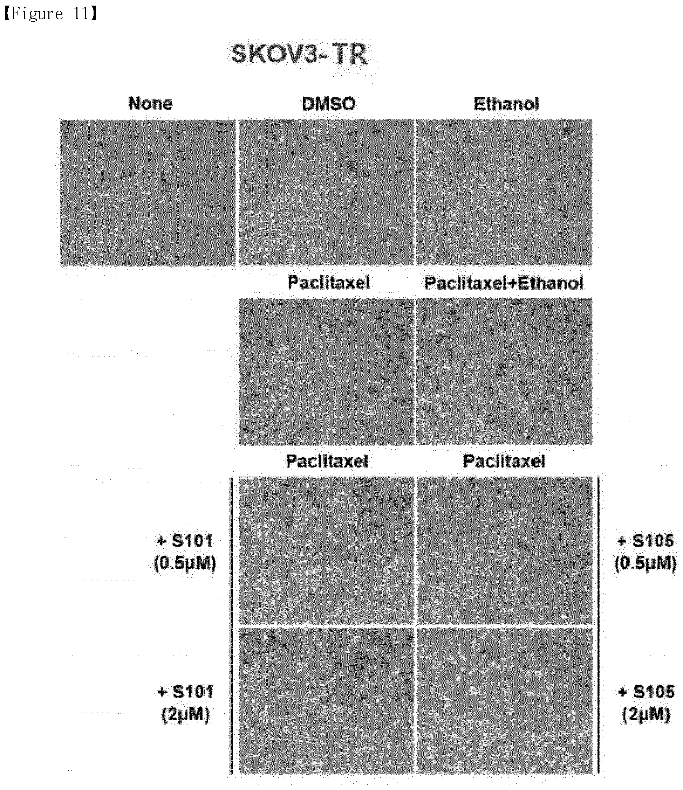

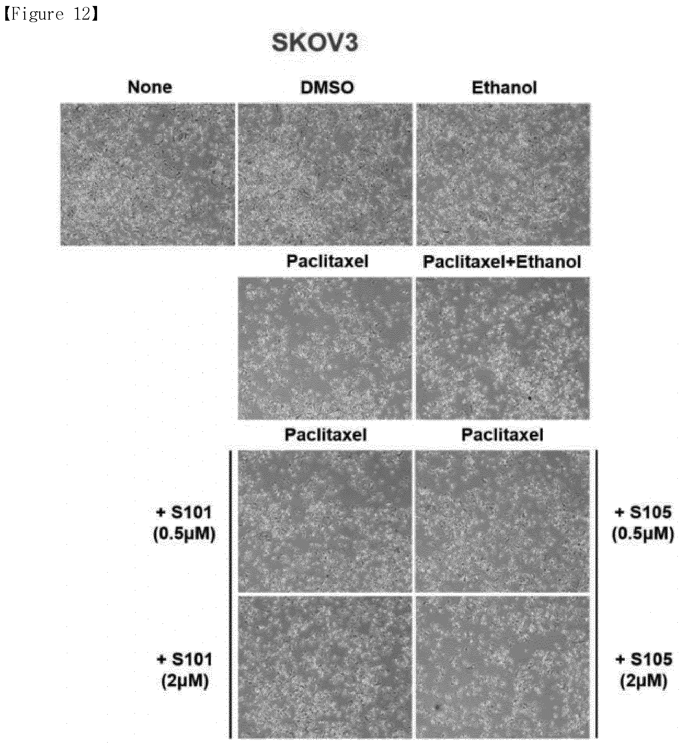

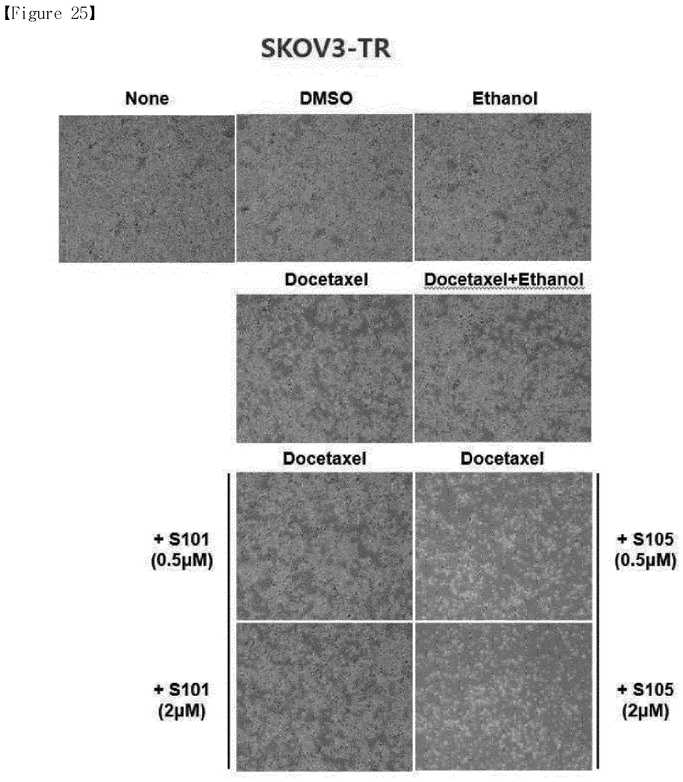

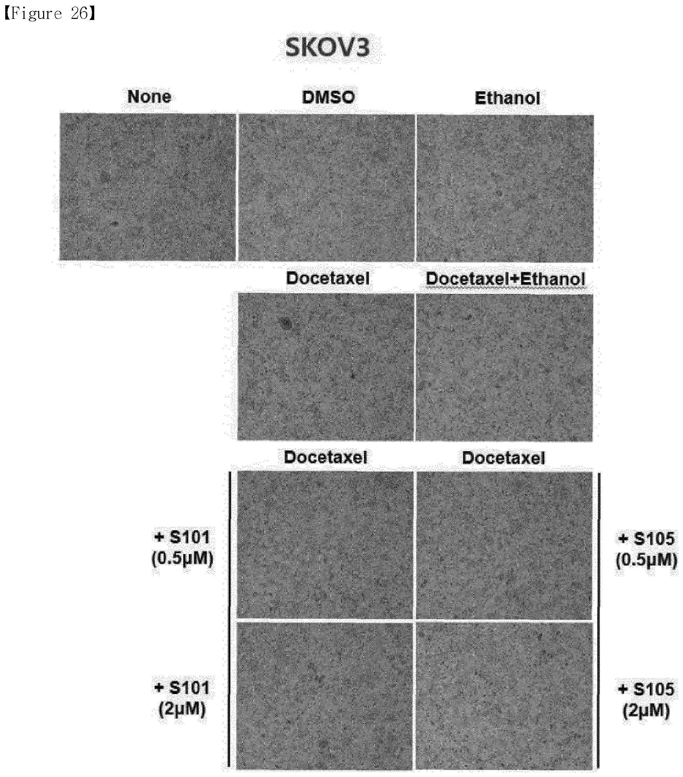

Based on the crystal violet staining in which the cell survival rates and the cell counts were analyzed, a decrease in cell counts in SKOV3-TR and SKOV3 by treatment with paclitaxel was induced through a decrease in cell survival rate. Representatively, images of the SKOV3-TR and SKOV3 cell lines were captured after the cell lines were treated with paclitaxel and doxetaxel alone for 3 days or treated with S101 and S105, which exhibited the anticancer-enhancing effect of paclitaxel and doxetaxel, in combination with paclitaxel or doxetaxel for 3 days. The crystal violet staining results of SKOV3-TR by treatment with paclitaxel are shown in FIG. 11, and the crystal violet staining results of SKOV3 are shown in FIG. 12. The crystal violet staining results of SKOV3-TR by treatment with doxetaxel are shown in FIG. 25, and the crystal violet staining results of SKOV3 are shown in FIG. 26.

The optical densities at 570 nm were reduced with the decreasing cell counts in the paclitaxel-treated groups due to the phenomena such as the induction of cell death and the inhibition of cell growth in SKOV3-TR and SKOV3, compared to the None or DMSO-treated groups. When the resistant cancer cell lines were treated with the S-forms in combination with paclitaxel, the S-forms enhanced the anticancer effect of paclitaxel on SKOV3-TR, which is a resistant cancer cell line, to exhibit lower optical densities as compared to the paclitaxel+ethanol (Pacli+EtOH)-treated group, but did not enhance the anticancer effect of paclitaxel on SKOV3. Also, it can be seen that the dose-response relationship was satisfied because a decrease in cell survival rate by paclitaxel in SKOV3-TR was further promoted at a high concentration (2 µM) of the S-forms as compared to a low concentration (0.5 µM) of the S-forms. For the purpose of analysis, images of the 102 experimental groups after the cell lines were stained with crystal violet were captured after 3 days of the treatment. The staining degree was determined by ELISA by measuring the optical densities at 570 nm through dye elution as in the WST-1 analysis. During the ELISA analysis, the baseline was applied to the optical density of the paclitaxel+ethanol (Pacli+EtOH)-treated group as the control group. The optical density results of SKOV3-TR by treatment with the 102 S-forms and the optical density results of SKOV3 by treatment with the 102 S-forms are shown in FIGS. 13 to 20.

The optical densities at 570 nm were reduced with the decreasing cell counts in the doxetaxel-treated groups due to the phenomena such as the induction of cell death and the inhibition of cell growth in SKOV3-TR and SKOV3, compared to the None or DMSO-treated groups. When the resistant cancer cell lines were treated with the S-forms in combination with doxetaxel, the S-forms enhanced the anticancer effect of doxetaxel on SKOV3-TR, which is a resistant cancer cell line, to exhibit lower optical densities as compared to the doxetaxel+ethanol (Doxetaxel+EtOH)-treated group, but did not enhance the anticancer effect of doxetaxel on SKOV3. Also, it can be seen that the dose-response relationship was satisfied because a decrease in cell survival rate by doxetaxel in SKOV3-TR was further promoted at a high concentration (2 µM) of the S-forms as compared to a low concentration (0.5 µM) of the S-forms. For the purpose of analysis, images of the 102 experimental groups after the cell lines were stained with crystal violet were captured after 3 days of the treatment. The staining degree was determined by ELISA by measuring the optical densities at 570 nm through dye elution as in the WST-1 analysis. During the ELISA analysis, the baseline was applied to the optical density of the doxetaxel+ethanol (Doxetaxel+EtOH)-treated group as the control group. The optical density results of SKOV3-TR by treatment with the S-forms and the optical density results of SKOV3 by treatment with the S-forms are shown in FIGS. 27 to 30.

### 4. Measurement of anticancer efficacy

### 1) Analysis of changes in cell counts (in vitro)

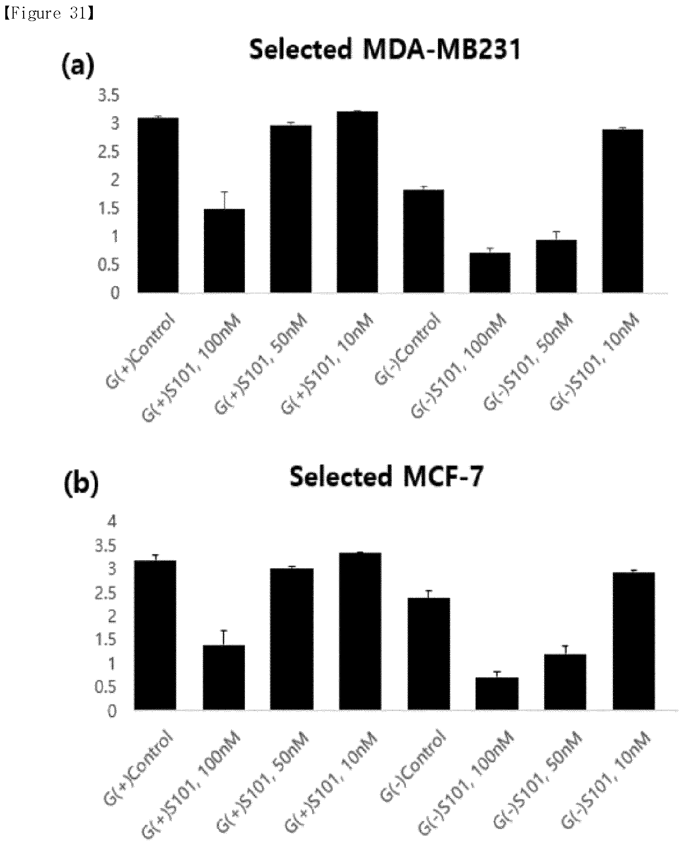

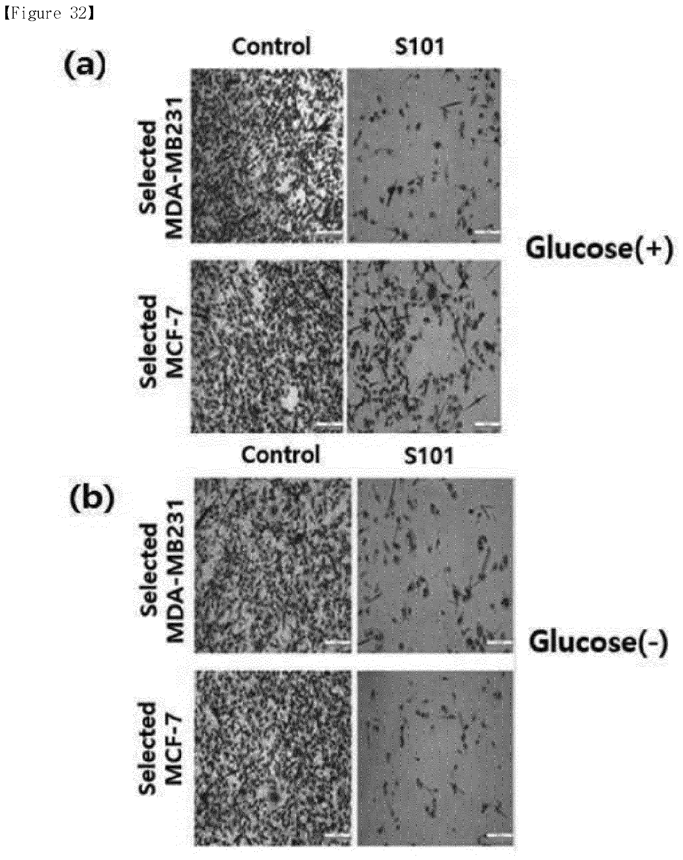

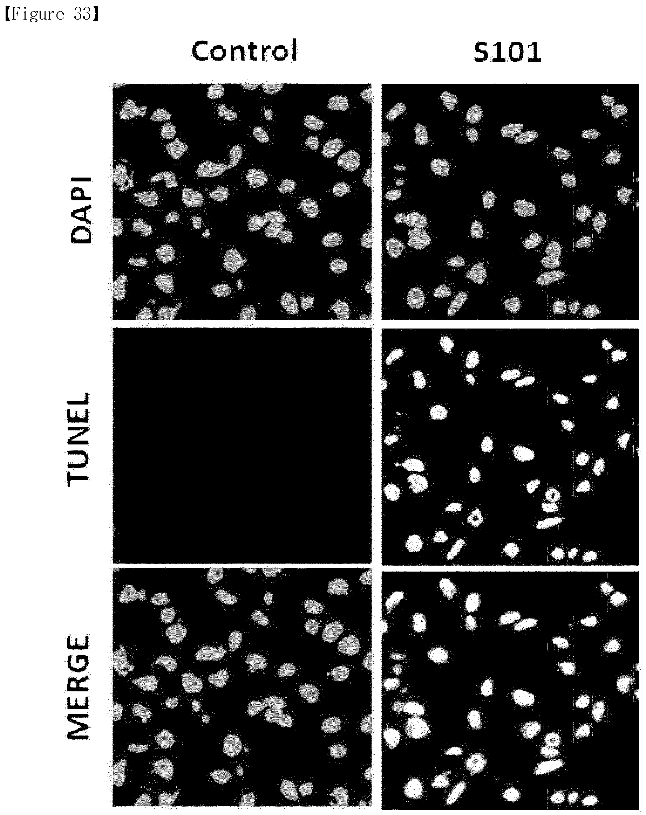

The changes in cell counts after the selected-MDA-MB231 and selected-MCF7 cells, which are stem cell-like cancer cells, are treated with 100 nM, 50 nM, and 10 nM of the S101 compound when no ER stress is applied to the cancer cells in the presence of glucose (G(+)) and ER stress is applied to the cancer cells in the absence of glucose (G(-)) are shown in FIG. 31, and the results of capturing images of the selected-MDA-MB231 and selected-MCF7 cells stained with crystal violet are shown FIG. 32. Also, the effect of S101 on cancer cell death after glucose deprivation was measured using a TUNEL assay. The results are shown in FIG. 33.

### 2) Verification of in vivo mouse model

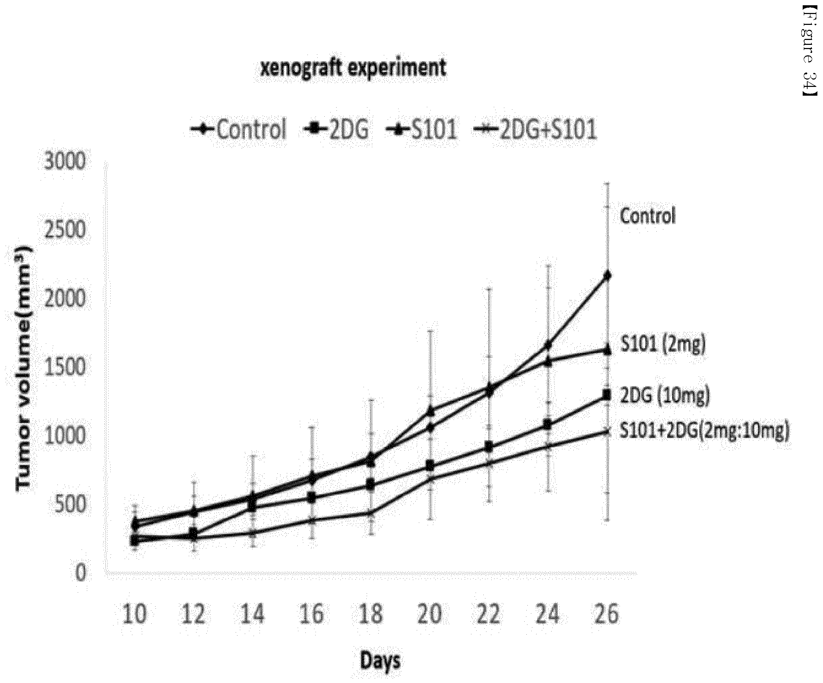

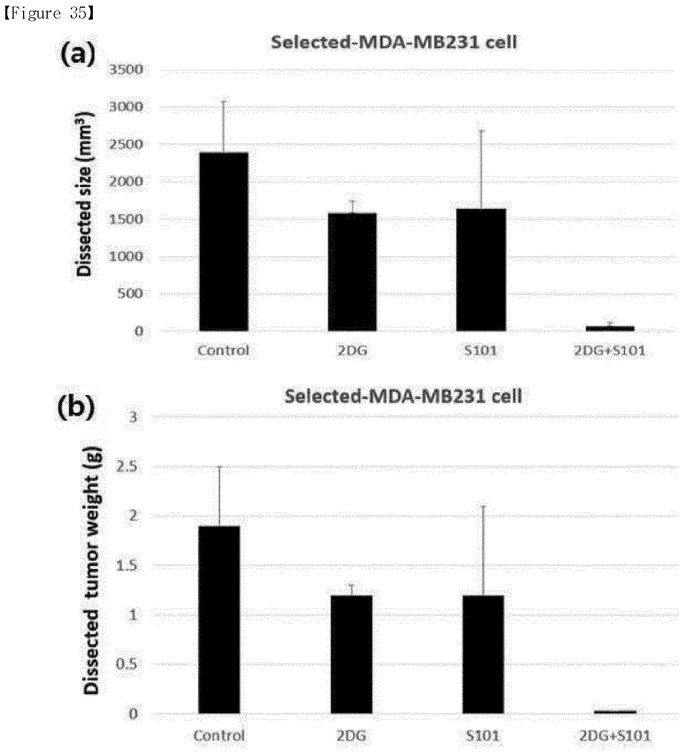

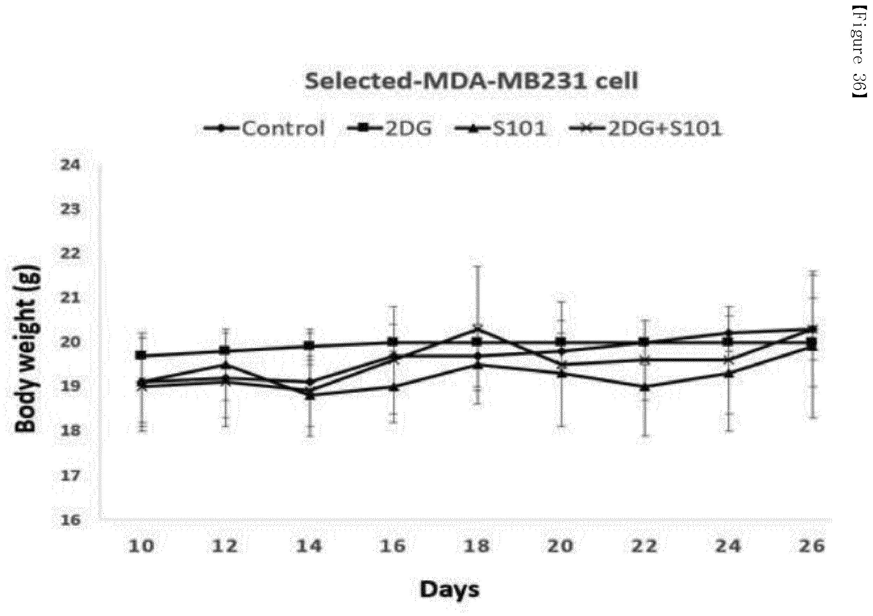

The selected-MDA-MB231 cells verified as cancer stem cells were cultured *in vitro,* and harvested. Thereafter an *in vivo* mouse model was established to verify the anticancer effect. As the control group, a case in which severe ER stress was caused and 2-deoxy-d-glucose was used instead of the anticancer drug was used as Comparative Example 1 (2DG). A case in which 2 mg of S101 was used alone was used as Example 2 (S101 alone), and a case in which 10 mg of 2-deoxy-d-glucose and 2 mg of S101 were used was used as Example 3 (S101+2DG, 2 mg: 10 mg) in order to verify the anticancer effect. The results of changes in tumor volume are shown in FIG. 34. The results obtained by comparing the weights and sizes of dissected tumors between the respective groups are shown in FIGS. 35A and 35B, respectively. In the selected-MDA-MB231 cell xenograft model, changes in body weight between the respective groups were measured when the anticancer effect was measured after the combined treatment with S101 and 2DG. The results are shown in FIG. 36.

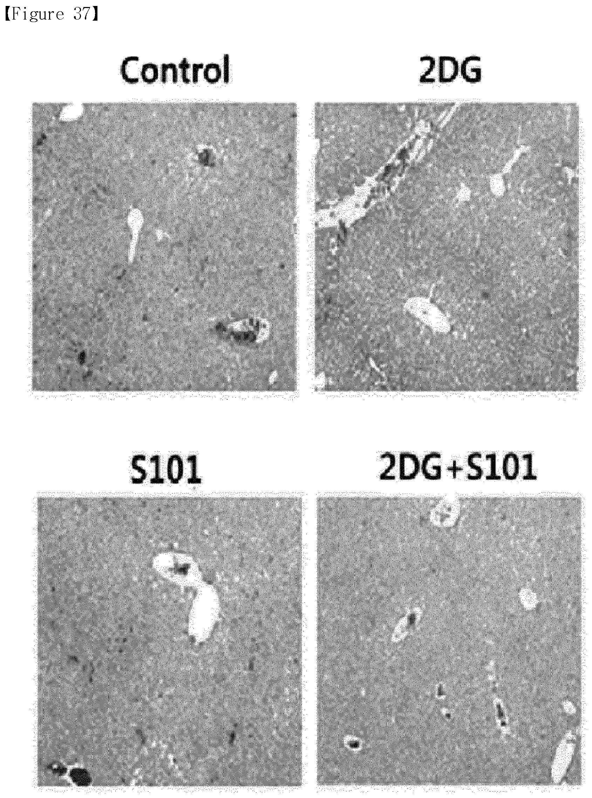

To determine the degree of cytotoxicity when the cells were treated with S101 and 2DG alone or treated with S101 and 2DG together, the liver was extracted from each group to prepare paraffin-embedded tissue blocks. Thereafter, the tissue blocks were microtomed to prepare slides, and then stained with H&E to pathologically decipher whether liver tissue degradation occurred, thereby inferring the degree of cytotoxicity. FIG. 37 shows liver tissue with a magnification of 400 x.

While the present invention has been described in detail with reference to exemplary embodiments of the present invention, it will be obvious to those of ordinary skill in the art to which the present invention belongs that this specific description is just a preferred embodiment, and is not intended to limit the scope of the present invention. Therefore, it should be understood that the technical scope of the present invention should be defined by the appended claims and equivalents thereof.

## Claims

1. A compound represented by the following Formula 1, or a pharmaceutically acceptable salt thereof: 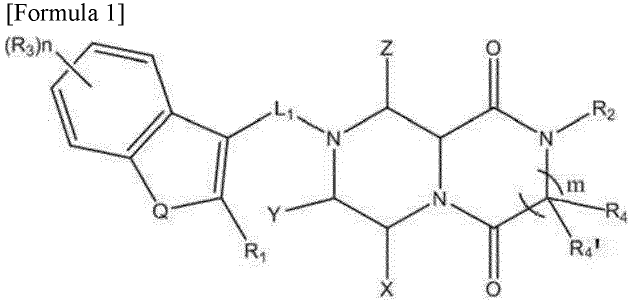 wherein:
R₁ is hydrogen, a linear or branched alkyl, an alkoxy, a haloalkyl, a haloalkoxy, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₃~C₆ cycloalkyl, a C₁~C₆ haloalkyl, a C₁~C₆ haloalkoxy, oxo, cyano, an unsubstituted or substituted aryl, or an unsubstituted or substituted heteroaryl;
R₃ is hydrogen, a linear or branched alkyl, a cycloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted aryl, or an unsubstituted or substituted heteroaryl;
L₁ is a C₁~C₁₀ alkylene, wherein the alkylene may be substituted with at least one selected from a C₁~C₆ alkyl, a C₃~C₆ cycloalkyl, a C₁~C₆ alkoxy, hydroxyl, oxo, or a halogen, and the C₁~C₆ alkyl, the C₃~C₆ cycloalkyl, and the C₁~C₆ alkoxy may be substituted with an unsubstituted or substituted aryl;
Q is S, Se, NR, P, P(O), P(O)₂, or P(O)OR, wherein R is hydrogen, a linear or branched alkyl, a cycloalkyl, a bi- or tri-cycloalkyl, an alkoxy, a haloalkyl, a haloalkoxy, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted aryl, or an unsubstituted or substituted heteroaryl;
R₂ is hydrogen, a linear alkyl, a cycloalkyl, an alkoxy, a haloalkoxy, a haloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl;
R₄ and R₄' are each independently hydrogen, a linear or branched alkyl, a cycloalkyl, an alkenyl, an alkynyl, an alkylthio, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl;
R₂ and R₄ may be connected to form a 5- to 10-membered monocyclic or bicyclic ring, wherein R₄' is hydrogen;
m is an integer of 1 or 2, and n is an integer ranging from 1 to 4; and
X, Y, and Z are each independently hydrogen, a linear or branched alkyl, a cycloalkyl, an aryl, a heteroaryl, an alkylaryl, or an alkylheteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted aryl, an unsubstituted or substituted heteroaryl, oxo, nitro, cyano, or trifluoromethyl.

2. The compound or the pharmaceutically acceptable salt thereof of claim 1, wherein m and n are 1.

3. The compound or the pharmaceutically acceptable salt thereof of claim 2, wherein R₁ is hydrogen, a C₁~C₆ linear alkyl, a C₃~C₆ branched alkyl, a C₁~C₆ alkoxy, a C₁~C₆ haloalkyl, a C₁~C₆ haloalkoxy, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₃~C₆ cycloalkyl, a C₁~C₆ haloalkyl, a C₁~C₆ a haloalkoxy, oxo, cyano, an unsubstituted or substituted C₆~C₁₀ aryl, or an unsubstituted or substituted C₅~C₁₀ heteroaryl;
R₃ is hydrogen, a C₁~C₆ linear alkyl, a C₃~C₆ branched alkyl, a C₃~C₆ cycloalkyl, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, oxo, cyano, an unsubstituted or substituted C₆~C₁₀ aryl, or an unsubstituted or substituted C₅~C₁₀ heteroaryl;
R₂ is hydrogen, a C₁~C₆ linear alkyl, a C₃~C₆ cycloalkyl, a C₁~C₆ alkoxy, a C₁~C₆ haloalkoxy, a C₁~C₆ haloalkyl, aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or substituted C₆~C₁₀ aryl, an unsubstituted or substituted C₅~C₁₀ heteroaryl, oxo, nitro, cyano, or trifluoromethyl;
R₄ and R₄' are each independently hydrogen, a C₁~C₆ linear alkyl, a C₃~C₆ branched alkyl, a C₃~C₆ cycloalkyl, a C₂~C₆ alkenyl, a C₂~C₆ alkynyl, a C₁~C₆ alkylthio, an aryl, a heteroaryl, an alkyl(C₁~C₆) aryl, or an alkyl(C₁~C₆) heteroaryl, each of which may be independently substituted with at least one selected from hydroxyl, a halogen, a C₁~C₆ alkyl, a C₁~C₆ alkoxy, a C₁~C₆ haloalkyl, a C₁~C₆ haloalkoxy, an unsubstituted or an substituted C₆~C₁₀ aryl, an unsubstituted or substituted C₅~C₁₀ heteroaryl, oxo, nitro, cyano, or trifluoromethyl; and
R₂ and R₄ may be connected to form a 5- to 10-membered monocyclic or bicyclic ring, wherein R₄' is hydrogen.

4. The compound or the pharmaceutically acceptable salt thereof of claim 2, wherein R₁ is hydrogen, an unsubstituted or substituted C₁~C₆ linear alkyl, unsubstituted or substituted benzyl, or unsubstituted or substituted 2-phenylethyl, wherein the substituted C₁~C₆ linear alkyl is substituted with a C₃~C₆ cycloalkyl, and the substituted benzyl or the substituted 2-phenylethyl is substituted with halo or substituted with phenyl at least one of the ortho, meta, and para positions;
R₃ is hydrogen, an unsubstituted or substituted C₁~C₆ linear alkyl, an unsubstituted or substituted C₁~C₆ alkoxy, a halogen, or unsubstituted or substituted 2-phenylethyl, wherein the substituted 2-phenylethyl is substituted with halo at least one of the ortho, meta, and para positions, and the substituted C₁~C₆ alkoxy is substituted with phenyl or benzyl;
L₁ is a C₁~C₄ alkylene, wherein the alkylene is substituted with hydrogen, oxo, hydroxyl, a C₁~C₄ alkoxy, or a C₁~C₄ alkyl, and the C₁~C₄ alkoxy is substituted with benzyl;
R₂ is hydrogen, an unsubstituted or substituted C₁~C₆ linear alkyl, unsubstituted or substituted benzyl, or unsubstituted or substituted 2-phenylethyl, wherein the substituted linear alkyl is substituted with halo, the substituted benzyl is substituted with halo, a C₁~C₃ linear alkyl, trifluoromethyl, cyano, nitro, a C₁~C₃ alkoxy, or an aryl at least one of the ortho, meta, and para positions, and the 2-phenylethyl is substituted with halo at least one of the ortho, meta, and para positions;
R₄ and R₄' are each independently hydrogen, an unsubstituted or substituted C₁~C₆ linear alkyl, a C₃~C₆ branched alkyl, a C₃~C₆ cycloalkyl, a C₂~C₆ alkynyl, a C₁~C₆ alkylthio, unsubstituted or substituted benzyl, 2-naphthylmethyl, or 1-naphthylmethyl, wherein the substituted linear alkyl is substituted with methylthio, an alkynyl, or benzyloxycarbonylamino, and the substituted benzyl is substituted with halo, an aryl, nitro, cyano, a C₁~C₃ alkoxy, trifluoromethyl, or benzyloxy at least one of the ortho, meta, and para positions; and
R₂ and R₄ may be connected to form a 5- to 10-membered monocyclic or bicyclic ring, wherein R₄' is hydrogen.

5. The compound or the pharmaceutically acceptable salt thereof of claim 2, wherein R₁ is hydrogen, a C₁~C₆ linear alkyl, a C₃~C₆ branched alkyl, a C₃~C₆ cycloalkyl, 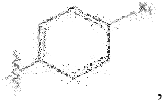 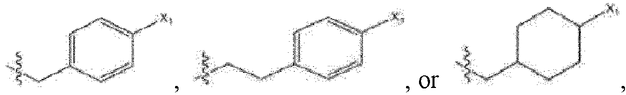 wherein X₁ is hydrogen, halo, a C₁~C₃ linear alkyl, CF₃, CN, NO₂, or an aryl;
R₃ is hydrogen, a C₁~C₃ linear alkyl, an unsubstituted or substituted C₁~C₃ alkoxy, a halogen, 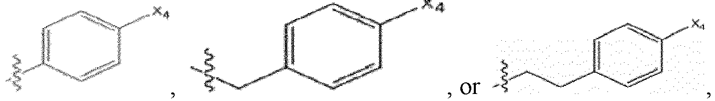 wherein X₄ is hydrogen or halo, and the substituted C₁~C₃ alkoxy is substituted with phenyl or benzyl;
L₁ is a C₁~C₄ alkylene, wherein the alkylene is substituted with hydrogen, oxo, hydroxyl, a C₁~C₄ alkyl, or 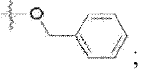
R₂ is hydrogen, an unsubstituted or substituted C₁~C₆ linear alkyl, an unsubstituted or substituted C₁~C₃ alkoxy, 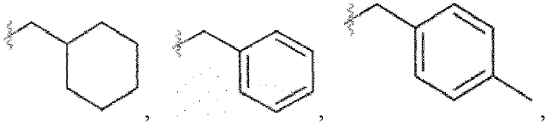 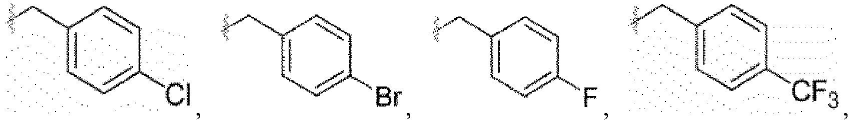 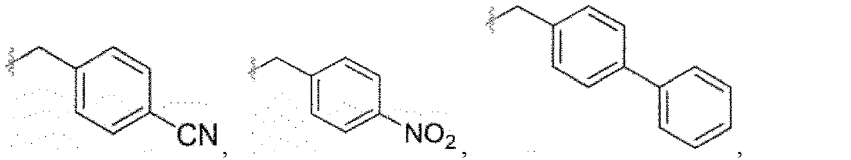 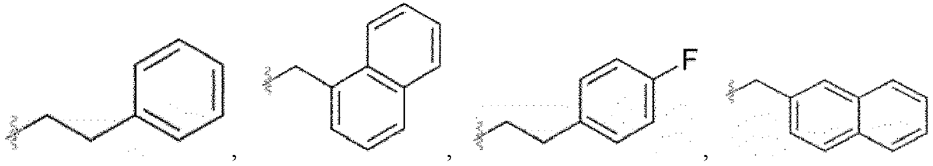 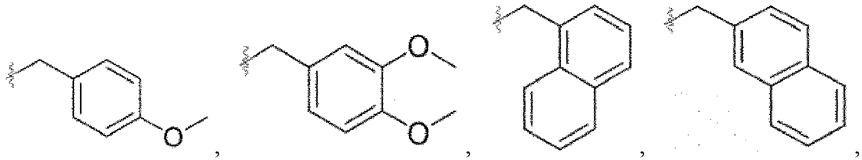 or 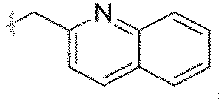 , wherein the substituted C₁~C₆ linear alkyl is substituted with halo;
R₄ and R₄' are each independently hydrogen, an unsubstituted or substituted C₁~C₄ linear alkyl, a C₃~C₄ branched alkyl, a C₃~C₆ cycloalkyl, 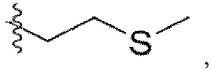 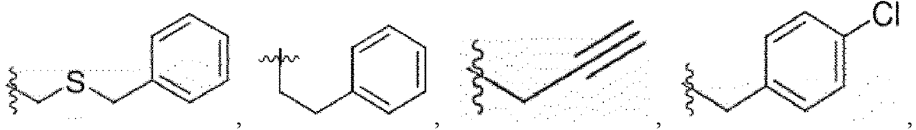 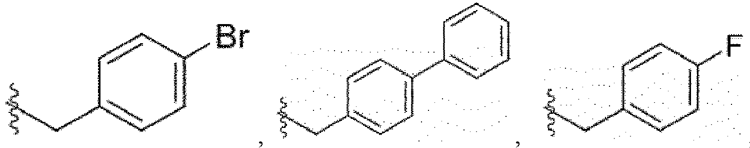 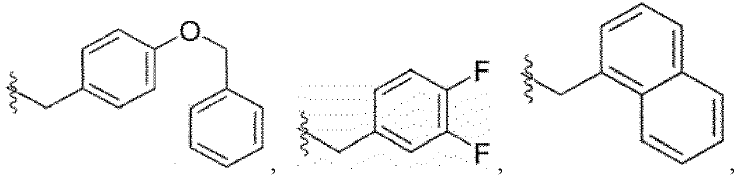 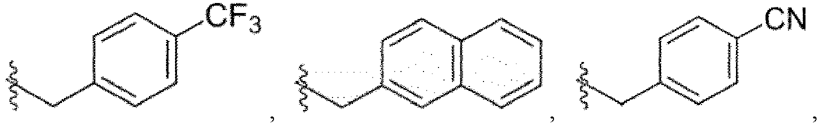 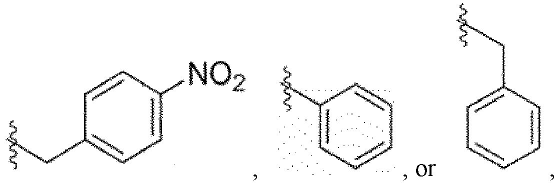 wherein the substituted C₁~C₄ linear alkyl is substituted with a C₃~C₆ cycloalkyl;
R₂ and R₄ may be connected to form a 5- to 10-membered monocyclic or bicyclic ring, wherein R₄' is hydrogen; and
X, Y, and Z are hydrogen.

6. The compound or the pharmaceutically acceptable salt thereof of claim 2, wherein Q is S or NR; and
R is hydrogen, an unsubstituted or substituted C₁~C₄ linear alkyl, 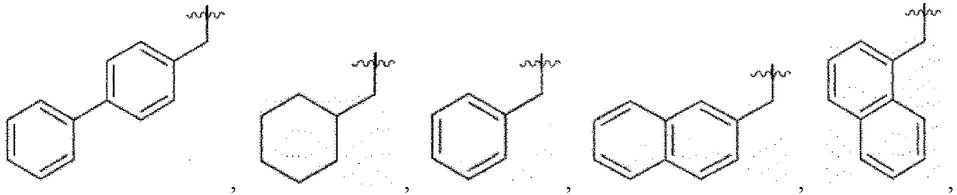 or

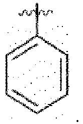

7. The compound or the pharmaceutically acceptable salt thereof of claim 2, wherein the compound of Formula 1 comprises a compound represented by the following Formula 3 or Formula 4 or a pharmaceutically acceptable salt thereof: 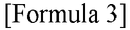 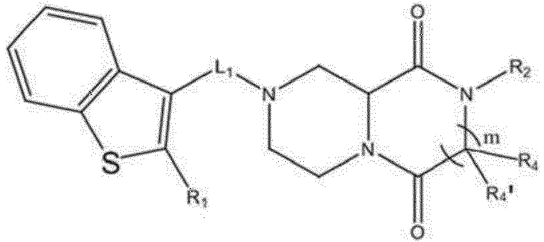 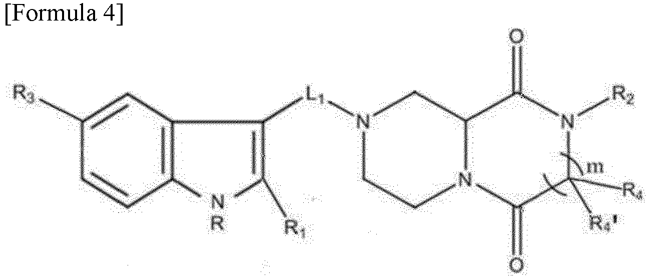 wherein R₁ is hydrogen, a C₁~C₆ linear alkyl, a C₃~C₆ branched alkyl, a C₃~C₆ cycloalkyl, 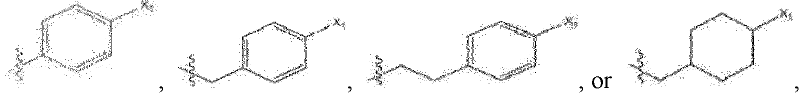 wherein X₁ is hydrogen, halo, a C₁~C₃ linear alkyl, CF₃, CN, NO₂, or an aryl;
R₃ is hydrogen, a C₁~C₃ linear alkyl, an unsubstituted or substituted C₁~C₃ alkoxy, a halogen, 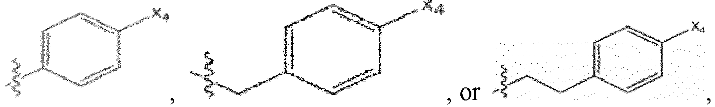 wherein X₄ is hydrogen or halo, wherein the substituted C₁~C₃ alkoxy is substituted with phenyl or benzyl;
L₁ is a C₁~C₄ alkylene, wherein the alkylene is substituted with hydrogen, oxo, hydroxyl, a C₁~C₄ alkyl, or 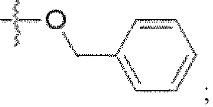 R₂ is hydrogen, an unsubstituted or substituted C₁~C₆ linear alkyl, an unsubstituted or substituted C₁~C₃ alkoxy, 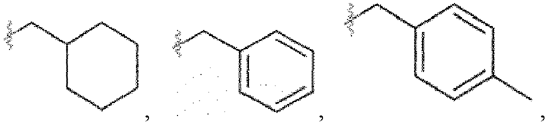 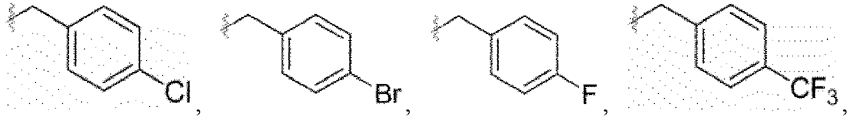 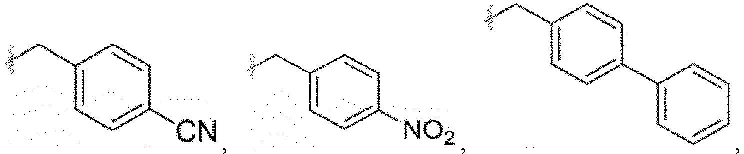 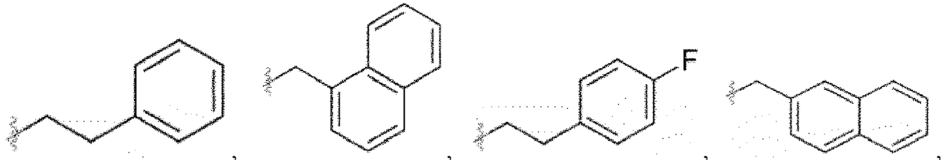 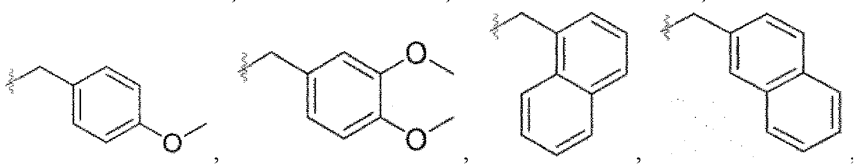 or 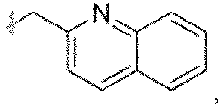 wherein the substituted C₁~C₆ linear alkyl is substituted with halo; R₄ and R₄' are each independently hydrogen, an unsubstituted or substituted C₁~C₄ linear alkyl, a C₃~C₄ branched alkyl, a C₃~C₆ cycloalkyl, 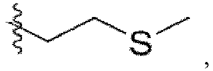 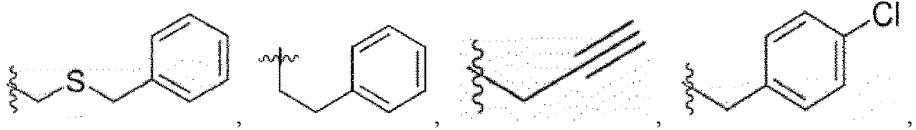 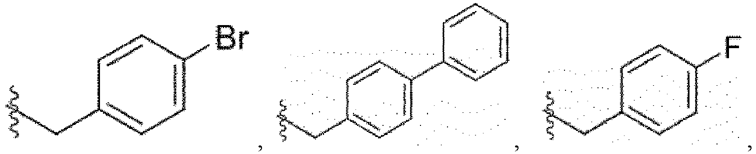 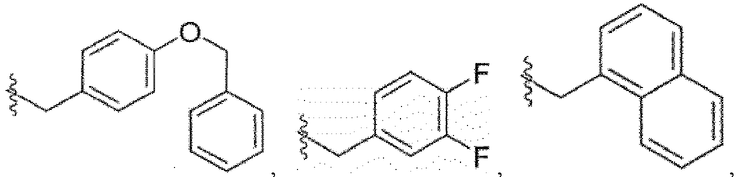 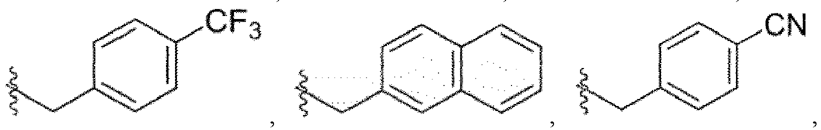 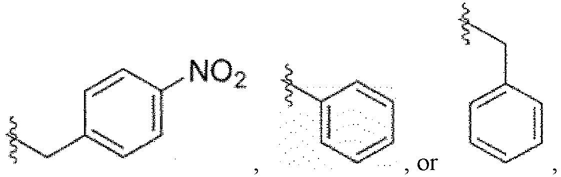 wherein the substituted C₁~C₄ linear alkyl is substituted with a C₃~C₆ cycloalkyl;
R₂ and R₄ may be connected to form a 5- to 10-membered monocyclic or bicyclic ring, wherein R₄' is hydrogen; and
R is hydrogen, an unsubstituted or substituted C₁~C₄ linear alkyl, 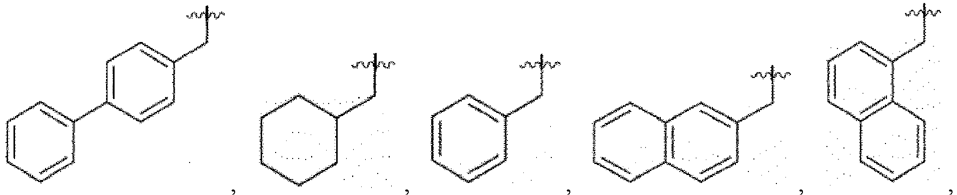 or

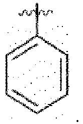

8. A pharmaceutical composition for preventing or treating cancer or resistant cancer, comprising the compound defined in any one of claims 1 to 7, or a pharmaceutically acceptable salt, a hydrate, a solvate, a structural isomer, an optical isomer, or a stereoisomer thereof, or a combination thereof.

9. The pharmaceutical composition of claim 8, wherein the resistant cancer has resistance to an anticancer drug or resistance to radiation.

10. The pharmaceutical composition of claim 8, wherein the resistant cancer comprises one or more selected from the group consisting of ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, bile duct cancer, blood cancer, bladder cancer, kidney cancer, melanoma, colon cancer, bone cancer, skin cancer, head and neck cancer, uterine cancer, rectal cancer, brain cancer, anal cancer, fallopian tube carcinoma, endometrial carcinoma, vaginal cancer, vulvar carcinoma, esophagus cancer, small intestine cancer, endocrine carcinoma, adrenal cancer, soft tissue sarcoma, urethral cancer, penile cancer, ureteral cancer, renal cell carcinoma, renal pelvic carcinoma, a central nervous system (CNS) tumor, a spinal cord tumor, brainstem glioma, and pituitary adenoma.

11. The pharmaceutical composition of claim 9, wherein the anticancer drug comprises one or more selected from the group consisting of nitrogen mustard, imatinib, oxaliplatin, rituximab, erlotinib, neratinib, lapatinib, gefitinib, vandetanib, nilotinib, semaxanib, bosutinib, axitinib, masitinib, cediranib, lestaurtinib, trastuzumab, gefitinib, bortezomib, sunitinib, pazopanib, toceranib, nintedanib, regorafenib, semaxanib, tivozanib, ponatinib, cabozantinib, carboplatin, sorafenib, lenvatinib, bevacizumab, cisplatin, cetuximab, viscum album, asparaginase, tretinoin, hydroxycarbamide, dasatinib, estramustine, gemtuzumab ozogamicin, ibritumomab tiuxetan, heptaplatin, methylaminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacytidine, methotrexate, uracil, cytarabine, fluorouracil, fludarabine, enocitabine, flutamide, capecitabine, decitabine, mercaptopurine, thioguanine, cladribine, carmofur, raltitrexed, doxetaxel, paclitaxel, cabazitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacabazine, thiotepa, nimustine, chlorambucil, mitolactol, leucovorin, tretinoin, exmestane, aminoglutethimide, anagrelide, olaparib, navelbine, padrazol, tamoxifen, toremifene, testolactone, anastrozole, letrozole, borozol, bicalutamide, lomustine, vorinostat, entinostat, and carmustine.

12. A method of treating cancer exhibiting resistance to oncologic therapies, comprising:
administering a therapeutically effective amount of the compound defined in any one of claims 1 to 7, or a pharmaceutically acceptable salt, a hydrate, a solvate, a structural isomer, an optical isomer, or a stereoisomer thereof, or a combination thereof to a subject with resistant cancer.

13. The method of claim 12, wherein chemotherapeutic agents useful for treating cancer or a proliferative disease are administered simultaneously, separately, or sequentially.

14. A use of the compound defined in any one of claims 1 to 7, or a pharmaceutically acceptable salt thereof for preparing a medicament for the treatment of cancer or resistant cancer.

15. A use of the compound defined in any one of claims 1 to 7, or a pharmaceutically acceptable salt thereof for preparing a medicament for the treatment of stem cell-like cancer.
